# FASCICULE DE BREVET EUROPEEN

(11) **EP 0 506 539 B1**
(45) Date de publication et mention de la délivrance du brevet: **02.05.1997**
(21) Numéro de dépôt: 92400782.6
(22) Date de dépôt: 24.03.1992
(51) Int. Cl.: C07D 263/58, C07D 277/68, C07D 413/12, A61K 31/425, A61K 31/42

(54) **Amides alkyl hétérocycliques, leur procédé de préparation et les compositions pharmaceutiques qui les contiennent**
Heterozyklische Alkylamide, Verfahren zu ihrer Herstellung und diese enthaltende pharmazeutische Zubereitungen
Heterocyclic alkylamides, process for their preparation and pharmaceutical compositions containing them

(30) Priorité: 25.03.1991 FR 9103538
(43) Date de publication de la demande: 30.09.1992
(73) Titulaire: ADIR ET COMPAGNIE, 92415 Courbevoie Cédex (FR)
(72) Inventeur: Yous, Said,UFR de Pharmacie,Laboratoire, F-59045 Lille Cedex (FR); Lesieur, Isabelle, F-59147 Gondecourt (FR); Depreux, Patrick, F-59280 Armentières (FR); Caignard, Daniel-Henri, F-75015 Paris (FR); Guardiola, Béatrice, F-92200 Neuilly sur Seine (FR); Adam, Gérard, F-78600 Le Mesnil le Roi (FR); Renard, Pierre, F-78000 Versailles (FR)

(56) Documents cités:
- EP-A- 0 007 772
- EP-A- 0 110 781
- EP-A- 0 174 811
- EP-A- 0 242 173
- EP-A- 0 281 309
- JOURNAL OF MEDICINAL CHEMISTRY vol. 30, no. 7, Juillet 1987, WASHINGTON D. C. (US) pages 1166 - 1176; J. WEINSTOCK ET AL: 'Synthesis and Evaluation of Non-Catechol D-1 and D-2 Dopamine Receptor Agonists:Benzimidazol-2-one,Benzoxazol-2-one,and the Highly Potent Benzothiazol-2-one 7-Ethylamines'

## Description

La présente invention concerne de nouveaux amides alkyl hétérocycliques, leur procédé de préparation et les compositions pharmaceutiques qui les contiennent.

Très peu d'amides alkyl hétérocycliques à motif benzoxazolinone, benzothiazolinone ou benzoxazinone ont été décrits. Quelques 3-(acélamido méthyl) benzoxazolinones ont été synthétisées (Khim. Geterotsikl. Soedin, 85, (9), 1185-8 et God. Sofii, Univ. Khim. Fak, 79, 70, Pt 2, 35-39) sans qu'il soit fait mention d'activité pharmacologique. Par ailleurs on connaît des 2-halo acétamides benzoxazolinoniques ou benzothiazolinoniques décrits comme herbicides (brevets GB 209478, Fr 2479221, US 4311858, US 4258196 et EP 07 772). D'autre part, le brevet EP 242173 décrit des aminoalkylhétérocycles en tant qu'antiarrythmiques.

La 7-(2 7-(2-trifluoroacétamido éthyl) benzothiazolinone a par ailleurs été décrite (J. Med. Chem. 87, 30, (7), 1166-1176) en tant que simple intermédiaire de synthèse.

La demanderesse a présentement découvert de nouveaux amides éthyl hétérocycliques qui possèdent la propriété de se fixer avec une très haute affinité sur les récepteurs de la mélatonine. Cette caractéristique rend les dérivés de l'invention intéressants dans les troubles du système nerveux central, - notamment en tant qu'anxiolytiques et antipsychotiques - de la circulation cérébrale et en tant qu'analgésiques. Les dérivés de l'invention sont également susceptibles d'être utilisés comme régulateur de l'ovulation ainsi que dans le traitement de certains cancers de par leurs propriétés immunostimulantes.

Plus particulièrement, l'invention concerne les dérivés de formule générale (I) : 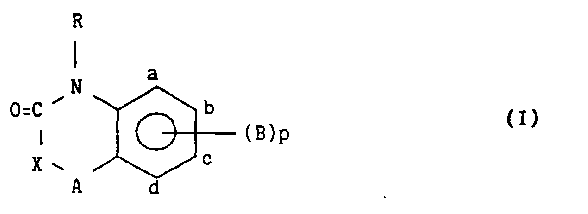 dans laquelle :
A représente un atome d'oxygène ou de soufre,
X représente un groupement CH₂ ou une liaison simple,
R représente :
   ^{*} ou bien un atome d'hydrogène ou un groupement alkyle inférieur et dans ce cas p = 1 et B représente un chaînon -CH₂-CH₂-NR₁-CO-R₂ où R₁ représente un atome d'hydrogène ou un groupement alkyle inférieur linéaire ou ramifié,
   et R₂ représente :
      un atome d'hydrogène,
      un groupement cycloalkyle ou alkyle inférieur linéaire ou ramifié, éventuellement substitué par un atome d'halogène,
      un groupement cycloalkyle substitué par un groupement alkyle inférieur linéaire ou ramifié,
      un groupement aryle ou héléroaryle ou arylalkyle inférieur ou aryle substitué ou héléroaryle substitué ou arylakyle substitué, étant entendu que par groupement hétéroaryle on entend un groupement insaturé mono ou bicyclique comprenant de 1 à 3 hétéroatomes choisis parmi azote, oxygène, ou soufre chaque cycle comprenant 4 ou 5 sommets,
      un groupement de formule : 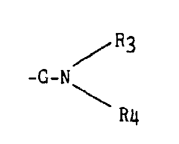
      G représentant un groupement alkyle inférieur linéaire ou ramifié,
      R₃ et R₄ identiques ou différents représentent chacun un groupement alkyle inférieur ou un atome d'hydrogène ou un groupement phényle ou phénylalkyle inférieur, ou R₃ et R₄ forment avec l'atome d'azote auquel ils sont attachés un système hétérocyclique, aromatique ou non, mono ou bicyclique comprenant éventuellement un autre hétéroatome choisis parmi azote, oxygène ou soufre et éventuellement substitué par un ou plusieurs groupements alkyle inférieur ou oxo, aryle ou arylalkyle inférieur, ou aryle substitué ou arylalkyle inférieur substitué, étant entendu que dans les définitions de R₂, R₃ et R₄, le terme substitué affectant les groupements aryle et arylalkyle et hétéroaryle signifie que ces groupements sont substitués par un ou plusieurs radicaux choisis parmi alkyle inférieur, alkoxy inférieur, trifluorométhyle ou atome d'halogène,
   ou bien R₁ forme avec R₂ et le groupement N-CO un système hétérocyclique de formule : 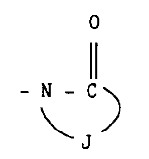 avec J étant un radical alkyle linéaire ou ramifié comprenant de 2 à 8 atomes de carbone,
   ^{*} ou bien un chaînon (CH₂)₂-NR₁-CO-R₂ avec R₁ et R₂ ayant la même définition que précédemment et dans ce cas p vaut 0 ou 1 et B représente un groupement alkoxy inférieur,

   le cas échéant leurs isomères, épimères et diastéréoisomères ainsi que le cas échéant, leurs sels d'addition à un acide ou une base pharmaceutiquement acceptable étant entendu que alkyle inférieur et alkoxy inférieur signifient des groupements comprenant de 1 à 6 atomes de carbone et que cycloalkyle signifie des groupements comprenant de 3 à 8 atomes de carbone.

Parmi les acides pharmaceutiquement acceptables que l'on peut, le cas échéant, ajouter aux composés des formule (1) pour obtenir un sel, on peut citer à titre non limitatif, les acides chlorhydrique, sulfurique, tartrique, maléique, fumarique, oxalique, méthane-sulfonique, camphorique etc...

Parmi les bases pharmaceutiquement acceptables que l'on peut, le cas échéant ajouter aux composés de formule (1) pour obtenir un sel, on peut citer à titre non limitatif les hydroxydes de sodium, potassium, calcium ou aluminium, les carbonates de métaux alcalino ou alcalins terreux ou des bases organiques comme latriéthylamine, la benzylamine, la diéthylamine, la tert-butylamine, la dicyclohexylamine, l'arginine etc...

La présente invention a également pour objet le procédé de préparation des composés de formule (1) caractérisé : . lorsque B est différent du chaînon -CH₂-CH₂NR₁COR₂, en ce que l'on utilise comme matière première un dérivé de formule (II) : 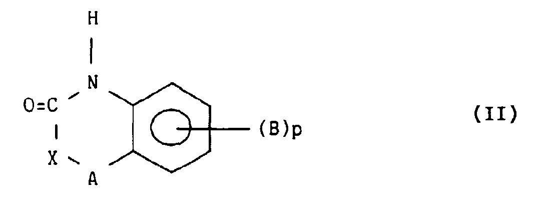 dans laquelle A, B, p et X ont la même définition que dans la formule (I) à l'exception du cas où B représente un chaînon -CH₂-CH₂-NR₁COR₂; que l'on traite par un agent alcalin pour obtenir un dérivé de formule (III) : 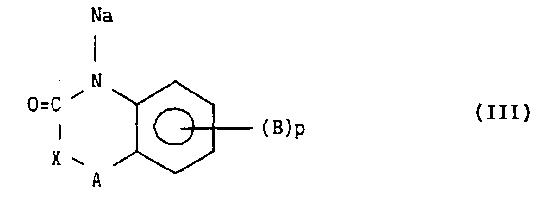 que l'on traite :
- ou bien par un dérivé de formule (IV/A) : 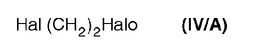 ou Hal et Halo identiques ou différents représentent un atome d'halogène, pour obtenir un dérivé de formule (V/A) : 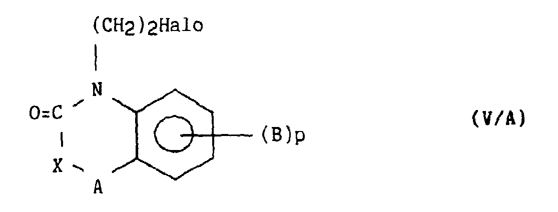 dans laquelle A, X, B, p et Halo ont la même signification que précédemment,
   que l'on traite par une amine de formule HNR₁ où R₁ a la même définition que dans la formule (I) pour obtenir un dérivé de formule (VI) : 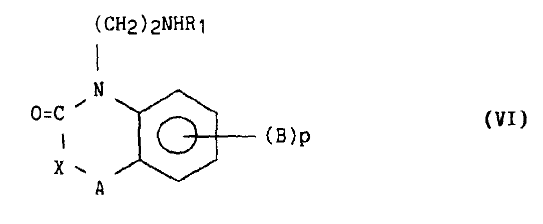 dans laquelle A, B, p, R₁ et X ont la même définition que précédemment,
- ou bien par un dérivé de formule (IV/B) : 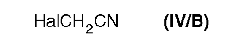 dans laquelle Hal a la même signification que précédemment,
   pour obtenir un dérivé de formule (V/B) : 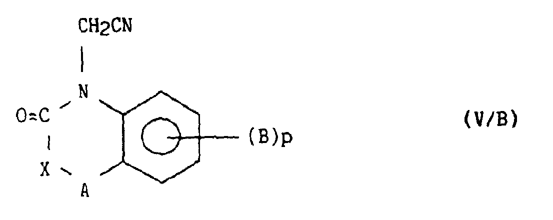 dans laquelle X, A, B et p ont la même signification que précédemment, dérivé de formule (V/B) que l'on soumet à un agent de réduction puis, lorsque dans le dérivé de formule (I) que l'on souhaite obtenir R₁ représente un groupement alkyle inférieur, à un agent d'alkylation, pour obtenir un dérivé de formule (VI) telle que définie précédemment,
   dérivé de formule (VI) que l'on traite :
- soit par un dérivé de formule (VII) : 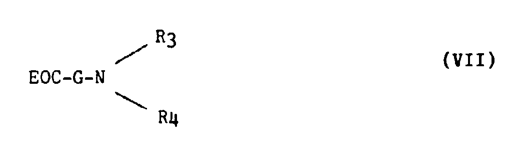 dans laquelle E signifie un groupe partant choisi parmi hydroxyle, alkoxy inférieur ou halogène, G, R₃ et R₄ ayant la même signification que dans la formule (I),
   éventuellement en présence d'un agent alcalin,
   pour conduire à un dérivé de formule (I/A), cas particulier des dérivés de formule (I) : 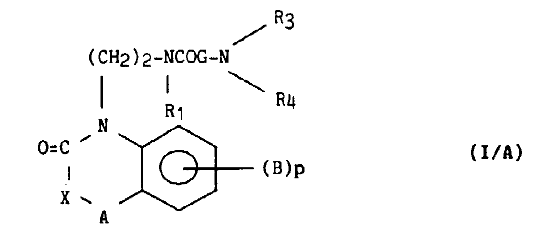 dans laquelle A, B, X, R₁, R₃, R₄, G et p ont la même définition que précédemment, R₂ signifiant ici un groupement : 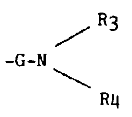 à la condition que B ne représente pas un groupement : 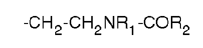 que l'on purifie, si on le désire, par des techniques classiques telles que chromatographie ou cristallisation et que l'on salifie, si on le désire par un acide pharmaceutiquement acceptable,
- soit par un chlorure d'acide de formule (IX) : 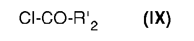 ou par l'anhydride d'acide correspondant,
   R'₂ signifiant ici :
   - un groupement alkyle inférieur linéaire ou ramifié ou cycloalkyle éventuellement substitué par un atome d'halogène, ou cycloalkyle substitué par un groupement alkyle inférieur linéaire ou ramifié,
   - un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle, le terme hétéroaryle ayant la même signification que précedemment,

   pour conduire à un dérivé de formule (I/B) : 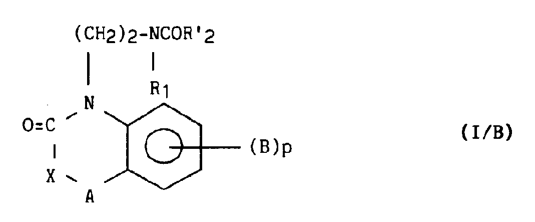 cas particulier des dérivés de formule (I) dans laquelle m, X, A, B, p, R₁ et R'₂ ont la même définition que précédemment, B ne pouvant représenter un chaînon -CH₂-CH₂-NR₁COR₂,
   que l'on purifie si nécessaire par des techniques classiques telles que chromatographie et/ou cristallisation, dérivé de formule (I/B) qui dans le cas où R'₂ représente un groupement alkyle inférieur, linéaire ou ramifié, substitué par un atome d'halogène, peut-être soumis, si on le désire à l'action d'une amine de formule (X) : 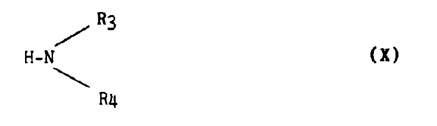 dans laquelle R₃ et R₄ ont la même définition que précédemment,
   en excès ou en présence d'une amine tertiaire ou d'un sel de métal alcalin pour conduire à un dérivé de formule (I/A) tel que précédemment défini, qui si on le désire est purifié par une technique classique telle que chromatographie et/ ou cristallisation, et/ou salifié par un acide pharmaceutiquement acceptable,
   dérivé de formule (I/B) qui lorsque R'₂ représente un substituant alkyle linéaire ou ramifié comprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/C) : 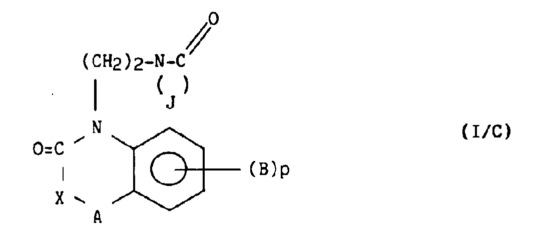 dans laquelle B, p, X et A ont la même signification que précédemment étant entendu que B ne peut représenter un groupement -CH₂-CH₂NRCOR₂ et J représente un groupement alkyle linéaire ou ramifié comprenant de 2 à 8 atomes de carbone, cas particulier des dérivés de formule (I) pour lesquels R₁ et R₂ forment avec NCO un système monocyclique substitué par un groupement oxo et éventuellement substitué par un ou plusieurs groupements alkyles inférieur, que l'on purifie, si on le désire, par une technique choisie parmi cristallisation ou chromatographie,
   . lorsque B représente un chaînon -CH₂-CH₂NR₁COR₂ et p représente 1,
   en ce que l'on utilise comme matière première un dérivé de formule (VIII) : 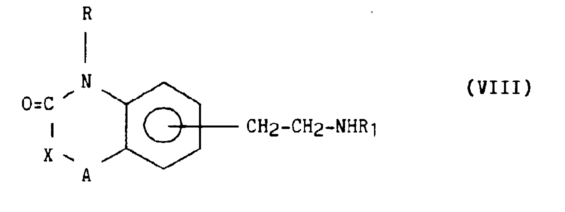 dans laquelle R₁, R, X et A ont la même définition que dans la formule (I),
   dérivés décrits dans la demande de brevet Fr 90.11866, dans les brevets US 4554284 et 4558060, que l'on traite :
^{*} ou bien par un dérivé de formule (VII) : 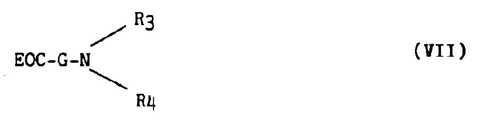 dans laquelle E signifie un goupe partant choisi parmi hydroxyle, alkoxy inférieur ou halogène, G, R₃ et R₄ ayant la même signification que dans la formule (1),
   éventuellement en présence d'un agent alcalin,
   pour conduire à un dérivé de formule (I/D), cas particulier des dérivés de formule (I) : 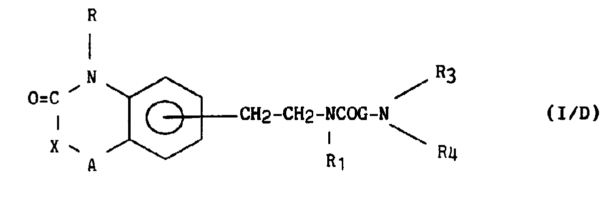 dans laquelle X et A, R₁, R₃, R₄ et G ont la même définition que précédemment, et R représente un atome d'hydrogène ou un groupement alkyle inférieur,
   R₂ signifiant ici un groupement : 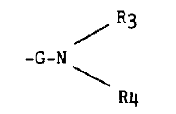 que l'on purifie, si on le désire, par des techniques classiques telles que chromatographie, cristallisation et que l'on salifie, si on le désire, par un acide pharmaceutiquement acceptable, ou lorsque R représente un atome d'hydrogène par une base pharmaceutiquement acceptable,
^{*} ou bien par un chlorure d'acide de formule (IX) : 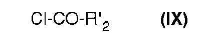 ou par l'anhydride d'acide correspondant,
   R'₂ signifiant ici :
      - un groupement alkyle inférieur linéaire ou ramifié ou cycloalkyle éventuellement substitué par un atome d'halogène, ou cycloalkyle substitué par un groupement alkyle inférieur linéaire ou ramifié,
      - un groupement aryle ou hétéroaryle ou arylalkyle inférieur éventuellememt substitué par un ou plusieurs atomes d'halogène ou groupements choisis parmi alkyle inférieur, alkoxy inférieur, ou trifluorométhyle, le terme hétéroaryle ayant la même signification que précédemment,

      pour conduire à un dérivé de formule (I/E) : 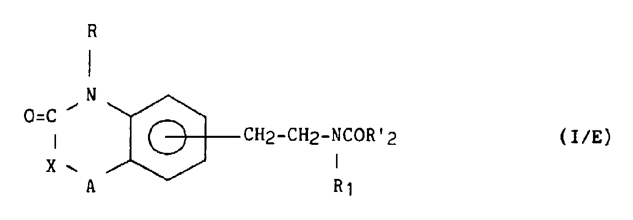 cas particulier des dérivés de formule (1) dans laquelle R₁; X, A et R'₂ ont la même définition que précédemment, R représente un groupement alkyle inférieur ou un atome d'hydrogène,
      que l'on purifie si nécessaire par des techniques classiques telles que chromatographie et/ou cristallisation, que l'on salifie si on le désire par une base pharmaceutiquement acceptable lorsque R représente un atome d'hydrogène, dérivé de formule (I/E) qui dans le cas où R'₂ représente un groupement alkyle inférieur, linéaire ou ramifié, substitué par un atome d'halogène, peut-être soumis, si on le désire à l'action d'une amine de formule (X) : 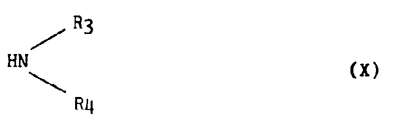 dans laquelle R₃ et R₄ ont la même définition que précédemment,
      en excès ou en présence d'une amine tertiaire ou d'un sel de métal alcalin pour conduire à un dérivé de formule (I/D) tel que précédemment défini, qui si on le désire est purifié par une technique classique telle que chromatographie et/ ou cristallisation, et/ou salifié par un acide pharmaceutiquement acceptable,
      dérivé de formule (I/E) qui lorsque R'₂ représente un substituant alkyle linéaire ou ramifié comprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/F) : 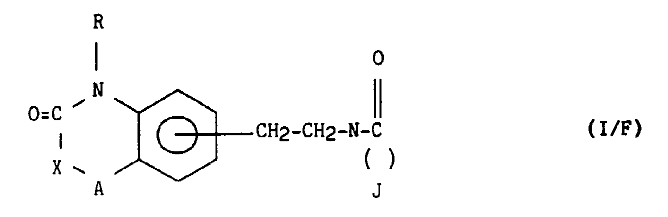 dans laquelle R représente un atome d'hydrogène ou un groupement alkyle inférieur, X et A ont la même signification que précédemment et J représente un groupement alkyle linéaire ou ramifié comprenant de 2 à 8 atomes de carbone, cas particulier des dérivés de formule (I) pour lesquels R₁ et R₂ forment avec NCO un système monocyclique substitué par un groupement oxo et éventuellement substitué par un ou plusieurs groupements alkyles inférieurs,
      que l'on purifie, si on le désire, par une technique choisie parmi cristallisation ou chromatographie et que l'on salifie, si on le désire, lorsque R représente un atome d'hydrogène par une base pharmaceutiquement acceptable.

Les produits de formule (VI) sont nouveaux et font partie de l'invention au même titre que les produits de formule (I) dont ils constituent des intermédiaires de synthèse à l'exception des composés pour lesquels :
- X représente une liaison simple,
- A représente un atome de soufre, p représente 0.

Les produits de formule (V/A) et (V/B) pour lesquels p est différent de 0 sont également nouveaux et font également partie de l'invention au même titre que les produits de formule (1) dont ils constituent des intermédiaires de synthèse.

Un autre cas particulier concerne les dérivés de formule (I/K) : 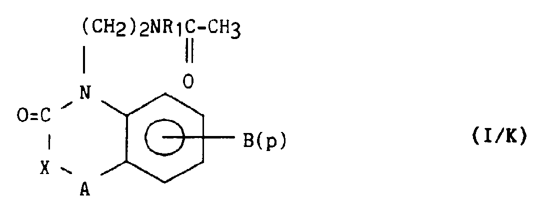 que l'on obtient en une seule étape par réduction catalytique des dérivés de formule (V/K) en milieu d'anhydride acétique : 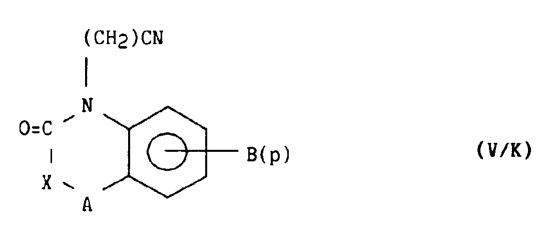 suivie si nécessaire de purification et lorsque R₁ représente un groupement alkyle inférieur du traitement par un agent alkylant.

Les composés de formule (1) possèdent des propriétés pharmacologiques intéressantes.

L'étude pharmacologique des dérivés de l'invention a en effet montré qu'ils sont peu toxiques, doués d'une bonne affinité pour les récepteurs de la mélatonine et qu'en outre ils augmentent de façon importante la synthèse de mélatonine par la glande pinéale. De plus, ils possèdent d'importantes activités sur le système nerveux central et en particulier, on a relevé des propriétés sédatives, anxiolytiques, antipsychotiques, analgésiques ainsi que sur la microcirculation qui permettent d'établir que les produits de l'invention sont utiles dans le traitement du stress, des troubles du sommeil, de l'anxiété, les dépressions saisonnières, les insomnies et fatigues dues aux décalages horaires, schizophrénies, attaque de panique, mélancolie, la régulation de l'appétit, l'insomnie, les troubles psychotiques, l'épilepsie, la maladie de Parkinson, la démence sénile, les divers désordres liés au vieillissement normal ou pathologique, migraine, pertes de mémoire, maladie d'Alzheimer, ainsi que les troubles de la circulation cérébrale.

Dans un autre domaine d'activité, il apparaît que les produits de l'invention possèdent des propriétés d'inhibiteurs de l'ovulation, d'immunomodulateurs et qu'ils sont donc susceptibles d'être utilisés dans le traitement de certains cancers et qu'administrés par voie externe, ils sont utiles dans le traitement du psoriasis, de l'acné, de la séborrhée, protègent la peau et favorisent la pousse des cheveux. Ils peuvent également avoir un usage vétérinaire pour leur propriété sur le pelage.

La présente invention a également pour objet les compositions pharmaceutiques contenant les produits de formule (1) ou le cas échéant un de leurs sels d'addition à un acide pharmaceutiquement acceptable, seuls ou en combinaison avec un ou plusieurs excipients ou véhicules inertes, non toxiques, pharmaceutiquement acceptables.

Parmi les compositions pharmaceutiques selon l'invention, on pourra citer, plus particulièrement celles qui conviennent pour l'administration orale, parentérale, nasale, per/ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables, injectables, etc...

La posologie varie selon l'âge et le poids du patient, la voie d'administration, la nature de l'indication thérapeutique, ou des traitements éventuellement associés et s'échelonne entre 0,1 mg et 1 gramme par 24 heures.

Les exemples suivants illustrent l'invention, mais ne la limitent en aucune façon.

### EXEMPLE 1 :

### N[2-(6-METHOXY 3-BENZOXAZOLINONYL) ETHYL] ACETAMIDE

### STADE A :

### (6-METHOXY 3-BENZOXAZOLINONYL)ACETONITRILE

Dans un ballon de 250 cm³ contenant 100 cm³ d'éthanol absolu, ajouter par petits morceaux 0,1 atome-gramme de sodium sous agitation magnétique.

Laisser agiter jusqu'à dissolution totale du sodium. Ajouter 0,1 mole de la 6-méthoxy benzoxazolinone, poursuivre l'agitation pendant 30 minutes, puis porter à sec.

Le dérivé sodé obtenu est solubilisé dans 80 cm³ de diméthylformamide anhydre. Sous agitation magnétique ajouter 0,12 mole de chloroacétonitrile par l'intermédiaire d'une ampoule à brome. Chauffer à 80°C pendant 1 heure 30 minutes. Laisser refroidir, puis verser le milieu réactionnel dans 400 cm³ d'eau glacée.

Essorer le précipité formé, laver jusqu'à neutralité des eaux de lavage, sécher et recristalliser.
Solvant de recristallisation : Alcool à 95°
Rendement : 95%
Point de fusion : 142-143°C 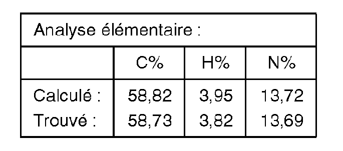
Spectrométrie dans l'infrarouge
(3060-2940) cm⁻¹, v C-H
1770 cm⁻¹, v CO(O-CO-N)
1630 cm⁻¹, v C=C (aromatique)
Spectrométrie de Résonance Magnétique Nucléaire (CDCl₃) :
3,85 ppm, (s, 3H), OCH₃
4,75 ppm, (s, 2H), CH₂-CN
6,80 ppm, (massif, 2H), H₄, H₆
7,05 ppm, (d, 1 H), H₇

### STADE B:

### CHLORHYDRATE DE LA 2-(6-METHOXY 3-BENZOXAZOLINONYL)ETHYLAMINE

Dans un ballon, dissoudre sous agitation magnétique 0,03 mole de (6-méthoxy 3-benzoxazolinonyl)acétonitrile dans 50 cm³ de tétrahydrofuranne à température ambiante.

Ajouter lentement sous atmosphère d'azote 0,05 mole de sulfure de méthylborane à l'aide d'une ampoule à brome. Chauffer à reflux pendant 30 minutes. Refroidir dans un bain de glace, puis ajouter très lentement 30 cm³ d'une solution d'acide chlorhydrique 6N. Chauffer à reflux pendant 30 minutes.

Laisser refroidir, essorer le précipité formé, laver avec 30 cm³ d'acétone, sécher et recristalliser dans un solvant convenable.
Solvant de recristallisation : Alcool à 95°
Rendement : 83%
Point de fusion : > 260°C
Spectrométrie dans l'infrarouge
(3120-2500) cm⁻¹, v NH₂ sel
1770 cm⁻¹, v CO(O-CO-N)
(1635-1620)cm⁻¹, v C=C (aromatique)
Spectrométrie de Résonance Magnétique Nucléaire (CDCl₃) :
3,25 ppm, (t, 2H), CH₂-NH₂
3,75 ppm, (s, 3H), OCH₃
4,15 ppm, (t, 2H), =N-CH₂
6,82 ppm, (dd, 1 H), H₅
7,00 ppm, (d, 1 H), H₇
7,40 ppm, (d, 1 H), H₄
8,40 ppm, (signal, 2H), NH₂ disparait dans D₂0

### STADE C:

### N[2-(6-METHOXY 3-BENZOXAZOLINONYL)ETHYL]ACETAMIDE

Dissoudre 0,02 mole de chlorhydrate de 3-(amino-2-éthyl)6-méthoxy benzoxazolinone dans un mélange eau/chloroforme. Ajouter 0,02 mole de carbonate de potassium et laisser agiter pendant 1 heure. Refroidir dans un bain de glace, puis ajouter lentement 0,022 mole de chlorure d'acétyle. Maintenir l'agitation pendant 1/2 heure. Porter à sec la phase chloroformique, puis recristalliser dans un solvant convenable.
Solvant de recristallisation : Alcool absolu
Rendement : 86%
Point de fusion : 162-164°C 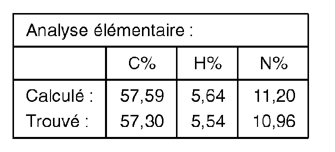
Spectrométrie dans l'infrarouge
3320 cm⁻¹, v N-H
(3060-2840)cm⁻¹ ,v C-H
1765 cm⁻¹ v CO(O-CO-N)
1640 cm⁻¹, v CO (amide)
1620 cm⁻¹, v C=C (aromatique)
Spectrométrie de Résonance Magnétique Nucléaire (CDCl₃)
1,94 ppm, (s, 3H), COCH₃
3,60 ppm, (multiplet, 2H), CH₂-CH₂-NH
3,80 ppm, (s, 3H), OCH₃
3,95 ppm, (t, 2H), N-CH₂
6,20 ppm, (signal, 1H), NH
6,65 ppm, (massif, 2H), H₅, H₇
6,96 ppm, (d, 1 H), H₄

### EXEMPLE 2 :

### N[2-(5-METHOXY 3-BENZOXAZOLINONYL) ETHYL] ACETAMIDE

En procédant comme dans l'exemple 1, mais en remplaçant au stade A la 6-méthoxy benzoxazolinone par la 5-méthoxy benzoxazolinone, on obtient :

### STADE A :

### (5-METHOXY 3-BENZOXAZOLINONYL)ACETONITRILE

### Point de fusion : 148-149°C

### Rendement : 93%

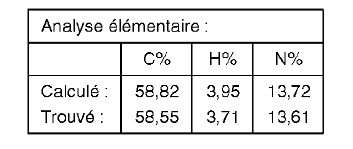

Spectrométrie dans l'infrarouge
(3060-2900) cm⁻¹, v C-H
1790 cm⁻¹, v CO(O-CO-N)
1620 cm-¹, v C=C (aromatique)

### Spectrométrie de Résonance Magnétique Nucléaire (CDCl₃) :

3,86 ppm, (s, 3H), OCH₃
4,75 ppm, (s, 2H), ÇH₂-CN
6,70 ppm, (massif, 2H), H₄, H₆
7,20 ppm, (d, 1 H), H₇

### STADE B :

### CHLORHYDRATE DE 2-(5-METHOXY 3-BENZOXAZOLINONYL)ETHYLAMINE

### Solvant de recristallisation : Alcool à 95°

Rendement : 81%
Point de fusion : >260°C
Spectrométrie dans l'infrarouge :
(3120-2500), v NH₂ sel
1765 cm⁻¹ v CO(O-CO-N)
1620 cm⁻¹, v C=C (aromatique)
Spectrométrie de Résonance Magnétique Nucléaire (CDCl₃) :
₃,₂₅ ppₘ, ₍ₜ, 2H); = CH2 - CH2 - NH₂
3,75 ppm, (s, 3H), OCH₃
4,16 ppm, (t, 2H); -CH2-CH2
6,₇0 ppm, (dd, 1 H), H6
(7-7,40) ppm, (massif, 2H), H₄, H₇
9,50 ppm (signal, 2H) NH₂ disparait dans D₂0

### STADE C :

### N[2-(5-METHOXY 3-BENZOXAZOLINONYL) ETHYL] ACETAMIDE

### Solvant de recristallisation : Toluène

Rendement : 87%
Point de fusion : 118-120°C 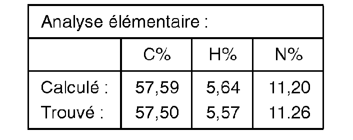
Spectrométrie dans l'infrarouge
3300 cm⁻¹, v N-H (amide)
(3060-2840) cm⁻¹, v C-H
1765 cm-¹, v CO(O-CO-N)
1655 cm⁻⁷, v CO (amide)
1630 cm⁻¹, v C=C (aromatique)
Spectrométrie de Résonance Magnétique Nucléaire (CDCl₃) :
1,93 ppm, (s, 3H), COCH₃
3,58 ppm, (multiplet, 2H), CH₂-NH
3,80 ppm, (s, 3H), OCH₃
3,94 ppm, (t, 2H); CH₂N=
6,25 ppm, (signal, 1H), NH
6,64 ppm, (massif, 2H), H₄, H₆
7,10 ppm, (d, 1 H), H₇,

### EXEMPLE 3 :

### N[2-(5-METHOXY 3-BENZOXAZOLINONYL) ETHYL] ISOBUTYRAMIDE

En procédant comme dans l'exemple 2, Stade C, mais en remplaçant le chlorure d'acétyle par le chlorure d'iso- butyryle, on obtient le produit du titre.
Solvant de recristallisation : Toluène-Hexane
Rendement : 82%
Point de fusion: 130-131 °C 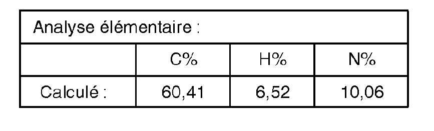 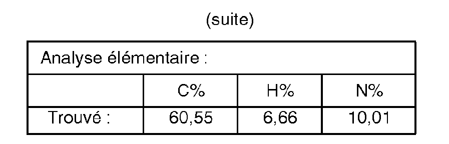
Spectrométrie dans l'infrarouge
3300 cm⁻¹, v N-H
(3080-2840) cm⁻¹, v C-H
1760 cm⁻¹ , v CO (O-CO-N)
1640 cm⁻¹, v CO (amide)
1625 cm⁻¹, v C=C (aromatique)
Spectrométrie de Résonance Magnétique Nucléaire (CDCl₃) :
1,10 ppm, (d, 6H), CH(CH₃)₂
2,25 ppm, (multiplet, 1H), CH
3,75 ppm, (s, 3H), OCH₃
6,20 ppm, (signal, 1H), NH
6,60 ppm, (massif, 2H), H₄, H₆
7,10 ppm, (d, 1 H), H₇

### EXEMPLE 4 :

### N[2-(6-METHOXY 3-BENZOXAZOLINONYL) ETHYL ] ISOBUTYRAMIDE

En procédant comme dans l'exemple 2 - stade C, mais en remplaçant le chlorure d'acétyle par le chlorure d'iso- butyryle, on obtient le produit du titre.
Solvant de recristallisation : Toluène
Point de fusion : 161-162°C
Rendement : 86% 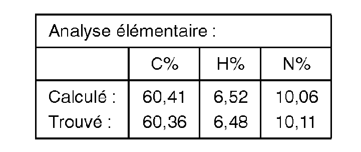
Spectrométrie dans l'infrarouge
3300 cm-¹, v N-H
(3080-2840) cm⁻¹, v C-H
1760 cm⁻¹, v CO (O-CO-N)
1640 cm⁻¹, v CO (amide)
1625 cm⁻¹, v C=C (aromatique)
Spectrométrie de Résonance Magnétique Nucléaire (CDCl₃) :
1,10 ppm, (d, 6H), CH(CH₃)₂
2,25 ppm, (multiplet, 1 H), CH
3,60 ppm, (multiplet, 2H), CH₂-NH
3,75 ppm, (s, 3H), OCH₃
3,95 ppm, (t, 2H) CH₂-N=
6,15 ppm, (signal, 1H), NH
6,65 ppm, (massif, 2H), H₅, H₇
6,95 ppm, (d, 1 H), H₄

### EXEMPLE 5 :

### N[2-(6-METHOXY 3-BENZOXAZOLINONYL) ETHYL] PHENYLACETAMIDE

En procédant comme dans l'exemple 2, mais en remplaçant le chlorure d'acétyle par le chlorure de phénacétyle, on obtient le produit du titre.
Spectrométrie dans l'infrarouge:
3300 cm⁻¹, v N-H
1760 cm⁻¹, v CO(O-CO-N)
Spectrométrie de Résonance Magnétique Nucléaire (CDCl₃) :
3,75 ppm, singulet, 3H, OCH₃

### EXEMPLE 6 :

### N[2-(6-METHOXY 3-BENZOXAZOLINONYL) ETHYL](2-OXO 1-PYRROLIDINYL)ACETAMIDE

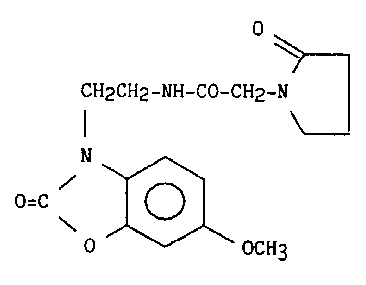

Chauffer sous agitation magnétique à la température de 80°C pendant 3 heures un mélange de 0,02 mole de 3-(2-aminoéthyl)6-méthoxy benzoxazolinone et de 0,022 mole de (2-oxo pyrrolidin-1-yl) acétate de méthyle. Reprendre le milieu par de l'eau légèrement acide, essorer le précipité.
Spectrométrie dans l'infrarouge
3300 cm⁻¹, v N-H
1760 cm⁻¹, v CO(O-CO-N)
Spectrométrie de Résonance Magnétique Nucléaire (CDCl₃) :
3,75 ppm, singulet, 3H, OCH₃

### EXEMPLE 7 :

### N[2-(6-METHOXY 3-BENZOXAZOLINONYL) ETHYL]4-CHLORO BUTYRAMIDE

En procédant comme dans l'exemple 2, mais en remplaçant le chlorure d'acétyle par le chlorure de 4-chloro butyryle, on obtient le produit du titre.
Spectrométrie dans l'infrarouge :
1760 cm⁻¹, v CO(O-CO-N)
Spectrométrie de Résonance Magnétique Nucléaire (CDCl₃) :
3,80 ppm, singulet, 3H, OCH₃

### EXEMPLE 8 :

### N[2-(6-METHOXY 3-BENZOXAZOLINONYL) ETHYL]2-PYRROLIDINONE

Dissoudre 0,01 atome-gramme de sodium dans 50 cm³ d'éthanol. Ajouter sous agitation magnétique le N[2-(6-méthoxy benzoxazolinon-3-yl)éthyl]4-chloro butyramide obtenu dans l'exemple précédent. Maintenir l'agitation 20 minutes. Porter à sec. Solubiliser dans 40 cm³ de diméthylformamide anhydre. Chauffer à ébullition pendant 7 heures. Evaporer sous vide et reprendre par l'éther. Filtrer et porter à sec. Recristalliser.
Spectrométrie dans l'infrarouge :
1760 cm⁻¹, v CO(O-CO-N)
Spectrométrie de Résonance Magnétique Nucléaire (CDCl₃) :
3,80 ppm, singulet, 3H, OCH₃

### EXEMPLE 9 :

### N[2-(6-METHOXY 3-BENZOXAZOLINONYL) ETHYL]2-BROMO ACETAMIDE

Même mode opératoire que l'exemple 7 en remplaçant le chlorure de 4-chloro butyryle par le chlorure de l'acide bromacétique.

### EXEMPLE 10:

### N[2-(6-METHOXY 3-BENZOXAZOLINONYL) ETHYL]2-MORPHOLINO ACETAMIDE

Dissoudre sous agitation magnétique 0,01 mole de morpholine dans 50 cm³ d'acétone. Ajouter 0,012 mole de triéthylamine et 0,01 mole de N[2-(6-méthoxy benzoxazolinon-3-yl)éthyl] 2- bromoacétamide. Porter à reflux une heure sous agitation magnétique. Essorer le précipité formé et évaporer le filtrat. Reprendre le résidu par de l'eau alcaline. Essorer le précipité, laver, sécher et recristalliser.
Spectrométrie dans l'infrarouge:
1760 cm⁻¹, v CO(O-CO-N)

### EXEMPLE 11 :

### N[2-(6-METHOXY 3-BENZOXAZOLINONYL) ETHYL]2-{4-[(2,3,4-TRIMETHOXY PHENYL) METHYL] 1-PIPERAZINYL}ACETAMIDE

En procédant comme dans l'exemple précédent, mais en remplaçant la morpholine par la 1-[(2,3,4-triméthoxy phényl)méthyl]pipérazine, on obtient le produit du titre.

### EXEMPLE 12:

### N[2-(6-METHOXY 3-BENZOXAZOLINONYL) ETHYL]N METHYLACETAMIDE

En remplaçant dans l'exemple 1 la 2-(6-méthoxy-3 benzoxazolinonyl)éthylamine par la N-[2(6-méthoxy 3-benzoxazolinonyl)éthyl]N méthylamine, on obtient le produit du titre.

### EXEMPLE 13:

### N[2-(6-METHOXY 3-BENZOXAZOLINONYL) ETHYL]BENZAMIDE

En remplaçant dans l'exemple 1, stade C le chlorure d'acétyle par le chlorure de benzoyle, on obtient le produit du titre.
Spectrométrie dans l'infrarouge :
   1760 cm⁻¹, v CO

### EXEMPLE 14:

### N[2-(6-METHOXY 3-BENZOXAZOLINONYL) ETHYL]PARATOLUOYL CARBOXAMIDE

En remplaçant dans l'exemple 1, stade C le chlorure d'acétyle par le chlorure de paratoluoyle, on obtient le produit du titre.
Spectrométrie dans l'infrarouge :
   1760 cm⁻¹, v CO

### EXEMPLE 15:

### N[2-(6-METHOXY 3-BENZOXAZOLINONYL) ETHYL]4-FLUORO BENZAMIDE

En remplaçant dans l'exemple 1, stade C le chlorure d'acétyle par le chlorure de parafluorobenzoyle, on obtient le produit du titre.
Spectrométrie dans l'infrarouge :
   1765 cm⁻¹, v CO

### EXEMPLE 16:

### N[2-(6-METHOXY 3-BENZOXAZOLINONYL) ETHYL]3-TRIFLUOROMETHYL BENZAMIDE

En remplaçant dans l'exemple 1, stade C le chlorure d'acétyle par le chlorure de 3-trifluorométhyl benzoyle on obtient le produit du titre.
Spectrométrie dans l'infrarouge :
   1760 cm⁻¹, v CO

### EXEMPLE 17:

### N[2-(6-METHOXY 3-BENZOXAZOLINONYL) ETHYL]3,5-DICHLORO BENZAMIDE

En remplaçant dans l'exemple 1, stade C le chlorure d'acétyle par le chlorure de 3,5-dichloro benzoyle, on obtient le produit du titre.

### EXEMPLE 18:

### N[2-(6-METHOXY 3-BENZOXAZOLINONYL) ETHYL]ISONICOTINAMIDE

En remplaçant dans l'exemple 1, stade C le chlorure d'acétyle par le chlorure d'isonicotinoyle, on obtient le produit du titre.

### EXEMPLE 19:

### N[2-(6-METHOXY 3-BENZOXAZOLINONYL) ETHYL]-2 INDOLYL CARBOXAMIDE

En remplaçant dans l'exemple 1, stade C le chlorure d'acétyle par le chlorure de 2-indolyle, on obtient le produit du titre.
Spectrométrie dans l'infrarouge :
   3400 cm⁻¹, v NH (indole)
   1760 cm⁻¹, v CO

### EXEMPLE 20:

### N[2-(6-METHOXY 3-BENZOXAZOLINONYL) ETHYL]2-BENZYLAMINO ACETAMIDE

En procédant comme dans l'exemple 10 et en remplaçant la morpholine par la benzylamine, on obtient le produit du titre.
Spectrométrie dans l'infrarouge :
   1760 cm⁻¹, v CO

### EXEMPLE 21 :

### N[2-(6-METHOXY 3-BENZOXAZOLINONYL) ETHYL]2-(N',N'- DIETHYL)AMINOACETAMIDE

En procédant comme dans l'exemple 10, mais en remplaçant la morpholine par la N,N diéthylamine, on obtient le produit du titre.
Spectrométrie dans l'infrarouge :
   1765 cm⁻¹, v CO (OCON)

### EXEMPLE 22 :

### N[2-(6-METHOXY 3-BENZOXAZOLINONYL) ETHYL]2-AMINOACETAMIDE, CHLORHYDRATE

Dissoudre sous agitation magnétique 0,012 mole d'hexaméthylène tétramine dans 15 cm³ de chloroforme et introduire 0,01 mole de N[2-(6-méthoxy 3-benzoxazolinonyl) éthyl]2-bromo acétamide obtenu dans l'exemple 9 dissous dans 20 cm³ de chloroforme. Porter à reflux pendant 100 heures. Essorer, sécher. Introduire le précipité dans une fiole à col rodé. Ajouter 150 cm³ d'alcool et 30 cm³ d'acide chlorhydrique concentré. Porter deux heures à reflux. Evaporer le solvant. Recristalliser dans l'alcool à 90°.
Spectrométrie dans l'infrarouge :
   1760 cm⁻¹, v CO (OCON)

### EXEMPLE 23 :

### N[2-(6-METHOXY 3-BENZOXAZOLINONYL) ETHYL]2-[4-(4-FLUOROPHENYL)-1 PIPERAZINYL]ACETAMIDE

En procédant comme dans l'exemple 10, mais en remplaçant la morpholine par la 1-(4-fluoro phényl)pipérazine, on obtient le produit du titre.

### EXEMPLE 24 :

### N[2-(6-METHOXY 3-BENZOXAZOLINONYL) ETHYL]2-[4-(3-TRIFLUOROMETHYL PHENYL) 1- PIPERAZINYL] ACETAMIDE

En procédant comme dans l'exemple 10, mais en remplaçant la morpholine par la 1-(3-trifluorométhyl)pipérazine, on obtient le produit du titre.

### EXEMPLE 25 :

### N[2-(6-METHOXY 3-BENZOXAZOLINONYL) ETHYL]CYCLOHEXANE CARBOXAMIDE (R₂ = cyclohexyle)

En procédant comme dans l'exemple 1, mais en remplaçant le chlorure d'acétyle par le chlorure d'acide cyclohexane carboxylique, on obtient le produit du titre.

### EXEMPLE 26 :

### N[2-(6-METHOXY 3-BENZOXAZOLINONYL) ETHYL]FORMAMIDE

Dans un creuset en porcelaine, placer 0,01 mole de 2-(6-méthoxy 3-benzoxazolinonyl) éthylamine et 0,02 mole d'acide formique. Chauffer à 120°C jusqu'à l'obtention d'un résidu sec. Recristalliser.

### EXEMPLE 27 :

### N[2-(6-METHOXY 3-BENZOXAZOLINONYL) ETHYL]CYCLOPROPANE CARBOXAMIDE

En procédant comme dans l'exemple 1, mais en remplaçant le chlorure d'acétyle par le chlorure d'acide cyclopropane carboxylique on obtient le produit du titre.

### EXEMPLE 28 :

### N[2-(6-METHOXY 3-BENZOXAZOLINONYL) ETHYL]PENTANAMIDE

En procédant comme dans l'exemple 1, mais en remplaçant le chlorure d'acétyle par le chlorure de valéroyle, on obtient le produit du titre.

### EXEMPLE 29 :

### N[2-(6-METHOXY 3-BENZOXAZOLINONYL) ETHYL]CYCLOBUTANE CARBOXAMIDE

En procédant comme dans l'exemple 1, mais en remplacant le chlorure d'acétyle par le chlorure d'acide cyclobutane carboxylique, on obtient le produit du titre.

En procédant comme dans les exemples précédents, on obtient de même :
N[2-(6-METHOXY 3-BENZOXAZOLINONYL) ETHYL]4-BROMO BUTYRAMIDE
N[2-(6-METHOXY 3-BENZOXAZOLINONYL) ETHYL]5-BROMO PENTANAMIDE
N[2-(6-METHOXY 3-BENZOXAZOLINONYL) ETHYL]3-BROMO PROPIONAMIDE
N[2-(6-METHOXY 3-BENZOXAZOLINONYL) ETHYL]-3-MORPHOLINO PROPIONAMIDE
N[2-(6-METHOXY 3-BENZOXAZOLINONYL) ETHYL]-4-MORPHOLINO BUTYRAMIDE
N[2-(6-METHOXY 3-BENZOXAZOLINONYL) ETHYL]-4-{4-[(2,3,4-TRIMETHOXY PHENYL) METHYL]-1-PIPERAZINYL}BUTYRAMIDE
N[2-(6-METHOXY 3-BENZOXAZOLINONYL) ETHYL]-3-{4-[(2,3,4-TRIMETHOXY PHENYL) METHYL]-1-PIPERAZINYL}PROPIONAMIDE
N[2-(6-METHOXY 3-BENZOXAZOLINONYL) ETHYL] 2-PIPERIDINONE
N[2-(6-METHOXY 3-BENZOXAZOLINONYL) ETHYL] 2-BROMO PROPIONAMIDE
N[2-(6-METHOXY 3-BENZOXAZOLINONYL) ETHYL]2-{4-[(2,3,4-TRIMETHOXY PHENYL) METHYL]-1-PIPERAZINYL}PROPIONAMIDE

En remplaçant dans les exemples 5 à 9 la 2-(6-méthoxy 3-benzoxazolinonyl)éthylamine par la 2-(5-méthoxy 3-benzoxazolinonyl) éthylamine, on obtient les produits des exemples précédents méthoxylés respectivement en position 5 de la benzoxazolinone au lieu des dérivés méthoxylés en position 6.

### EXEMPLE 30 :

### N[2-(6-METHOXY 3-BENZOTHIAZOLINONYL) ETHYL]ACETAMIDE

### STADE A :

### (6-METHOXY 3-BENZOTHIAZOLINONYL)ACETONITRILE

Procéder comme dans l'exemple 1, stade A en remplaçant la 6-méthoxy benzoxazolinone par la 6-méthoxy benzothiazolinone.
Solvant de recristallisation : Alcool à 95°
Rendement : 93%
Point de fusion : 168-169°C 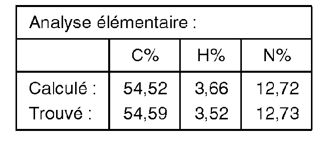
Spectrométrie dans l'infrarouge :
(3080-2940) cm-¹, v C-H
1680 cm⁻¹ v CO (S-CO-N)
(1630-1580) cm⁻¹, v C=C (aromatique)
Spectrométrie de Résonance Magnétique Nucléaire (CDCl₃) :
3,85 ppm, (s, 3H), OCH₃
4,75 ppm, (s, 2H), CH₂-CN
6,75 ppm, (massif, 2H), H₄, H₅
7,30 ppm, (d, 1 H), H₇

### STADE B:

### CHLORHYDRATE DE 2-(6-METHOXY 3-BENZOTHIAZOLINONYL) ETHYLAMINE

Procéder comme dans l'exemple 1, stade B en remplaçant le (6-méthoxy 3-benzoxazolinonyl) acétonitrile par le (6-méthoxy 3-benzothiazolinonyl) acétonitrile obtenu dans le stade A.
Solvant de recristallisation : alcool à 95° - eau (5-1)
Rendement : 83%
Point de fusion : > 260°C
Spectrométrie dans l'infrarouge :
(3120-2500) cm⁻¹, v NH₂ sel
1640 cm⁻¹, v CO (S-CO-N)
1600 cm⁻¹, v C=C (aromatique)

### STADE C:

### N[2-(6-METHOXY 3-BENZOTHIAZOLINONYL) ETHYL]ACETAMIDE

Procéder comme dans l'exemple 1, stade C en remplaçant le chlorhydrate de 2-(6-méthoxy 3-benzoxazolinonyl) éthylamine par le chlorhydrate de 2-(6-méthoxy 3-benzothiazolinonyl)éthylamine obtenu dans le stade B.
Solvant de recristallisation : Toluène
Rendement : 80%
Point de fusion : 152-154°C 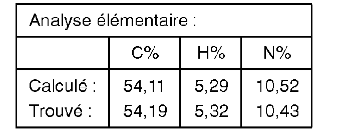
Spectrométrie dans l'infrarouge :
3240 cm⁻¹, v N-H
(3060-2840) cm⁻⁷, v C-H
1675 cm⁻¹, v CO (S-CO-N)
1650 cm⁻¹, v CO (amide)
1580 cm⁻¹, v C=C (aromatique)
Spectrométrie de Résonance Magnétique Nucléaire (CDCl₃) :
1,94 ppm, (s, 3H), COCH₃
3,80 ppm, (s, 3H), OCH₃
6,10 ppm, (signal, 1H), NH
6,80 ppm, (dd, 1 H), H₅
7,00 ppm, (d, 1 H), H₇
7,20 ppm, (d, 1 H), H₄

### EXEMPLE 31 :

### N[2-(5-METHOXY 3-BENZOTHIAZOLINONYL) ETHYL]ACETAMIDE

### STADE A

### (5-METHOXY 3-BENZOTHIAZOLINONYL)ACETONITRILE

Procéder comme dans l'exemple 1, stade A en remplaçant la 6-méthoxy benzoxazolinone par la 5-méthoxy benzothiazolinone.
Solvant de recristallisation : alcool à 95°
Rendement : 93%
Point de fusion : 154-156°C 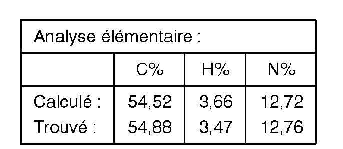
Spectrométrie dans l'infrarouge :
(3080-2940) cm⁻¹, v C-H
1680 cm⁻¹, v CO (S-CO-N)
(1630-1580) cm⁻¹, v C=C (aromatique)
Spectrométrie de Résonance Magnétique Nucléaire (CDCl₃) :
3,85 ppm, (s, 3H), OCH₃
4,75 ppm, (s, 2H), CH₂-CN
6,75 ppm, (massif, 2H), H₄, H₆
7,30 ppm, (d, 1 H), H₇

### STADE B:

### CHLORHYDRATE DE 2-(5-METHOXY 3-BENZOTHIAZOLINONYL)ETHYLAMINE

Procéder comme dans l'exemple 1, stade B en remplaçant le (6-méthoxy 3-benzoxazolinonyl)acétonitrile par le (5-méthoxy 3-benzothiazolinonyl) acétonitrile obtenu dans le stade A.
Solvant de recristallisation : alcool à 95°
Rendement : 86%
Point de fusion : >260°C
Spectrométrie dans l'infrarouge :
(3100-2500) cm⁻¹, v NH₂ sel
1640 cm-¹, v CO (S-CO-N)
1590 cm⁻¹, v C=C (aromatique)
Spectrométrie de Résonance Magnétique Nucléaire (CDCl₃) :
3,75 ppm, (s, 3H), OCH₃
6,80 ppm, (dd, 1 H), H₆
7,25 ppm, (d, 1 H), H₄
7,50 ppm, (d, 1 H), H₇
8,50 ppm, (signal, 2H), NH₂ disparaît dans D₂0

### STADE C:

### N[2-(5-METHOXY 3-BENZOTHIAZOLINONYL) ETHYL]ACETAMIDE

Procéder comme dans l'exemple 1, stade B en remplaçant le chlorhydrate de 2-(6-méthoxy 3-benzoxazolinonyl) éthylamine par le chlorhydrate 2-(5-méthoxy 3-benzothiazolinonyl)éthylamine obtenu dans le stade B.
Solvant de recristallisation : Toluène
Rendement : 86%
Point de fusion : 120-122°C 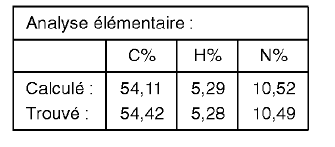
Spectrométrie dans l'infrarouqe :
3280 cm⁻¹, v N-H amide
(3080-2840) cmv, v C-H
1675 cm⁻¹, v CO(S-CO-N)
1650 cm⁻¹, v CO (amide)
1610 cm⁻¹, v C=C (aromatique)
Spectrométrie de Résonance Magnétique Nucléaire (CDCl₃) :
1,93 ppm,(s, 3H), COCH₃
3,55 ppm, (multiplet, 2H), CH₂-CH₂-NHCOCH₃
3,80 ppm, (s, 3H), OCH₃
4,15 ppm, (t, 2H), CH₂-CH₂-NHCOCH₃
6,10 ppm, (signal, 1H), NH
6,75 ppm, (dd, 1 H), H₆
6,90 ppm, (d, 1 H), H4
7,30 ppm, (d, 1 H), H₇

### EXEMPLE 32 :

### N[2-(5-METHOXY 3-BENZOTHIAZOLINONYL) ETHYL] ISOBUTYRAMIDE

Procéder comme dans l'exemple 31 en remplaçant au stade C le chlorure d'acétyle par le chlorure d'isobutyryle. Solvant de recristallisation : Toluène-Cyclohexane
Rendement : 87%
Point de fusion : 158-159°C 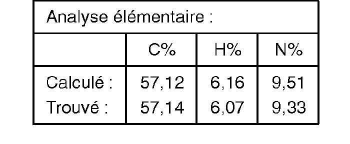
Spectrométrie dans l'infrarouge :
3280 cm⁻¹, v N-H
(3080-2840) cm⁻¹, v C-H
1675 cm⁻¹, v CO(S-CO-N)
1640 cm⁻¹, v CO (amide)
1600 cm⁻¹, v C=C (aromatique)
Spectrométrie de Résonance Magnétique Nucléaire (CDCl₃) :
1,10 ppm,(s, 6H), CH(CH₃)₂
2,25 ppm, (multiplet, 1H), CH
3,58 ppm, (multiplet, 2H), CH₂NHCO
3,85 ppm, (s, 3H), OCH₃
4,10 ppm, (t, 2H), CH₂CH₂NHCO
6,15 ppm, (signal, 1H), NH
6,70 ppm, (dd, 1 H), H₆
6,80 ppm, (d, 1 H), H₄
7,26 ppm, (d, 1 H), H₇

### EXEMPLE 33 :

### N[2-(6-METHOXY 3-BENZOTHIAZOLINONYL) ETHYL] ISOBUTYRAMIDE

Procéder comme dans l'exemple 31 en remplaçant au stade C le chlorure d'acétyle par le chlorure d'isobutyryle. Solvant de recristallisation : Toluène-Cyclohexane
Rendement : 85%
Point de fusion : 136-138°C 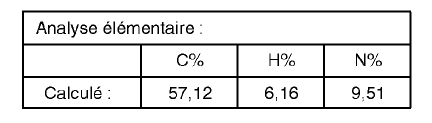 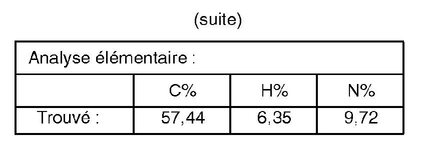
Spectrométrie dans l'infrarouge :
3300 cm⁻¹, v N-H
(3080-2840) cm⁻¹, v C-H
1680 cm⁻¹, v CO(S-CO-N)
1640 cm⁻¹, v CO (amide)
1600 cm⁻¹, v C=C (aromatique)
Spectrométrie de Résonance Magnétique Nucléaire (CDCl₃):
1,10 ppm,(d, 6H), CH(CH₃)₂
2,25 ppm, (multiplet, 1H), CH
3,80 ppm, (s, 3H), OCH₃
6,10 ppm, (signal, 1H), NH
6,85 ppm, (dd, 1 H), H₅
7,00 ppm, (d, 1 H), H₇
7,20 ppm, (d, 1 H), H₄

### EXEMPLES 34 A 58 :

En procédant comme dans les exemples 5 à 29, mais en remplaçant au stade C le chlorhydrate de 2-(6-méthoxy 3-benzoxazolinonyl)éthylamine par le chlorhydrate de 2-(6-méthoxy 3-benzothiazolinonyl) éthylamine, on obtient respectivement :

### EXEMPLE 34:

N[2-(6-METHOXY 3-BENZOTHIAZOLINONYL) ETHYL]PHENYL ACETAMIDE

### EXEMPLE 35 :

N[2-(6-METHOXY 3-BENZOTHIAZOLINONYL) ETHYL](2-OXO 1-PYRROLIDINYL)ACETAMIDE

### EXEMPLE 36:

N[2-(6-METHOXY 3-BENZOTHIAZOLINONYL) ETHYL](2-OXO 1-PYRROLIDINYL)4-CHLORO BUTYRAMIDE

### EXEMPLE 37:

N[2-(6-METHOXY 3-BENZOTHIAZOLINONYL) ETHYL]2-PYRROLIDINONE

### EXEMPLE 38:

N[2-(6-METHOXY 3-BENZOTHIAZOLINONYL) ETHYL]2-BROMOACETAMIDE

### EXEMPLE 39:

N[2-(6-METHOXY 3-BENZOTHIAZOLINONYL) ETHYL]2-MORPHOLINOACETAMIDE

### EXEMPLE 40:

N[2-(6-METHOXY 3-BENZOTHIAZOLINONYL) ETHYL ]2-{4-[2,3,4-TRIMETHOXY PHENYL METHYL] 1-PIPERAZINYL}ACETAMIDE

### EXEMPLE 41 :

N[2-(6-METHOXY 3-BENZOTHIAZOLINONYL) ETHYL]N METHYL ACETAMIDE

### EXEMPLE 42 :

N[2-(6-METHOXY 3-BENZOTHIAZOLINONYL) ETHYL] BENZAMIDE

### EXEMPLE 43 :

### N[2-(6-METHOXY 3-BENZOTHIAZOLINONYL) ETHYL]PARATOLUOYLCARBOXAMIDE

### EXEMPLE 44 :

N[2-(6-METHOXY 3-BENZOTHIAZOLINONYL) ETHYL] 4-FLUOROBENZAMIDE

### EXEMPLE 45 :

N[2-(6-METHOXY 3-BENZOTHIAZOLINONYL) ETHYL] 3-TRIFLUOROMETHYL BENZAMIDE

### EXEMPLE 46 :

N[2-(6-METHOXY 3-BENZOTHIAZOLINONYL) ETHYL] 3,5-DICHLOROBENZAMIDE

### EXEMPLE 47 :

N[2-(6-METHOXY 3-BENZOTHIAZOLINONYL) ETHYL]ISONICOTINAMIDE

### EXEMPLE 48 :

N[2-(6-METHOXY 3-BENZOTHIAZOLINONYL) ETHYL] 2-INDOLYL CARBOXAMIDE

### EXEMPLE 49 :

N[2-(6-METHOXY 3-BENZOTHIAZOLINONYL) ETHYL] 2-BENZYLAMINOACETAMIDE

### EXEMPLE 50:

N[2-(6-METHOXY 3-BENZOTHIAZOLINONYL)ETHYL]2-(N',N' DIETHYL AMINO ACETAMIDE

### EXEMPLE 51 :

N[2-(6-METHOXY 3-BENZOTHIAZOLINONYL)ETHYL]2-AMINOACETAMIDE

### EXEMPLE 52 :

N[2-(6-METHOXY 3-BENZOTHIAZOLINONYL) ETHYL]2-[4-(4-FLUOROPHENYL)PIPERAZIN-1-YL]ACETAMIDE

### EXEMPLE 53 :

N[2-(6-METHOXY 3-BENZOTHIAZOLINONYL)ETHYL]2-[4-(3-TRIFLUOROMETHYLPHENYL) PIPERAZIN-1-YL] ACETAMIDE

### EXEMPLE 54:

N[2-(6-METHOXY 3-BENZOTHIAZOLINONYL) ETHYL]CYCLOHEXANE CARBOXAMIDE

### EXEMPLE 55 :

N[2-(6-METHOXY 3-BENZOTHIAZOLINONYL) ETHYL] FORMAMIDE

### EXEMPLE 56:

N[2-(6-METHOXY 3-BENZOTHIAZOLINONYL) ETHYL]CYCLOPROPANE CARBOXAMIDE

### EXEMPLE 57:

N[2-(6-METHOXY 3-BENZOTHIAZOLINONYL) ETHYL] VALERAMIDE

### EXEMPLE 58:

N[2-(6-METHOXY 3-BENZOTHIAZOLINONYL) ETHYL] CYCLOBUTANE CARBOXAMIDE

En remplaçant dans les exemples 34 à 58 la 2-(6-méthoxy 3-benzothiazolinonyl)éthylamine par la 2-(5-méthoxy 3-benzothiazolinonyl) éthylamine, on obtient les produits des exemples précédents méthoxylés respectivement en position 5 au lieu des dérivés méthoxylés en position 6.

### EXEMPLE 59 :

### N[2-(6-METHOXY 4-BENZOXAZINONYL) ETHYL] ACETAMIDE

En remplaçant dans l'exemple 1, stade A la 6-méthoxy benzoxazolinone par la 6-méthoxy benzoxazinone, on obtient respectivement :

### STADE A :

### 2-(6-METHOXY 4-BENZOXAZINONYL) ACETONITRILE

### STADE B:

CHLORHYDRATE DE 2-(6-METHOXY 4-BENZOXAZINONYL) ETHYLAMINE

STADE C:

### N[3-(6-METHOXY 3-BENZOXAZINONYL)ETHYL] ACETAMIDE

### EXEMPLE 60 :

### N[2-(7-METHOXY 4-BENZOXAZINONYL) ETHYL] ACETAMIDE

En procédant comme dans l'exemple 1, mais en remplaçant la 6-méthoxy benzoxazolinone par la 7-méthoxy benzoxazinone, on obtient respectivement :
. 2-(7-METHOXY 4-BENZOXAZINONYL) ACETONITRILE
. CHLORHYDRATE DE 2-(7-METHOXY 4-BENZOXAZINONYL)ETHYLAMINE
N[2-(7-METHOXY 4-BENZOXAZINONYL) ETHYL] ACETAMIDE

### EXEMPLE 61 :

### N-[2-(3-METHYL 6-BENZOXAZOLINONYL) ETHYL]ACETAMIDE

Dissoudre 0,02 mole de chlorhydrate de 2-(3-méthyl 6-benzoxazolinonyl)éthylamine décrite dans la demande de brevet EP 0110781 dans un mélange eau/chloroforme. Ajouter 0,02 mole de carbonate de potassium et laisser agiter pendant une heure.

Refroidir dans un bac à glace puis ajouter 0,022 mole de chlorure d'acétyle. Maintenir l'agitation 30 minutes. La phase chloroformique est lavée à l'eau, séchée sur chlorure de calcium et portée à sec. Le résidu est recristallisé dans le toluène.
Rendement : 76%
Point de fusion : 150°C
Spectrométrie dans l'infrarouge :
2260 cm⁻¹, v N-H
(3080-2880) cm⁻¹, v C-H
1775 cm⁻¹, v CO(O-CO-N)
1640 cm⁻¹, v CO (amide)
1615 cm⁻¹, v C=C (aromatique)
Spectrométrie de Résonance Magnétique Nucléaire (CDCl₃) :
1,96 ppm,(s, 3H), COCH₃
2,84 ppm, (t, 2H), CH₂CH₂NH
3,43 ppm, (s, 3H), NCH₃
3,50 ppm, (multiplet, 2H), CH₂-CH₂NH
5,60 ppm, (signal, 1 H), NH
6,80 ppm, (d, 1 H), H₄
7,00 ppm, (massif, 2H), H₅, H₇

### EXEMPLE 62 :

### N-[2-(3-METHYL 6-BENZOTHIAZOLINONYL) ETHYL]ACETAMIDE

Remplacer dans l'exemple 61 le chlorhydrate de 2-(3-méthyl 6-benzoxazolinonyl)éthylamine par le chlorhydrate de 2-(3-méthyl 6-benzothiazolinonyl)éthylamine décrit dans la demande de brevet français 90.11866, on obtient le produit du titre.
Rendement : 79%
Point de fusion : 134-136°C
Spectrométrie dans l'infrarouge :
3280 cm⁻¹, v N-H
(3080-2860) cm⁻¹, v C-H
1670 cm⁻¹, v CO(S-CO-N)
1630 cm⁻¹, v CO (amide)
1600 cm⁻¹, v C=C (aromatique)
Spectrométrie de Résonance Magnétique Nucléaire (CDCl₃) :
1,96 ppm,(s, 3H), COCH₃
2,85 ppm, (t, 2H), CH₂CH₂NHCOCH₃
3,50 ppm, (massif, 5H), CH₂NHCOCH₃ et NCH₃
5,50 ppm, (signal, 1 H), NH
6,95 ppm, (d, 1 H), H₄
7,20 ppm, (massif, 2H), H₅, H₇

### EXEMPLE 63 :

### N-[2-(3-METHYL 6-BENZOXAZOLINONYL) ETHYL]ISOBUTYRAMIDE

En procédant comme dans l'exemple 61, mais en remplaçant le chlorure d'acétyle par le chlorure d'isobutyryle, on obtient le produit du titre.
Spectrométrie dans l'infrarouge :
   1760 cm⁻¹, v CO (OCON)
   1640 cm⁻¹, v CO (amide)

### EXEMPLE 64 :

### N[2-(3-METHYL 6-BENZOXAZOLINONYL) ETHYL]PHENYLACETAMIDE

En procédant comme dans l'exemple 61, mais en remplaçant le chlorure d'acétyle par le chlorure de phénacétyle, on obtient le produit du titre.

### EXEMPLE 65 :

### N[2-(3-METHYL 6-BENZOXAZOLINONYL) ETHYL] (2-OXO 1-PYRROLIDINYL)ACETAMIDE

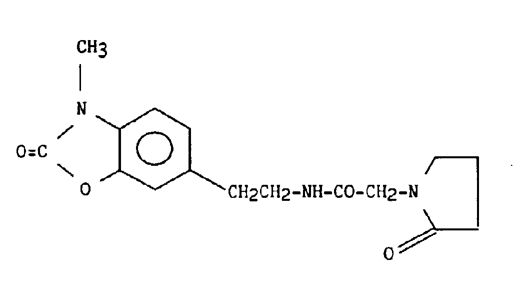

Procéder comme dans l'exemple 6, en remplaçant la 3-(2-aminoéthyl)6-méthoxy benzoxazolinone par la 2-(3-méthyl 6-benzoxazolinonyl)éthylamine, on obtient le produit du titre.

### EXEMPLES 66 A 88 :

En procédant comme dans les exemples 7 à 29, mais en remplaçant le chlorhydrate de 2-(6-méthoxy 3-benzoxazolinonyl)éthylamine par le chlorhydrate de 2-(3-méthyl 6-benzoxazolinonyl)éthylamine, on obtient :

### EXEMPLE 66:

N[2-(3-METHYL 6-BENZOXAZOLINONYL) ETHYL]4-CHLORO BUTYRAMIDE

### EXEMPLE 67:

N[2-(3-METHYL 6-BENZOXAZOLINONYL) ETHYL]2-PYRROLIDINONE

### EXEMPLE 68:

N[2-(3-METHYL 6-BENZOXAZOLINONYL) ETHYL]2-BROMOACETAMIDE

### EXEMPLE 69:

N[2-(3-METHYL 6-BENZOXAZOLINONYL) ETHYL]2-MORPHOLINOACETAMIDE

### EXEMPLE 70:

N[2-(3-METHYL6-BENZOXAZOLINONYL) ETHYL]2-{4-[2,3,4-TRIMETHOXYPHENYL METHYL] 1-PIPERAZINYL} ACETAMIDE

### EXEMPLE 71 :

N[2-(3-METHYL 6-BENZOXAZOLINONYL) ETHYL]N METHYL ACETAMIDE

### EXEMPLE 72 :

N[2-(3-METHYL 6-BENZOXAZOLINONYL) ETHYL] BENZAMIDE

### EXEMPLE 73:

N[2-(3-METHYL 6-BENZOXAZOLINONYL) ETHYL]PARATOLUOYLCARBOXAMIDE

### EXEMPLE 74:

N[2-(3-METHYL 6-BENZOXAZOLINONYL) ETHYL] 4-FLUOROBENZAMIDE

### EXEMPLE 75:

N[2-(3-METHYL 6-BENZOXAZOLINONYL) ETHYL] 3-TRIFLUOROMETHYL BENZAMIDE

### EXEMPLE 76:

N[2-(3-METHYL 6-BENZOXAZOLINONYL) ETHYL] 3,5-DICHLOROBENZAMIDE

### EXEMPLE 77:

N[2-(3-METHYL 6-BENZOXAZOLINONYL) ETHYL]ISONICOTINAMIDE

### EXEMPLE 78:

N[2-(3-METHYL 6-BENZOXAZOLINONYL) ETHYL] INDOL-2-YL CARBOXAMIDE

### EXEMPLE 79:

N[2-(3-METHYL 6-BENZOXAZOLINONYL) ETHYL] 2-BENZYLAMINOACETAMIDE

### EXEMPLE 80:

N[2-(3-METHYL6-BENZOXAZOLINONYL) ETHYL]2-(N',N' DIETHYL)AMINOACETAMIDE

### EXEMPLE 81 :

N[2-(3-METHYL6-BENZOXAZOLINONYL)ETHYL]2-AMINOACETAMIDE

### EXEMPLE 82 :

N[2-(3-METHYL6-BENZOXAZOLINONYL) ETHYL]2-[4-(4-FLUOROPHENYL)1-PIPERAZINYL]ACETAMIDE

### EXEMPLE 83 :

N[2-(3-METHYL 6-BENZOXAZOLINONYL)ETHYL]2-[4-(3-TRIFLUOROMETHYLPHENYL)1-PIPERAZINYL] ACETAMIDE

### EXEMPLE 84:

N[2-(3-METHYL 6-BENZOXAZOLINONYL)ETHYL]CYCLOHEXANE CARBOXAMIDE

### EXEMPLE 85 :

N[2-(3-METHYL 6-BENZOXAZOLINONYL)ETHYL] FORMAMIDE

### EXEMPLE 86:

N[2-(3-METHYL6-BENZOXAZOLINONYL)ETHYL]CYCLOPROPANE CARBOXAMIDE

### EXEMPLE 87 :

N[2-(3-METHYL 6-BENZOXAZOLINONYL)ETHYL] VALERAMIDE

### EXEMPLE 88 :

N[2-(3-METHYL 6-BENZOXAZOLINONYL)ETHYL]CYCLOBUTANE CARBOXAMIDE

### EXEMPLES 89 A 113:

En procédant comme dans les exemples 5 à 20, mais en remplaçant le chlorhydrate de 2-(6-méthoxy 3-benzoxazolinonyl)éthylamine par le chlorhydrate de 2-(3-méthyl 6-benzothiazolinonyl) éthylamine on obtient :

### EXEMPLE 89:

N[2-(3-METHYL 6-BENZOTHIAZOLINONYL) ETHYL]PHENYL ACETAMIDE

### EXEMPLE 90:

N[2-(3-METHYL 6-BENZOTHIAZOLINONYL) ETHYL](2-OXO 1-PYRROLIDINYL)ACETAMIDE

### EXEMPLE 91:

N[2-(3-METHYL 6-BENZOTHIAZOLINONYL) ETHYL]4-CHLORO BUTYRAMIDE

### EXEMPLE 92:

N[2-(3-METHYL 6-BENZOTHIAZOLINONYL) ETHYL]2-PYRROLIDINONE

### EXEMPLE 93:

N[2-(3-METHYL6-BENZOTHIAZOLINONYL) ETHYL]2-BROMOACETAMIDE

### EXEMPLE 94:

N[2-(3-METHYL 6-BENZOTHIAZOLINONYL) ETHYL]2-MORPHOLINOACETAMIDE

### EXEMPLE 95:

N[2-(3-METHYL 6-BENZOTHIAZOLINONYL) ETHYL]2-{4-[2,3,4-TRIMETHOXYPHENYL METHYL] 1-PIPERAZINYL}ACETAMIDE

### EXEMPLE 96:

N[2-(3-METHYL 6-BENZOTHIAZOLINONYL) ETHYL]N METHYL ACETAMIDE

### EXEMPLE 97:

N[2-(3-METHYL 6-BENZOTHIAZOLINONYL) ETHYL] BENZAMIDE

### EXEMPLE 98:

N[2-(3-METHYL6-BENZOTHIAZOLINONYL) ETHYL]PARATOLUOYLCARBOXAMIDE

### EXEMPLE 99:

N[2-(3-METHYL 6-BENZOTHIAZOLINONYL) ETHYL] 4-FLUOROBENZAMIDE

### EXEMPLE 100:

N[2-(3-METHYL 6-BENZOTHIAZOLINONYL) ETHYL] 3-TRIFLUOROMETHYL BENZAMIDE

### EXEMPLE 101 :

N[2-(3-METHYL 6-BENZOTHIAZOLINONYL) ETHYL] 3,5-DICHLOROBENZAMIDE

### EXEMPLE 102:

N[2-(3-METHYL 6-BENZOTHIAZOLINONYL) ETHYL]ISONICOTINAMIDE

### EXEMPLE 103:

N[2-(3-METHYL6-BENZOTHIAZOLINONYL) ETHYL] INDOL-2-YL CARBOXAMIDE

### EXEMPLE 104:

N[2-(3-METHYL6-BENZOTHIAZOLINONYL) ETHYL] 2-BENZYLAMINOACETAMIDE

### EXEMPLE 105:

N[2-(3-METHYL6-BENZOTHIAZOLINONYL) ETHYL]2-(N',N' DIETHYL)AMINO ACETAMIDE

### EXEMPLE 106:

N[2-(3-METHYL6-BENZOTHIAZOLINONYL)ETHYL]2-AMINOACETAMIDE

### EXEMPLE 107:

N[2-(3-METHYL 6-BENZOTHIAZOLINONYL) ETHYL]2-[4-(4-FLUOROPHENYL)PIPERAZIN-1-YL]ACETAMIDE

### EXEMPLE 108:

N[2-(3-METHYL 6-BENZOTHIAZOLINONYL)ETHYL]2-[4-(3-TRIFLUOROMETHYLPHENYL) PIPERAZIN-1-YL] ACETAMIDE

### EXEMPLE 109:

N[2-(3 METHYL 6-BENZOTHIAZOLINONYL)ETHYL]CYCLOHEXANE CARBOXAMIDE

### EXEMPLE 110:

N[2-(3-METHYL 6-BENZOTHIAZOLINONYL)ETHYL] FORMAMIDE

### EXEMPLE 111 .

N[2-(3-METHYL 6-BENZOTHIAZOLINONYL)ETHYL]CYCLOPROPANE CARBOXAMIDE

### EXEMPLE 112:

N[2-(3-METHYL 6-BENZOTHIAZOLINONYL)ETHYL] VALERAMIDE

### EXEMPLE 113:

N[2-(3-METHYL 6-BENZOTHIAZOLINONYL)ETHYL] CYCLOBUTANE CARBOXAMIDE

En remplaçant dans les exemples 89 à 113 la 2-(3-méthyl-6 benzothiazolinonyl)éthylamine par la 2-(3-méthyl 7-benzothiazolinonyl) éthylamine obtenue dans la demande de brevet EP 174811 on obtient les produits isomères des précedents, la chaîne latérale étant en position 7 et non plus en position 6 :

### EXEMPLE 114:

N[2-(3-METHYL 7-BENZOTHIAZOLINONYL) ETHYL]PHENYL ACETAMIDE

### EXEMPLE 115:

N[2-(3-METHYL 7-BENZOTHIAZOLINONYL) ETHYL](2-OXO 1-PYRROLIDINYL)ACETAMIDE

### EXEMPLE 116:

N[2-(3-METHYL 7-BENZOTHIAZOLINONYL) ETHYL]4-CHLORO BUTYRAMIDE

### EXEMPLE 117:

N[2-(3-METHYL 7-BENZOTHIAZOLINONYL) ETHYL]2-PYRROLIDINONE

### EXEMPLE 118:

N[2-(3-METHYL 7-BENZOTHIAZOLINONYL) ETHYL]2-BROMOACETAMIDE

### EXEMPLE 119:

N[2-(3-METHYL 7-BENZOTHIAZOLINONYL) ETHYL]2-MORPHOLINOACETAMIDE

### EXEMPLE 120:

N[2-(3-METHYL 7-BENZOTHIAZOLINONYL) ETHYL]2-{4-[2,3,4-TRIMETHOXYPHENYL METHYL] 1-PIPERAZINYL}ACETAMIDE

### EXEMPLE 121:

N[2-(3-METHYL 7-BENZOTHIAZOLINONYL) ETHYL]N METHYL ACETAMIDE

### EXEMPLE 122:

N[2-(3-METHYL 7-BENZOTHIAZOLINONYL) ETHYL] BENZAMIDE

### EXEMPLE 123:

N[2-(3-METHYL 7-BENZOTHIAZOLINONYL) ETHYL]PARATOLUOYLCARBOXAMIDE

### EXEMPLE 124:

N[2-(3-METHYL 7-BENZOTHIAZOLINONYL) ETHYL] 4-FLUOROBENZAMIDE

### EXEMPLE 125:

N[2-(3-METHYL 7-BENZOTHIAZOLINONYL) ETHYL] 3-TRIFLUOROMETHYL BENZAMIDE

### EXEMPLE 126:

N[2-(3-METHYL 7-BENZOTHIAZOLINONYL) ETHYL] 3,5-DICHLOROBENZAMIDE

### EXEMPLE 127:

N[2-(3-METHYL 7-BENZOTHIAZOLINONYL) ETHYL]ISONICOTINAMIDE

### EXEMPLE 128:

N[2-(3-METHYL 7-BENZOTHIAZOLINONYL) ETHYL]2-INDOLYL CARBOXAMIDE

### EXEMPLE 129:

N[2-(3-METHYL 7-BENZOTHIAZOLINONYL) ETHYL] 2-(BENZYLAMINO)ACETAMIDE

### EXEMPLE 130:

N[2-(3-METHYL 7-BENZOTHIAZOLINONYL)ETHYL]2-[(N',N'DIETHYL)AMINO]ACETAMIDE

### EXEMPLE 131 :

N[2-(3-METHYL 7-BENZOTHIAZOLINONYL)ETHYL]2-AMINOACETAMIDE

### EXEMPLE 132:

N[2-(3-METHYL 7-BENZOTHIAZOLINONYL) ETHYL]2-[4-(4-FLUOROPHENYL)PIPERAZIN-1-YL]ACETAMIDE

### EXEMPLE 133:

N[2-(3-METHYL 7-BENZOTHIAZOLINONYL)ETHYL]2-[4-(3-TRIFLUOROMETHYLPHENYL) 1-PIPERAZINYL] ACETAMIDE

### EXEMPLE 134:

N[2-(3-METHYL 7-BENZOTHIAZOLINONYL)ETHYL]CYCLOHEXANE CARBOXAMIDE

### EXEMPLE 135:

N[2-(3-METHYL 7-BENZOTHIAZOLINONYL)ETHYL] FORMAMIDE

### EXEMPLE 136:

N[2-(3-METHYL 7-BENZOTHIAZOLINONYL)ETHYL]CYCLOPROPANE CARBOXAMIDE

### EXEMPLE 137:

N[2-(3-METHYL 7-BENZOTHIAZOLINONYL)ETHYL] VALERAMIDE

### EXEMPLE 138:

N[2-(3-METHYL 7-BENZOTHIAZOLINONYL)ETHYL] CYCLOBUTANE CARBOXAMIDE

### EXEMPLES 139 A 162 :

En procédant comme dans les exemples 5 à 26, mais en remplaçant au stade C le chlorhydrate de 2-(6-méthoxy 3-benzoxazolinonyl)éthylamine par le chlorhydrate de 2-(4-méthyl 7-benzoxazinonyl)éthylamine, on obtient respectivement :

### EXEMPLE 139:

N[2-(4-METHYL 7-BENZOXAZINONYL) ETHYL]PHENYL ACETAMIDE

### EXEMPLE 140:

N[2-(4-METHYL 7-BENZOXAZINONYL) ETHYL](2-OXO 1-PYRROLIDINYL)ACETAMIDE

### EXEMPLE 141:

N[2-(4-METHYL 7-BENZOXAZINONYL) ETHYL]4-CHLORO BUTYRAMIDE

### EXEMPLE 142:

N[2-(4-METHYL 7-BENZOXAZINONYL) ETHYL]2-PYRROLIDINONE

### EXEMPLE 143:

N[2-(4-METHYL 7-BENZOXAZINONYL) ETHYL]2-BRONOACETAMIDE

### EXEMPLE 144:

N[2-(4-METHYL 7-BENZOXAZINONYL) ETHYL]2-MORPHOLINOACETAMIDE

### EXEMPLE 145:

N[2-(4-METHYL 7-BENZOXAZINONYL) ETHYL]2-{4-[2,3,4-TRIMETHOXYPHENYL METHYL] 1-PIPERAZINYL} ACETAMIDE

### EXEMPLE 146:

N[2-(4-METHYL 7-BENZOXAZINONYL) ETHYL]N METHYL ACETAMIDE

### EXEMPLE 147:

N[2-(4-METHYL 7-BENZOXAZINONYL) ETHYL] BENZAMIDE

### EXEMPLE 148:

N[2-(4-METHYL 7-BENZOXAZINONYL) ETHYL]PARATOLUOYLCARBOXAMIDE

### EXEMPLE 149:

N[2-(4-METHYL 7-BENZOXAZINONYL) ETHYL] 4-FLUOROBENZAMIDE

### EXEMPLE 150:

N[2-(4-METHYL 7-BENZOXAZINONYL) ETHYL] 3-TRIFLUOROMETHYL BENZAMIDE

### EXEMPLE 151:

N[2-(4-METHYL 7-BENZOXAZINONYL) ETHYL] 3,5-DICHLOROBENZAMIDE

### EXEMPLE 152:

N[2-(4-METHYL 7-BENZOXAZINONYL) ETHYL]ISONICOTINAMIDE

### EXEMPLE 153:

N[2-(4-METHYL 7-BENZOXAZINONYL) ETHYL] INDOL-2-YL CARBOXAMIDE

### EXEMPLE 154:

N[2-(4-METHYL 7-BENZOXAZINONYL) ETHYL] 2-BENZYLAMINOACETAMIDE

### EXEMPLE 155:

N[2-(4-METHYL 7-BENZOXAZINONYL) ETHYL]2-[(N',N' DIETHYL)AMINO ]ACETAMIDE

### EXEMPLE 156:

N[2-(4-METHYL 7-BENZOXAZINONYL)ETHYL]2-AMINOACETAMIDE

### EXEMPLE 157:

N[2-(4-METHYL 7-BENZOXAZINONYL) ETHYL]2-[4-(4-FLUOROPHENYL)PIPERAZIN-1-YL]ACETAMIDE

### EXEMPLE 158:

N[2-(4-METHYL 7-BENZOXAZINONYL)ETHYL]2-[4-(3-TRIFLUOROMETHYLPHENYL)1-PIPERAZINYL] ACETAMIDE

### EXEMPLE 159:

N[2-(4-METHYL 7-BENZOXAZINONYL)ETHYL]CYCLOHEXANE CARBOXAMIDE

### EXEMPLE 160:

N[2-(4-METHYL 7-BENZOXAZINONYL)ETHYL] FORMAMIDE

### EXEMPLE 161 :

N[2-(4-METHYL 7-BENZOXAZINONYL)ETHYL ]CYCLOPROPANE CARBOXAMIDE

### EXEMPLE 162:

N[2-(4-METHYL 7-BENZOXAZINONYL)ETHYL] VALERAMIDE

### EXEMPLE 163:

N[2-(4-METHYL 7-BENZOXAZINONYL) ETHYL] CYCLOBUTANE CARBOXAMIDE

En procédant, comme dans les exemples 5 à 29 mais en remplaçant au stade C le Chlorhydrate de 2-(6-méthoxy 3-benzoxazolinonyl) éthylamine par le chlorhydrate de 2-(4-méthyl 6-benzoxazinonyl) éthylamine décrit dans la demande de brevet FR 90.11.866, on obtient:

### EXEMPLE 164:

N[2-(4-METHYL 6-BENZOXAZINONYL) ETHYL]PHENYL ACETAMIDE

### EXEMPLE 165:

N[2-(4-METHYL 6-BENZOXAZINONYL) ETHYL](2-OXO 1-PYRROLIDINYL)ACETAMIDE

### EXEMPLE 166:

N[2-(4-METHYL 6-BENZOXAZINONYL) ETHYL]4-CHLORO BUTYRAMIDE

### EXEMPLE 167:

N[2-(4-METHYL 6-BENZOXAZINONYL) ETHYL]2-PYRROLIDINONE

### EXEMPLE 168:

N[2-(4-METHYL 6-BENZOXAZINONYL) ETHYL]2-BROMOACETAMIDE

### EXEMPLE 169:

N[2-(4-METHYL 6-BENZOXAZINONYL) ETHYL]2-MORPHOLINOACETAMIDE

### EXEMPLE 170:

N[2-(4-METHYL 6-BENZOXAZINONYL) ETHYL]2-(4-[2,3,4-TRIMETHOXYPHENYL) METHYL] 1-PIPERAZINYL) ACETAMIDE

### EXEMPLE 171:

N[2-(4-METHYL 6-BENZOXAZOLINONYL) ETHYL]N METHYL ACETAMIDE

### EXEMPLE 172:

N[2-(4-METHYL 6-BENZOXAZOLINONYL) ETHYL] BENZAMIDE

### EXEMPLE 173:

N[2-(4-METHYL 6-BENZOXAZINONYL) ]PARATOLUOYLCARBOXAMIDE

### EXEMPLE 174:

N[2-(4-METHYL 6-BENZOXAZOLINONYL) ETHYL] 4-FLUOROBENZAMIDE

### EXEMPLE 175:

N[2-(4-METHYL 6-BENZOXAZINONYL) ETHYL] 3-TRIFLUOROMETHYL BENZAMIDE

### EXEMPLE 176:

N[2-(4-METHYL 6-BENZOXAZOLINONYL) ETHYL] 3,5-DICHLOROBENZAMIDE

### EXEMPLE 177:

N[2-(4-METHYL 6-BENZOXAZOLINONYL) ETHYL]ISONICOTINAMIDE

### EXEMPLE 178:

N[2-(4-METHYL 6-BENZOXAZINONYL) ETHYL] INDOL-2-YL CARBOXAMIDE

### EXEMPLE 179:

N[2-(4-METHYL 6-BENZOXAZOLINONYL) ETHYL] 2-BENZYLAMINOACETAMIDE

### EXEMPLE 180:

N[2-(4-METHYL 6-BENZOXAZOLINONYL) ETHYL]2-(N',N' DIETHYL)AMINO ACETAMIDE

### EXEMPLE 181 :

N[2-(4-METHYL 6-BENZOXAZOLINONYL)ETHYL]2-AMINOACETAMIDE

### EXEMPLE 182:

N[2-(4-METHYL 6-BENZOXAZOLINONYL) ETHYL]2-[4-(4-FLUOROPHENYL)1-PIPERAZINYL]ACETAMIDE

### EXEMPLE 183:

N[2-(4-METHYL 6-BENZOXAZINONYL)METHYL]2-[4-(3-TRIFLUOROMETHYLPHENYL)1-PIPERAZINYL] ACETAMIDE

### EXEMPLE 184:

N[2-(4-METHYL 6-BENZOXAZINONYL)ETHYL]CYCLOHEXANE CARBOXAMIDE

### EXEMPLE 185:

N[2-(4-METHYL 6-BENZOXAZINONYL)ETHYL] FORMAMIDE

### EXEMPLE 186:

N[2-(4-METHYL 6-BENZOXAZINONYL)ETHYL]CYCLOPROPANE CARBOXAMIDE

### EXEMPLE 187:

N[2-(4-METHYL 6-BENZOXAZINONYL)ETHYL] VALERAMIDE

### EXEMPLE 188:

N[2-(4-METHYL 6-BENZOXAZINONYL)ETHYL] CYCLOBUTANE CARBOXAMIDE

### EXEMPLE 189:

N[2-(6-METHOXY 3-BENZOXAZOLINONYL)ETHYL]2-METHYL CYCLOPROPANE CARBOXAMIDE R₂ = 2-METHYL CYCLOPROPYL

En procédant comme dans l'exemple 1, mais en remplaçant au stade C le chlorure d'acétyle par le chlorure de l'acide 2-méthyl cyclopropane carboxylique, on obtient le produit du titre.

### EXEMPLE 190:

N[2-(5-METOXY 3-BENZOXALINONYL)ETHYL]2-METHYL CYCLOPROPANE CARBOXAMIDE

En procédant comme dans l'exemple 189, mais en remplaçant au stade A la 6-méthoxy benzoxazolinone par la 5-méthoxy benzoxazolinone, on obtient le produit du titre.

### EXEMPLE 191 :

### N[2-(5-METHOXY 3-BENZOXAZOLINONYL)ETHYL]CYCLOPROPANE CARBOXAMIDE

En procédant comme dans l'exemple 1, mais en remplaçant au stade A la 6-méthoxy benzoxazolinone par la 5-méthoxy benzoxazolinone, et le chlorure d'acétyle par le chlorure de l'acide cyclopropane carboxylique, on obtient le produit du titre.
Recristallisation : toluène
Point de fusion : 150-151°C

### EXEMPLE 192:

### N[2-(5-METHOXY 3-BENZOTHIAZOLINONYL)ETHYL]CYCLOPROPANE CARBOXAMIDE

En procédant comme dans l'exemple 1, mais en remplaçant au stade A la 6-méthoxy benzoxazolinone par la 5-méthoxy benzothiazolinone, et le chlorure d'acétyle par le chlorure de l'acide cyclopropane carboxylique, on obtient le produit du titre.
Recristallisation : toluène
Point de fusion : 135-136°C

En procédant comme dans les exemples 1, 4 à 29, mais en remplaçant la 6-méthoxy benzoxazolinone par la benzoxazolinone elle même, on obtient :
N[2-(3-BENZOXAZOLINONYL) ETHYL]ACETAMIDE
N[2-(3-BENZOXAZOLINONYL) ETHYL]ISOBUTYRAMIDE
N[2-(3-BENZOXAZOLINONYL) ETHYL]PHENYLACETAMIDE
N[2-(3-BENZOXAZOLINONYL) ETHYL](2-OXO 1-PYRROLIDINYL)ACETAMIDE
N[2-(3-BENZOXAZOLINONYL) ETHYL]4-CHLOROBUTYRAMIDE
N[2-(3-BENZOXAZOLINONYL) ETHYL]2-PYRROLIDINONE
N[2-(3-BENZOXAZOLINONYL) ETHYL]2-BROMOACETAMIDE
N[2-(3-BENZOXAZOLINONYL) ETHYL]2-MORPHOLINO ACETAMIDE
N[2-(3-BENZOXAZOLINONYL) ETHYL]2-(4-[(2,3,4-TRIMETHOXYPHENYL)METHYL] 1-PIPERAZINYL} ACETAMIDE
N[2-(3-BENZOXAZOLINONYL) ETHYL]N-METHYLACETAMIDE
N[2-(3-BENZOXAZOLINONYL) ETHYL]BENZAMIDE
N[2-(3-BENZOXAZOLINONYL) ETHYL]PARATOLUOYLCARBOXAMIDE
N[2-(3-BENZOXAZOLINONYL) ETHYL]4-FLUOROBENZAMIDE
N[2-(3-BENZOXAZOLINONYL) ETHYL]2-[4-(4-FLUOROPHENYL) 1-PIPERAZINYL] ACETAMIDE
N[2-(3-BENZOXAZOLINONYL) ETHYL] 3-TRIFLUOROMETHYL BENZAMIDE
N[2-(3-BENZOXAZOLINONYL) ETHYL] 3,5-DICHLOROBENZAMIDE
N[2-(3-BENZOXAZOLINONYL) ETHYL]ISONICOTINAMIDE
N[2-(3-BENZOXAZOLINONYL) ETHYL] INDOL-2-YL CARBOXAMIDE
N[2-(3-BENZOXAZOLINONYL) ETHYL] 2-BENZYLAMINOACETAMIDE
N[2-(3-BENZOXAZOLINONYL) ETHYL]2-(N',N' DIETHYL)AMINO ACETAMIDE
N[2-(3-BENZOXAZOLINONYL)ETHYL]2-AMINOACETAMIDE
N[2-(3-BENZOXAZOLINONYL)ETHYL]2-[4-(3-TRIFLUOROMETHYLPHENYL) PIPERAZIN-1-YL] ACETAMIDE
N[2-(3-BENZOXAZOLINONYL)ETHYL]CYCLOHEXANE CARBOXAMIDE
N[2-(3-BENZOXAZOLINONYL)ETHYL] FORMAMIDE
N[2-(3-BENZOXAZOLINONYL)ETHYL]CYCLOPROPANE CARBOXAMIDE
N[2-(3-BENZOXAZOLINONYL)ETHYL] VALERAMIDE
N[2-(3-BENZOXAZOLINONYL)ETHYL] CYCLOBUTANE CARBOXAMIDE

En procédant comme dans les exemples 1, 4 à 29, mais en remplaçant la 6-méthoxy benzoxazolinone par la benzothiazolinone, on obtient:
N[2-(3-BENZOTHIAZOLINONYL) ETHYL]ACETAMIDE
N[2-(3-BENZOTHIAZOLINONYL) ETHYL]ISOBUTYRAMIDE
N[2-(3-BENZOTHIAZOLINONYL) ETHYL]PHENYLACETAMIDE
N[2-(3-BENZOTHIAZOLINONYL) ETHYL](2-OXO 1-PYRROLIDINYL)ACETAMIDE
N[2-(3-BENZOTHIAZOLINONYL) ETHYL]4-CHLOROBUTYRAMIDE
N[2-(3-BENZOTHIAZOLINONYL) ETHYL]2-PYRROLIDINONE
N[2-(3-BENZOTHIAZOLINONYL) ETHYL]2-BROMOACETAMIDE
N[2-(3-BENZOTHIAZOLINONYL) ETHYL]2-MORPHOLINO ACETAMIDE
N[2-(3-BENZOTHIAZOLINONYL) ETHYL]2-{4-[(2,3,4-TRIMETHOXYPHENYL)METHYL] 1-PIPERAZINYL} ACETAMIDE
N[2-(3-BENZOTHIAZOLINONYL) ETHYL]N-METHYLACETAMIDE
N[2-(3-BENZOTHIAZOLINONYL) ETHYL]BENZAMIDE
N[2-(3-BENZOTHIAZOLINONYL) ETHYL]PARATOLUOYLCARBOXAMIDE
N[2-(3-BENZOTHIAZOLINONYL) ETHYL]4-FLUOROBENZAMIDE
N[2-(3-BENZOTHIAZOLINONYL) ETHYL]2[4-(4-FLUOROPHENYL) 1-PIPERAZINYL] ACETAMIDE
N[2-(3-BENZOTHIAZOLINONYL) ETHYL] 3-TRIFLUOROMETHYL BENZAMIDE N[2-(3-BENZOTHIAZOLINONYL) ETHYL] 3,5-DICHLOROBENZAMIDE
N[2-(3-BENZOTHIAZOLINONYL) ETHYL]ISONICOTINAMIDE
N[2-(3-BENZOTHIAZOLINONYL) ETHYL] INDOL-2-YL CARBOXAMIDE
N[2-(3-BENZOTHIAZOLINONYL) ETHYL] 2-BENZYLAMINOACETAMIDE
N[2-(3-BENZOTHIAZOLINONYL) ETHYL]2-(N',N' DIETHYL)AMINO ACETAMIDE
N[2-(3-BENZOTHIAZOLINONYL) ETHYL]2-AMINOACETAMIDE
N[2-(3-BENZOTHIAZOLINONYL) ETHYL]2-[4-(3-TRIFLUOROMETHYLPHENYL) PIPERAZIN-1-YL] ACETAMIDE
N[2-(3-BENZOTHIAZOLINONYL)ETHYL]CYCLOHEXANE CARBOXAMIDE
N[2-(3-BENZOTHIAZOLINONYL)ETHYL] FORMAMIDE
N[2-(3-BENZOTHIAZOLINONYL)ETHYL]CYCLOPROPANE CARBOXAMIDE
N[2-(3-BENZOTHIAZOLINONYL)ETHYL] VALERAMIDE
N[2-(3-BENZOTHIAZOLINONYL)ETHYL] CYCLOBUTANE CARBOXAMIDE

En procédant comme dans les exemples 61 à 88, mais en remplaçant la 3-méthyl benzoxazolinone par la benzoxazolinone elle même, on obtient:
N[2-(6-BENZOXAZOLINONYL) ETHYL]ACETAMIDE
N[2-(6-BENZOXAZOLINONYL) ETHYL]ISOBUTYRAMIDE
N[2-(6-BENZOXAZOLINONYL) ETHYL]PHENYLACETAMIDE
N[2-(6-BENZOXAZOLINONYL) ETHYL](2-OXO 1-PYRROLIDINYL)ACETAMIDE
N[2-(6-BENZOXAZOLINONYL) ETHYL]4-CHLOROBUTYRAMIDE
N[2-(6-BENZOXAZOLINONYL) ETHYL]2-PYRROLIDINONE
N[2-(6-BENZOXAZOLINONYL) ETHYL]2-BROMOACETAMIDE
N[2-(6-BENZOXAZOLINONYL) ETHYL]2-MORPHOLINO ACETAMIDE
N[2-(6-BENZOXAZOLINONYL) ETHYL]2-{4-[(2,3,4-TRIMETHOXYPHENYL)METHYL] 1-PIPERAZINYL} ACETAMIDE
N[2-(6-BENZOXAZOLINONYL) ETHYL]N-METHYLACETAMIDE
N[2-(6-BENZOXAZOLINONYL) ETHYL]BENZAMIDE
N[2-(6-BENZOXAZOLINONYL) ETHYL]PARATOLUOYLCARBOXAMIDE
N[2-(6-BENZOXAZOLINONYL) ETHYL]4-FLUOROBENZAMIDE
N[2-(6-BENZOXAZOLINONYL) ETHYL]2[4-(4-FLUOROPHENYL) 1-PIPERAZINYL] ACETAMIDE
N[2-(6-BENZOXAZOLINONYL) ETHYL] 3-TRIFLUOROMETHYL BENZAMIDE
N[2-(6-BENZOXAZOLINONYL) ETHYL] 3,5-DICHLOROBENZAMIDE
N[2-(6-BENZOXAZOLINONYL) ETHYL]ISONICOTINAMIDE
N[2-(6-BENZOXAZOLINONYL) ETHYL] INDOL-2-YL CARBOXAMIDE
N[2-(6-BENZOXAZOLINONYL) ETHYL] 2-BENZYLAMINOACETAMIDE
N[2-(6-BENZOXAZOLINONYL) ETHYL]2-(N',N' DIETHYL)AMINO ACETAMIDE
N[2-(6-BENZOXAZOLINONYL)ETHYL]2-AMINOACETAMIDE
N[2-(6-BENZOXAZOLINONYL)ETHYL]2-[4-(3-TRIFLUOROMETHYLPHENYL) PIPERAZIN-1-YL] ACETAMIDE
N[2-(6-BENZOXAZOLINONYL)ETHYL]CYCLOHEXANE CARBOXAMIDE
N[2-(6-BENZOXAZOLINONYL)ETHYL] FORMAMIDE
N[2-(6-BENZOXAZOLINONYL)ETHYL]CYCLOPROPANE CARBOXAMIDE
N[2-(6-BENZOXAZOLINONYL)ETHYL] VALERAMIDE
N[2-(6-BENZOXAZOLINONYL)ETHYL] CYCLOBUTANE CARBOXAMIDE

En procédant comme dans les exemples 61 à 88, mais en remplaçant la 3-méthyl benzoxazolinone par la benzothiazolinone elle même, on obtient:
N[2-(6-BENZOTHIAZOLINONYL) ETHYL]ACETAMIDE
N[2-(6-BENZOTHIAZOLINONYL) ETHYL]ISOBUTYRAMIDE
N[2-(6-BENZOTHIAZOLINONYL) ETHYL]PHENYLACETAMIDE
N[2-(6-BENZOTHIAZOLINONYL) ETHYL](2-OXO 1-PYRROLIDIHYL)ACETAMIDE
N[2-(6-BENZOTHIAZOLINONYL) ETHYL]4-CHLOROBUTYRAMIDE
N[2-(6-BENZOTHIAZOLINONYL) ETHYL]2-PYRROLIDINONE
N[2-(6-BENZOTHIAZOLINONYL) ETHYL]2-BROMOACETAMIDE
N[2-(6-BENZOTHIAZOLINONYL) ETHYL]2-MORPHOLINO ACETAMIDE
N[2-(3-BENZOTHIAZOLINONYL) ETHYL]2-{4-[(2,3,4-TRIMETHOXYPHENYL)METHYL] 1-PIPERAZINYL} ACETAMIDE
N[2-(6-BENZOTHIAZOLINONYL) ETHYL]N-METHYLACETAMIDE
N[2-(6-BENZOTHIAZOLINONYL) ETHYL]BENZAMIDE
N[2-(6-BENZOTHIAZOLINONYL) ETHYL]PARATOLUOYLCARBOXAMIDE
N[2-(6-BENZOTHIAZOLINONYL) ETHYL]4-FLUOROBENZAMIDE
N[2-(6-BENZOTHIAZOLINONYL) ETHYL]2[4-[4-FLUOROPHENYL) 1-PIPERAZINYL] ACETAMIDE
N[2-(6-BENZOTHIAZOLINONYL) ETHYL] 3-TRIFLUOROMETHYL BENZAMIDE
N[2-(6-BENZOXTHIZOLINONYL) ETHYL] 3,5-DICHLOROBENZAMIDE
N[2-(6-BENZOTHIAZOLINONYL) ETHYL]ISONICOTINAMIDE
N[2-(6-BENZOTHIAZOLINONYL) ETHYL] INDOL-2-YL CARBOXAMIDE
N[2-(6-BENZOTHIAZOLINONYL) ETHYL] 2-BENZYLAMINOACETAMIDE
N[2-(6-BENZOTHIAZOLINONYL) ETHYL]2-(N',N' DIETHYL)AMINO ACETAMIDE
N[2-(6-BENZOTHIAZOLINONYL)ETHYL]2-AMINOACETAMIDE
N[2-(6-BENZOTHIAZOLINONYL)ETHYL]2-[4-(3-TRIFLUOROMETHYLPHENYL) PIPERAZIN-1-YL] ACETAMIDE
N[2-(6-BENZOTHIAZOLINONYL)ETHYL]CYCLOHEXANE CARBOXAMIDE
N[2-(6-BENZOTHIAZOLINONYL)ETHYL] FORMAMIDE
N[2-(6-BENZOTHIAZOLINONYL)ETHYL]CYCLOPROPANE CARBOXAMIDE
N[2-(6-BENZOTHIAZOLINONYL)ETHYL] VALERAMIDE
N[2-(6-BENZOTHIAZOLINONYL)ETHYL] CYCLOBUTANE CARBOXAMIDE

### ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26± 2 grammes). Les animaux ont été observés à intervalles réguliers au cours de la première journée et quotidiennement pendant les deux semaines suivant le traitement. La DL₅₀ entraînant la mort de 50% des animaux, a été évaluée.

La DL₅₀ des produits testés est supérieure à 1000 mg.kg-¹ pour la plupart des composés étudiés ce qui indique la faible toxicité des composés de l'invention.

### EXEMPLE B : ACTIMETRIE

Les animaux sont placés dans des boites en plexiglass équipées avec des cellules photoélectriques placées dans une atmosphère assombrie. Six animaux sont testés simultanément et le nombre des interruptions des faisceaux photo électriques par chaque animal est enregistré informatiquement pendant une heure.

Les composés à tester sont administrés par voie intrapéritonéale immédiatement avant de placer les animaux dans l'appareil

Les produits de l'invention diminuent l'activité des animaux.

### EXEMPLE C : TEST DES QUATRE PLAQUES

Les produits de l'invention sont administrés par voie oesophagienne à des lots de dix souris. Un lot reçoit du sirop de gomme
30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre des passages est enregistré pendant une minute. Après administration, les composés de l'invention augmentent de façon significative le nombre de passages ce qui montre l'activité anxiolytique des dérivés de l'invention.

### EXEMPLE D : ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux C02/N2 5/95%. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesure simultanément :
- sur le crémaster perfusé normalement,
- sur le crémaster sous ligature, c'est-à-dire le crémaster ischémié 2, 7, 14 et 21 jours après ligature.

Deux groupes d'animaux ont été étudiés :
- un groupe témoin sans traitement,
- un groupe traité per os par un produit de l'invention, à raison de 0,1 mg.kg⁻¹ par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

### EXEMPLE E : STIMULATION DES REPONSES IMMUNITAIRES

A des groupes de six souris on a administré des globules rouges de moutons. Ces groupes de souris ont ensuite été traités par voie sous cutanée par les composés de l'invention pendant six jours et un groupe témoin a été traité par un placebo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel Elle est statistiquement accrue dans le groupe traité par les composés de l'invention.

### EXEMPLE F : INHIBITION DE L'OVULATION

On utilise des rates femelles adultes avec des cycles réguliers de quatre jours.

Des frottis vaginaux quotidiens ont été réalisés et des rates ont été sélectionnées après qu'elles ont montré au moins deux cycles consécutifs de quatre jours.

Chaque cycle est constitué de deux jours de dioestrus, un jour de proestrus et un jour d'oestrus.

L'après-midi du jour de proestrus, l'hormone lutéinisante est libérée dans le sang par l'hypophyse. Cette hormorne induit l'ovulation qui se traduit par la présence d'oeufs au niveau de l'oviducte le jour de l'oestrus.

Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées.

### EXEMPLE G : MISE EN EVIDENCE DE L'ACTIVITE ANALGESIQUE

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02%. Les étirements sont dénombrés entre la 5ème et 10ème minute après l'injection.

Il est apparu que certains composés de l'invention possèdent une activité analgésique.

### EXEMPLE H : POTENTIALISATION DU SOMMEIL INDUIT PAR LES BARBITURIQUES

50 mg.kg⁻¹ de pentobarbital sont injectés par voie intrapéritonéale à des souris (22-25 g). On mesure le temps d'apparition et la durée du sommeil. On admet qu'il y a sommeil lorsque les animaux perdent le réflexe de retournement. Les composés à tester sont administrés par voie intrapéritonéale 30 minutes avant l'injection du barbiturique. Les produits de l'invention augmentent la durée du sommeil induit par le pentobarbital.

### EXEMPLE 1: TEST DE BINDING AUX RECEPTEURS DE LA MELATONINE

Le binding aux récepteurs de la mélatonine des composés de l'invention a été réalisé selon des techniques classiques. Il apparaît que les composés de l'invention se lient de façon intéressante aux récepteurs de la mélatonine.

### EXEMPLE J : ETUDE DE L'ACTIVITE HYPOGLYCEMIANTE

Des souris mâles KK ont été placées dans des cages à l'âge de huit semaines. Elles sont utilisées pour l'expérience lorsque leur poids est supérieur à 40 grammes à l'âge de 4-5 mois.

Le composé de l'invention est placé en suspension dans du sirop de gomme. Chaque composé testé est administré oralement 18 heures avant le prélèvement sanguin

Le sang est recueilli par prélèvement au niveau de la veine caudale dans un tube à hématocrite, puis centrifugé. Le plasma est recueilli et le dosage de la glycémie effectué.

Il apparaît que certains composés de l'invention diminuent la glycémie de façon significative.

### EXEMPLE K : ETUDE DE L'INFLUENCE DES PRODUITS DE L'INVENTION SUR LA SYNTHESE DE MELATONINE

Les produits de l'invention sont administrés par voie intrapéritonéales à des rats (Sprague Dawley) à des doses de 1 5 et 25 mg.kg-1. Trois heures après l'administration, les animaux sont sacrifiés et la mélatonine dosée par radio immunologie. La mesure est validée par parallélisme. On observe une augmentation considérable du taux plasmatique de mélatonine après administration des produits de l'invention.

### EXEMPLE L : COMPOSITION PHARMACEUTIQUE : COMPRIMES

Comprimés dosés à 30 mg de N-[2-(3-méthyl 6-benzoxazolinonyl)éthyl] acétamide

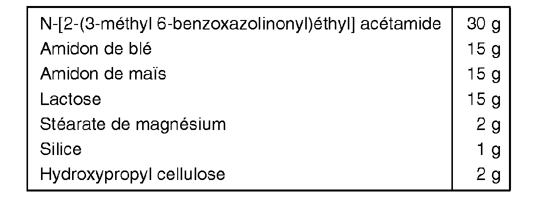

## Revendications (Revendications pour l'(les) Etat(s) contractant(s) suivant(s) : AT, BE, CH, DE, DK, FR, GB, IT, LI, LU, MC, NL, PT, SE)

1. Composés de formule générale (I): 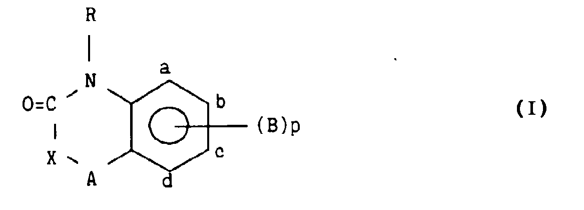 dans laquelle :
A représente un atome d'oxygène ou de soufre,
X représente un groupement CH₂ ou une liaison simple,
R représente
^{*} ou bien un atome d'hydrogène ou un groupement alkyle inférieur et dans ce cas p = et B représente un chaînon -CH₂-CH₂-NR₁-CO-R₂ où R₁ représente un atome d'hydrogène ou un groupement alkyle inférieur linéaire ou ramifié,
et R₂ représente :
un atome d'hydrogène,
un groupement cycloalkyle ou alkyle inférieur linéaire ou ramifié, éventuellement substitué par un atome d'halogène,
un groupement cycloalkyle substitué par un groupement alkyle inférieur linéaire ou ramifié,
un groupement aryle ou hétéroaryle ou arylalkyle inférieur ou aryle substitué ou hétéroaryle substitué ou arylakyle substitué, étant entendu que par groupement hétéroaryle on entend un groupement insaturé mono ou bicyclique comprenant de 1 à 3 hétéroatomes choisis parmi azote, oxygène, ou soufre chaque cycle comprenant 4 ou 5 sommets,
un groupement de formule : 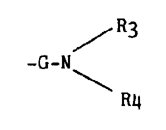
G représentant un groupement alkyle inférieur linéaire ou ramifié,
R₃ et R₄ identiques ou différents représentent chacun un groupement alkyle inférieur ou un atome d'hydrogène ou un groupement phényle ou phénylalkyle inférieur, ou R₃ et R₄ forment avec l'atome d'azote auquel ils sont attachés un système hétérocyclique, aromatique ou non, mono ou bicyclique comprenant éventuellement un autre hétéroatome choisis parmi azote, oxygène ou soufre et éventuellement substitué par un ou plusieurs groupements alkyle inférieur ou oxo, aryle ou arylalkyle inférieur, ou aryle substitué ou arylalkyle inférieur substitué, étant entendu que dans les définitions de R₂, R₃ et R₄, le terme substitué affectant les groupements aryle et arylalkyle et hétéroaryle signifie que ces groupements sont substitués par un ou plusieurs radicaux choisis parmi alkyle inférieur, alkoxy inférieur, trifluorométhyle ou atome d'halogène,
ou bien R₁ forme avec R₂ et le groupement N-CO un système hétérocyclique de formule : 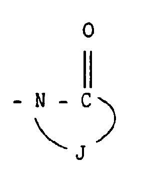
avec J étant un radical alkyle linéaire ou ramifié comprenant de 2 à 8 atomes de carbone,
ou bien un chaînon (CH₂)₂-NRᵢ-CO-R₂ avec R₁ et R₂ ayant la même définition que précédemment et dans ce cas p vaut 0 ou 1 et B représente un groupement alkoxy inférieur,
le cas échéant leurs isomères, épimères et diastéréoisomères ainsi que le cas échéant, leurs sels d'addition à un acide ou une base pharmaceutiquement acceptable étant entendu que alkyle inférieur et alkoxy inférieur signifient des groupements comprenant de 1 à 6 atomes de carbone et que cycloalkyle signifie des groupements comprenant de 3 à 8 atomes de carbone.

2. Composés selon la revendication 1 pour lesquels R représente un chaînon (CH₂)₂-NR,-CO-R₂, B représente un groupement méthoxy et p représente 1, leurs isomères ainsi que le cas échéant leurs sels d'addition à un acide pharmaceutiquement acceptable.

3. Composés selon la revendication 1 pour lesquels R représente un chaînon (CH₂)₂NHCOR₂ avec R₂ représentant un groupement alkyle inférieur ou cycloalkyle et B représente un groupement méthoxy et p vaut 1.

4. Composés selon la revendication 1 pour lesquels B représente un chaînon (CH₂)₂NHCOR₂ avec R₂ représentant un groupement alkyle inférieur ou cycloalkyle, ainsi que le cas échéant leurs sels d'addition à une base pharmaceutiquement acceptable.

5. Composés selon la revendication 1 pour lesquels X représente une liaison simple, le cas échéant leur isomères et leurs sels d'addition à un acide ou une base pharmaceutiquement acceptable.

6. Composé selon la revendication 1 qui est le N [2-(6-méthoxy 3-benzoxazolinonyl)éthyl]acétamide.

7. Composé selon la revendication 1 qui est le N [2-(5-méthoxy 3-benzoxazolinonyl)éthyl]acétamide.

8. Composé selon la revendication 1 pour lesquels R représente un groupement CH₂-CH₂NHC0-CH₃, B représente un groupement OCH₃ et p vaut 1.

9. Composé selon la revendication 1 qui est le N [2-(6-méthoxy 3-benzothiazolinonyl)éthyl]acétamide.

10. Composé selon la revendication 1 qui est le N [2-(5-méthoxy 3-benzothiazolinonyl)éthyl]acétamide.

11. Composés selon la revendication 1 pour lesquels R représente un atome d'hydrogène ou un groupement méthyle et B représente un groupement (CH₂)₂NHCOR₂ avec R₂ représentant un groupement alkyle inférieur ou cycloalkyle ainsi que le cas échéant leurs sels d'addition à une base pharmaceutiquement acceptable.

12. Composé selon la revendication 1 qui est le N [2-(3-méthyl 6-benzoxazolinonyl)éthyl]acétamide.

13. Composé selon la revendication 1 qui est le N[2-(5-méthoxy 3-benzoxazolinonyl)éthyl]cyclopropane carboxamide.

14. Composé selon la revendication 1 qui est le N[2-(5-méthoxy 3-benzothiazolinonyl)éthyl]cyclopropane carboxamide.

15. Procédé de préparation des composés de formule (1) selon la revendication 1 pour lesquels B est différent du chaînon -CH₂-CH₂NR₁COR₂,
caractérisé en ce que l'on utilise comme matière première un dérivé de formule (II): 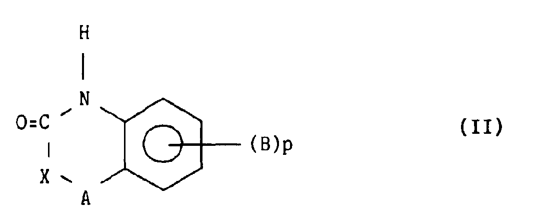 dans laquelle A, B, p et X ont la même définition que dans la formule (I) à l'exception du cas où B représente un chaînon -CH₂-CH₂-NR₁COR₂,
que l'on traite par un agent alcalin pour obtenir un dérivé de formule (III): 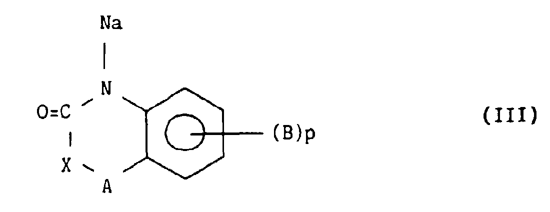 dérivé de formule (III) que l'on traite:
- ou bien par un dérivé de formule (IV/A) : 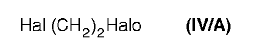 où Hal et Halo identiques ou différents représentent un atome d'halogène, pour obtenir un dérivé de formule (V/A): 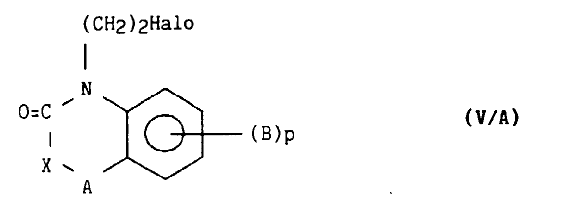 dans laquelle A, X, B, p et Halo ont la même signification que précédemment,
que l'on traite par une amine de formule HNR₁ où R₁ a la même définition que dans la formule (I) pour obtenir un dérivé de formule (VI) : 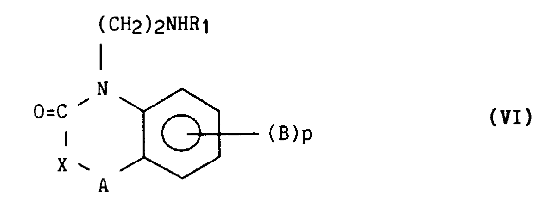 dans laquelle A, B, p, R₁ et X ont la même définition que précédemment,
- ou bien par un dérivé de formule (IV/B) : 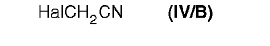 dans laquelle Hal a la même signification que précédemment,
pour obtenir un dérivé de formule (V/B) : 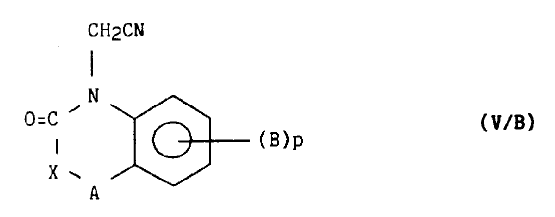 dans laquelle X, A, B et p ont la même signification que précédemment,
dérivé de formule (V/B) que l'on soumet à un agent de réduction puis, lorsque dans le dérivé de formule (I) que l'on souhaite obtenir R₁ représente un groupement alkyle inférieur, à un agent d'alkylation, pour obtenir un dérivé de formule (VI) telle que définie précédemment,
dérivé de formule (VI) que l'on traite :
- soit par un dérivé de formule (VII) : 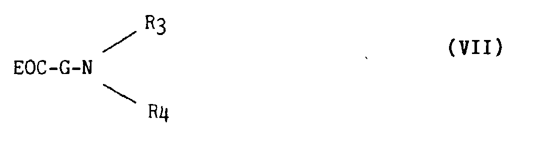 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/A), cas particulier des dérivés de formule (I): 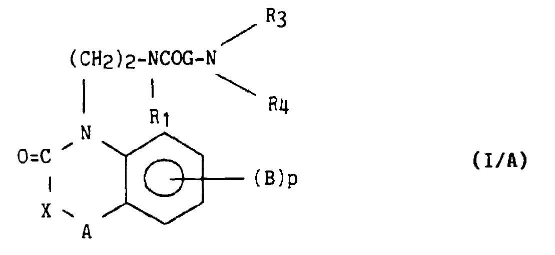 dans laquelle A, B, X, R₁, R₃, R₄, G et p ont la même définition que précédemment, R₂ signifiant ici un groupement : 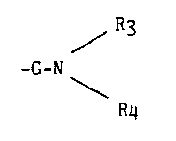 à la condition que B ne représente pas un groupement : 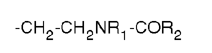 que l'on purifie, si on le désire, par des techniques classiques telles que chromatographie ou cristallisation et que l'on salifie, si on le désire par un acide pharmaceutiquement acceptable,
- soit par un chlorure d'acide de formule (IX) : 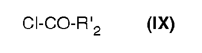 ou par l'anhydride d'acide correspondant,
R'₂ signifiant ici:
- un groupement alkyle inférieur linéaire ou ramifié ou cycloalkyle éventuellement substitué par un atome d'halogène,
- un groupement cycloalkyle substitué par un groupement alkyle inférieur linéaire ou ramifié,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle, le terme hétéroaryle ayant la même signification que précédemment,
pour conduire à un dérivé de formule (I/B) : 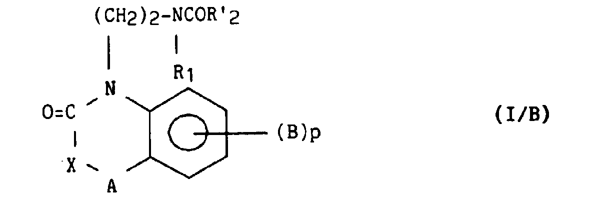 cas particulier des dérivés de formule (I) dans laquelle m, X, A, B, p, R₁ et R'₂ ont la même définition que précédemment, B ne pouvant représenter un chaînon -CH₂-CH₂-NR₁COR₂,
que l'on purifie si nécessaire par des techniques classiques telles que chromatographie et/ou cristallisation, dérivé de formule (I/B) qui dans le cas où R'₂ représente un groupement alkyle inférieur, linéaire ou ramifié, substitué par un atome d'halogène, peut-être soumis, si on le désire à l'action d'une amine de formule (X) : 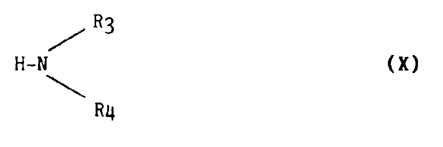 dans laquelle R₃ et R₄ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque R'₂ représente un substituant alkyle linéaire ou ramifié cmnprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/C) : 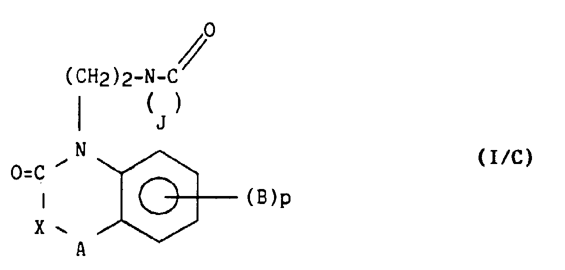 dans laquelle B, p, X et A ont la même signification que précédemment étant entendu que B ne peut représenter un groupement -CH₂-CH₂NRCOR₂ et J représente un groupement alkyle linéaire ou ramifié comprenant de 2 à 8 atomes de carbone, cas particulier des dérivés de formule (1) pour lesquels R₁ et R₂ forment avec NCO un système monocyclique substitué par un groupement oxo et éventuellement substitué par un ou plusieurs groupements alkyles inférieur,
que l'on purifie, si on le désire, par une technique choisie parmi cristallisation ou chromatographie.

16. Procédé de préparation de composés de formule (I) selon la revendication 1 pour lesquels B représente un chaînon -CH₂-CH₂NR₁COR₂ et p représente 1,
caractérisé en ce que l'on utilise comme matière première un dérivé de formule (VIII): 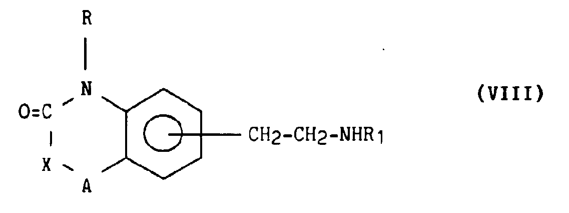 dans laquelle R₁, R, X et A ont la même définition que dans la formule (I), que l'on traite :
^{*} ou bien par un dérivé de formule (VII) : 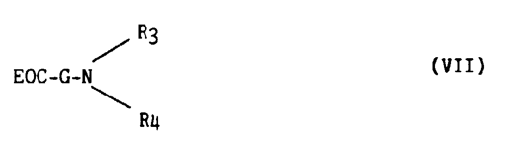 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/D), cas particulier des dérivés de formule (1) : 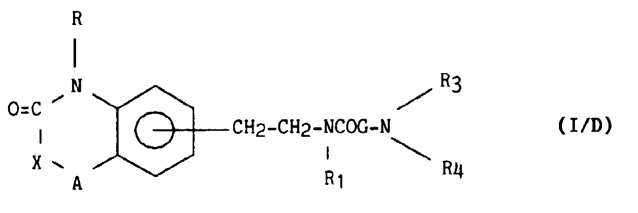 dans laquelle X et A, R₁, R₃, R₄ et G ont la même définition que précédemment, et R représente un atome d'hydrogène ou un groupement alkyle inférieur,
R₂ signifiant ici un groupement : 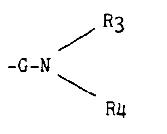 que l'on purifie, si on le désire, par des techniques classiques telles que chromatographie, cristallisation et que l'on salifie, si on le désire, par un acide pharmaceutiquement acceptable, ou lorsque R représente un atome d'hydrogène par une base pharmaceutiquement acceptable,
^{*} ou bien par un chlorure d'acide de formule(IX) : 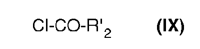 ou par l'anhydride d'acide correspondant,
R'₂ signifiant ici :
- un groupement alkyle inférieur linéaire ou ramifié ou cycloalkyle éventuellement substitué par un atome d'halogène, ou cycloalkyle substitué par un groupement alkyle inférieur linéaire ou ramifié,
- un groupement aryle ou hétéroaryle ou arylalkyle inférieur éventuellememt substitué par un ou plusieurs atomes d'halogène ou groupements choisis parmi alkyle inférieur, alkoxy inférieur, ou trifluorométhyle, le terme hétéroaryle ayant la même signification que précédemment,
- un groupement imidazolyle éventuellement réduit et/ou substitué par un groupement oxo,
pour conduire à un dérivé de formule (I/E) : 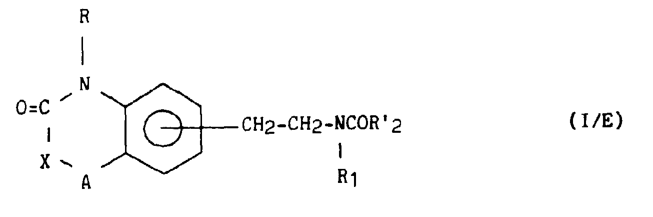 cas particulier des dérivés de formule (I) dans laquelle R₁, X, A et R'₂ ont la même définition que précédemment, R représente un groupement alkyle inférieur ou un atome d'hydrogène,
que l'on purifie si nécessaire par des techniques classiques telles que chromatographie et/ou cristallisation, que l'on salifie si on le désire par une base pharmaceutiquement acceptable lorsque R représente un atome d'hydrogène,
dérivé de formule (I/E) qui dans le cas où R'₂ représente un groupement alkyle inférieur, linéaire ou ramifié, substitué par un atome d'halogène, peut-être soumis, si on le désire à l'action d'une amine de formule (X) : 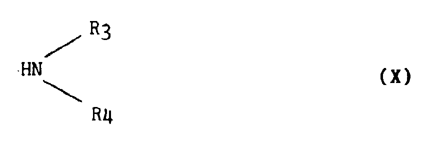 dans laquelle R₃ et R₄ ont la même définition que précédemment,
en excès ou en présence d'une amine tertiaire ou d'un sel de métal alcalin pour conduire à un dérivé de formule (I/D) tel que précédemment défini, qui si on le désire est purifié par une technique classique telle que chromatographie et/ou cristallisation, et/ou salifié par un acide pharmaceutiquement acceptable,
dérivé de formule (I/E) qui lorsque R'₂ représente un substituant alkyle linéaire ou ramifié comprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/F) : 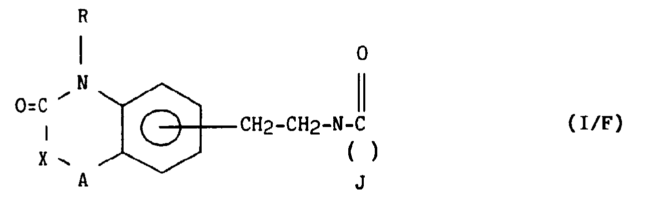 dans laquelle R représente un atome d'hydrogène ou un groupement alkyle inférieur, X et A ont la même signification que précédemment et J représente un groupement alkyle linéaire ou ramifié comprenant de 2 à 8 atomes de carbone, cas particulier des dérivés de formule (1) pour lesquels Rᵢ et R₂ forment avec NCO un système monocyclique substitué par un groupement oxo et éventuellement substitué par un ou plusieurs groupements alkyles inférieurs,
que l'on purifie, si on le désire, par une technique choisie parmi cristallisation ou chromatographie et que l'on salifie, si on le désire, lorsque R représente un atome d'hydrogène par une base pharmaceutiquement acceptable.

17. Procédé de préparation des dérivés de formule (I/K) : 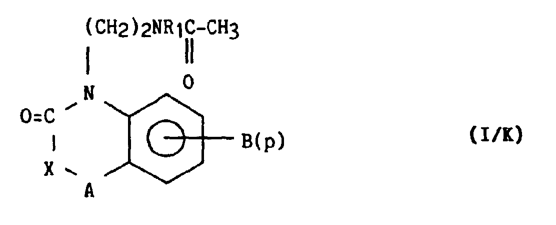 dans laquelle R₁, A, X, B et p ont la même signification que dans la formule (I) selon la revendication 1 par réduction catalytique des dérivés de formule (V/K) : 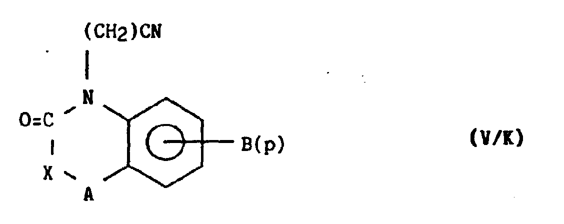 dans laquelle X, A, B et p ont la même signification que dans la formule (1) suivie si nécessaire de purification et lorsque R₁ représente un groupement alkyle inférieur du traitement par un agent alkylant.

18. Composition pharmaceutique contenant comme principe actif au moins un composé selon l'une des revendication 1 à 14 en combinaison avec un ou plusieurs excipients ou véhicules inertes non toxiques, pharmaceutiquement acceptables.

19. Composition pharmaceutique selon la revendication 18 utile dans le traitement du stress, de l'anxiété, les dépressions saisonnières, les insomnies et fatigues dues aux décalages horaires, schizophrénies, attaque de panique, mélancolie, la régulation de l'appétit, l'insomnie, les troubles psychotiques, l'épilepsie, la maladie de Parkinson, la démence sénile, les divers désordres liés au vieillissement normal ou pathologique, pertes de mémoire, maladie d'Alzheimer, ainsi que les troubles de la circulation cérébrale, du cancer, du psoriasis, de l'acné, de la séborrhée ou en tant qu'inhibiteur de l'ovulation ou en médecine vétérinaire dans les troubles du pelage.

## Revendications (Revendications pour l'(les) Etat(s) contractant(s) suivant(s) : ES)

1. Procédé de préparation de composés de formule générale (1) : 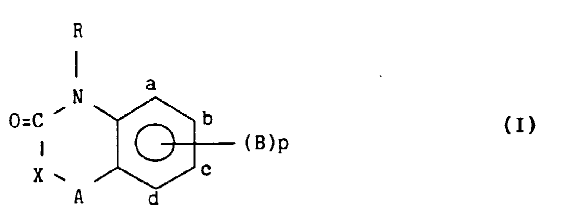 dans laquelle :
A représente un atome d'oxygène ou de soufre,
X représente un groupement CH₂ ou une liaison simple,
R représente
^{*} ou bien un atome d'hydrogène ou un groupement alkyle inférieur et dans ce cas p = 1 et B représente un chaînon -CH₂-CH₂-NR₁-CO-R₂ ou R₁ représente un atome d'hydrogène ou un groupement alkyle inférieur linéaire ou ramifié,
et R₂ représente :
un atome d'hydrogène,
un groupement cycloalkyle ou alkyle inférieur linéaire ou ramifié, éventuellement substitué par un atome d'halogène,
un groupement cycloalkyle substitué par un groupement alkyle inférieur linéaire ou ramifié,
un groupement aryle ou hétéroaryle ou arylalkyle inférieur ou aryle substitué ou hétéroaryle substitué ou arylakyle substitué, étant entendu que par groupement hétéroaryle on entend un groupement insaturé mono ou bicyclique comprenant de 1 à 3 hétéroatomes choisis parmi azote, oxygène, ou soufre chaque cycle comprenant 4 ou 5 sommets,
un groupement de formule : 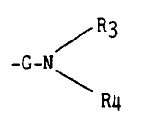
G représentant un groupement alkyle inférieur linéaire ou ramifié,
R₃ et R₄ identiques ou différents représentent chacun un groupement alkyle inférieur ou un atome d'hydrogène ou un groupement phényle ou phénylalkyle inférieur, ou R₃ et R₄ forment avec l'atome d'azote auquel ils sont attachés un système hétérocyclique, aromatique ou non, mono ou bicyclique comprenant éventuellement un autre hétéroatome choisis parmi azote, oxygène ou soufre et éventuellement substitué par un ou plusieurs groupements alkyle inférieur ou oxo, aryle ou arylalkyle inférieur, ou aryle substitué ou arylalkyle inférieur substitué, étant entendu que dans les définitions de R₂, R₃ et R₄, le terme substitué affectant les groupements aryle et arylalkyle et hétéroaryle signifie que ces groupements sont substitués par un ou plusieurs radicaux choisis parmi alkyle inférieur, alkoxy inférieur, trifluorométhyle ou atome d'halogène,
ou bien R₁ forme avec R₂ et le groupement N-CO un système hétérocyclique de formule: 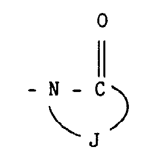
avec J étant un radical alkyle linéaire ou ramifié comprenant de 2 à 8 atomes de carbone,
ou bien un chaînon (CH₂)₂-NR₁-CO-R₂ avec R₁ et R₂ ayant la même définition que précédemment et dans ce cas p vaut 0 ou 1 et B représente un groupement alkoxy inférieur,
le cas échéant leurs isomères, épimères et diastéréoisomères ainsi que le cas échéant, leurs sels d'addition à un acide ou une base pharmaceutiquement acceptable étant entendu que alkyle inférieur et alkoxy inférieur signifient des groupements comprenant de 1 à 6 atomes de carbone et que cycloalkyle signifie des groupements comprenant de 3 à 8 atomes de carbone,
caractérisé lorsque B est différent du chaînon -CH₂CH₂NR₁COR₂ en ce que l'on utilise comme matière première un dérivé de formule (II): 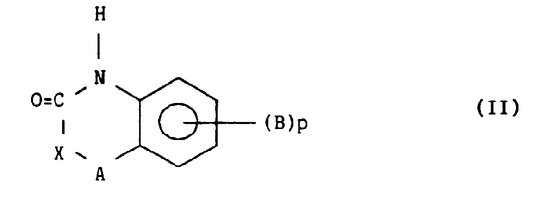
dans laquelle A, B, p et Xont la même définition que dans la formule (I) à l'exception du cas où B représente un chaînon -CH₂-CH₂-NR₁COR₂,
que l'on traite par un agent alcalin pour obtenir un dérivé de formule (III): 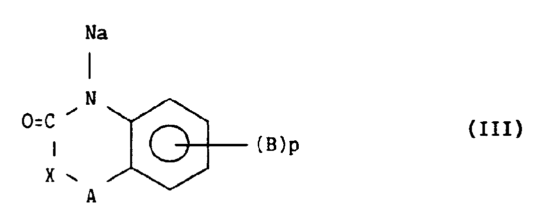
dérivé de formule (III) que l'on traite :
- ou bien par un dérivé de formule (IV/A) : 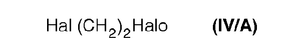 où Hal et Halo identiques ou différents représentent un atome d'halogène, pour obtenir un dérivé de formule (V1A): 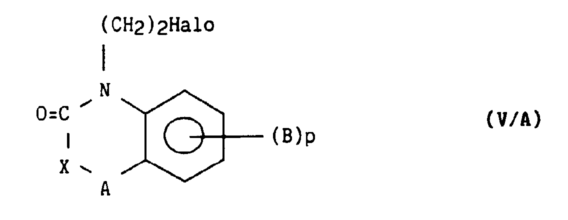 dans laquelle A, X, B, p et Halo ont la même signification que précédemment,
que l'on traite par une amine de formule HNR₁ où R₁ a la même définition que dans la formule (I) pour obtenir un dérivé de formule (VI) : 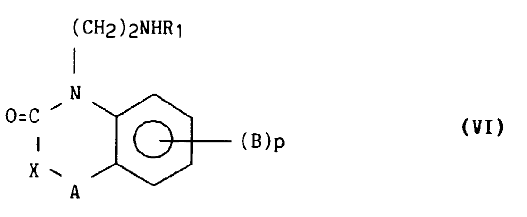 dans laquelle A, B, p, R₁ et X ont la même définition que précédemment,
- ou bien par un dérivé de formule (IV/B) : 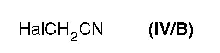 dans laquelle Hal a la même signification que précédemment, pour obtenir un dérivé de formule (V/B) : 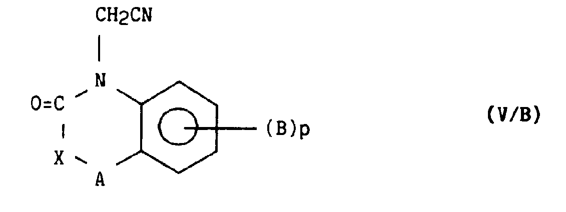 dans laquelle X, A, B et p ont la même signification que précédemment, dérivé de formule (V/B) que l'on soumet à un agent de réduction puis, lorsque dans le dérivé de formule (1) que l'on souhaite obtenir R₁ représente un groupement alkyle inférieur, à un agent d'alkylation, pour obtenir un dérivé de formule (VI) telle que définie précédemment, dérivé de formule (VI) que l'on traite:
- soit par un dérivé de formule (VII) : 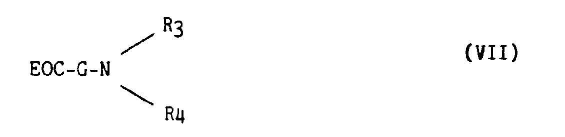 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/A), cas particulier des dérivés de formule (I): 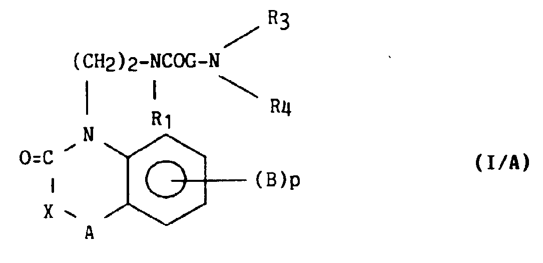 dans laquelle A, B, X, R₁, R₃, R₄, G et p ont la même définition que précédemment, R₂ signifiant ici un groupement : 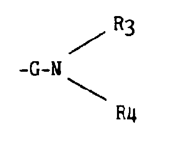 à la condition que B ne représente pas un groupement : 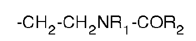 que l'on purifie, si on le désire, par des techniques classiques telles que chromatographie ou cristallisation et que l'on salifie, si on le désire par un acide pharmaceutiquement acceptable,
- soit par un chlorure d'acide de formule (IX): 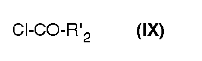 ou par l'anhydride d'acide correspondant,
R'₂ signifiant ici :
- un groupement alkyle inférieur linéaire ou ramifié ou cycloalkyle éventuellement substitué par un atome d'halogène,
- un groupement cycloalkyle substitué par un groupement alkyle inférieur linéaire ou ramifié,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle, le terme hétéroaryle ayant la même signification que précédemment,
pour conduire à un dérivé de formule (I/B) : 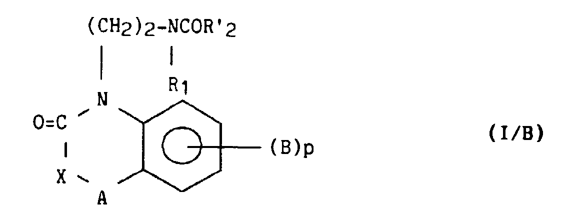 cas particulier des dérivés de formule (I) dans laquelle m, X, A, B, p, R₁ et R'₂ ont la même définition que précédemment, B ne pouvant représenter un chaînon -CH₂-CH₂-NR₁COR₂,
que l'on purifie si nécessaire par des techniques classiques telles que chromatographie et/ou cristallisation, dérivé de formule (I/B) qui dans le cas où R'₂ représente un groupement alkyle inférieur, linéaire ou ramifié, substitué par un atome d'halogène, peut-être soumis, si on le désire à l'action d'une amine de formule (X) : 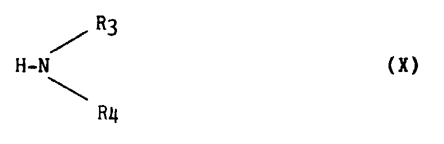 dans laquelle R₃ et R₄ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque R'₂ représente un substituant alkyle linéaire ou ramifié cmnprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/C) : 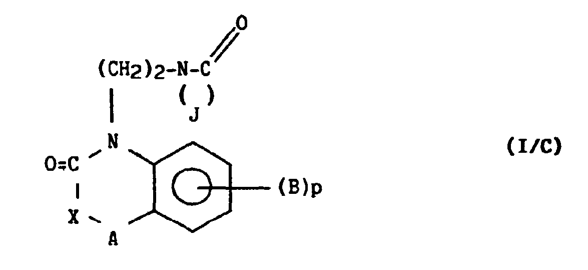 dans laquelle B, p, X et A ont la même signification que précédemment étant entendu que B ne peut représenter un groupement -CH₂-CH₂NRCOR₂ et J représente un groupement alkyle linéaire ou ramifié comprenant de 2 à 8 atomes de carbone, cas particulier des dérivés de -formule (1) pour lesquels R₁ et Reforment avec NCO un système monocyclique substitué par un groupement oxo et éventuellement substitué par un ou plusieurs groupements alkyles inférieur,
que l'on purifie, si on le désire, par une technique choisie parmi cristallisation ou chromatographie.

2. Procédé de préparation de composés de formule (I) selon la revendication 1 pour lesquels B représente un chaînon-CH₂ CH₂ NR₁ et p représente 1 caractérisé en ce que l'on utilise comme matière première un dérivé de formule (VIII): 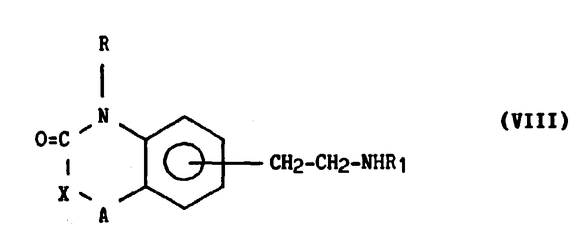 dans laquelle R₁, R, X et A ont la même définition que dans la formule (I),
que l'on traite:
^{*} ou bien par un dérivé de formule (VII) : 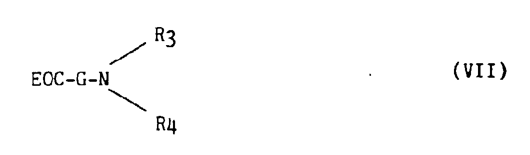 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/D), cas particulier des dérivés de formule (1) : 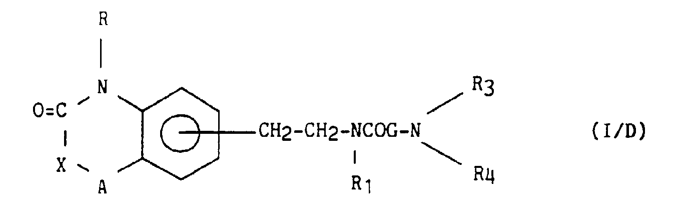 dans laquelle X et A, R₁, R₃, R₄ et G ont la même définition que précédemment, et R représente un atome d'hydrogène ou un groupement alkyle inférieur,
R₂ signifiant ici un groupement : 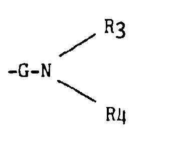 que l'on purifie, si on le désire, par des techniques classiques telles que chromatographie, cristallisation et que l'on salifie, si on le désire, par un acide pharmaceutiquement acceptable, ou lorsque R représente un atome d'hydrogène par une base pharmaceutiquement acceptable,
^{*} ou bien par un chlorure d'acide de formule (IX) : 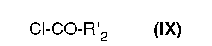 ou par l'anhydride d'acide correspondant,
R'₂ signifiant ici:
- un groupement alkyle inférieur linéaire ou ramifié ou cycloalkyle éventuellement substitué par un atome d'halogène, ou cycloalkyle substitué par un groupement alkyle inférieur linéaire ou ramifié,
- un groupement aryle ou hétéroaryle ou arylalkyle inférieur éventuellememt substitué par un ou plusieurs atomes d'halogène ou groupements choisis parmi alkyle inférieur, alkoxy inférieur, ou trifluorométhyle, le terme hétéroaryle ayant la même signification que précédemment,
- un groupement imidazolyle éventuellement réduit et/ou substitué par un groupement oxo,
pour conduire à un dérivé de formule (I/E) : 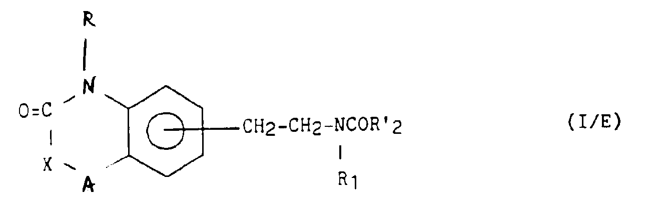 cas particulier des dérivés de formule (1) dans laquelle R₁, X, A et R'₂ ont la même définition que précédemment, R représente un groupement alkyle inférieur ou un atome d'hydrogène,
que l'on purifie si nécessaire par des techniques classiques telles que chromatographie et/ou cristallisation, que l'on salifie si on le désire par une base pharmaceutiquenent acceptable lorsque R représente un atome d'hydrogène,
dérivé de formule (I/E) qui dans le cas où R'₂ représente un groupement alkyle inférieur, linéaire ou ramifié, substitué par un atome d'halogène, peut-être soumis, si on le désire à l'action d'une amine de formule (X) : 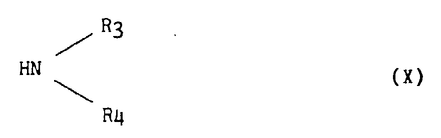 dans laquelle R₃ et R₄ ont la même définition que précédemment,
en excès ou en présence d'une amine tertiaire ou d'un sel de métal alcalin pour conduire à un dérivé de formule (I/D) tel que précédemment défini, qui si on le désire est purifié par une technique classique telle que chromatographie et/ou cristallisation, et/ou salifié par un acide pharmaceutiquement acceptable,
dérivé de formule (I/E) qui lorsque R'₂ représente un substituant alkyle linéaire ou ramifié comprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/F) : 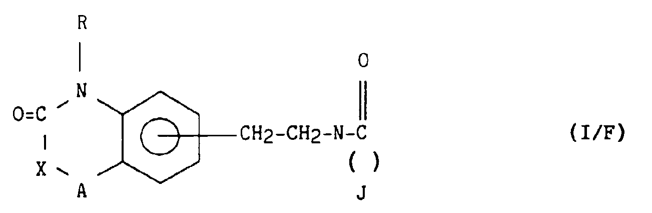 dans laquelle R représente un atome d'hydrogène ou un groupement alkyle inférieur, X et A ont la même signification que précédemment et J représente un groupement alkyle linéaire ou ramifié comprenant de 2 à 8 atomes de carbone, cas particulier des dérivés de formule (1) pour lesquels R₁ et R₂ forment avec NCO un système monocyclique substitué par un groupement oxo et éventuellement substitué par un ou plusieurs groupements alkyles inférieurs,
que l'on purifie, si on le désire, par une technique choisie parmi cristallisation ou chromatographie et que l'on salifie, si on le désire, lorsque R représente un atome d'hydrogène par une base pharmaceutiquement acceptable.

3. Procédé de préparation des dérivés de formule (I/K) : 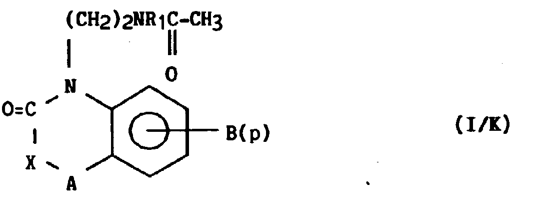 dans laquelle R₁, A, X, B et p ont la même signification que dans la formule (1) selon la revendication 1, par réduction catalytique des dérivés de formule (V/K) : 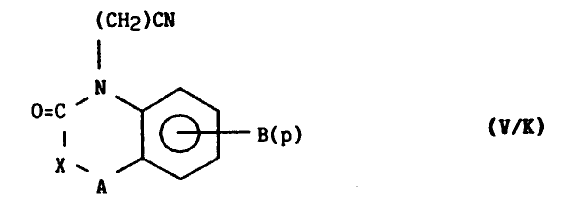 dans laquelle X, A, B et p ont la même signification que dans la formule (I) suivie si nécessaire de purification et lorsque R₁ représente un groupement alkyle inférieur du traitement par un agent alkylant.

4. Procédé de préparation selon la revendication 1 de composés pour lesquels R représente un chaînon (CH₂)₂-NR₁-CO-R₂, B représente un groupement méthoxy et p représente 1, leurs isomères ainsi que le cas échéant leurs sels d'addition à un acide pharmaceutiquement acceptable.

5. Procédé de préparation selon la revendication 1 de composés selon la revendication 1 pour lesquels R représente un chaînon (CH₂)₂NHCOR₂ avec R₂ représentant un groupement alkyle inférieur ou cycloalkyle et B représente un groupement méthoxy et p vaut 1.

6. Procédé de préparation selon la revendication 1 de composés selon la revendication 1 pour lesquels B représente un chaînon (CH₂)₂NHCOR₂ avec R₂ représentant un groupement alkyle inférieur ou cycloalkyle, ainsi que le cas échéant leurs sels d'addition à une base pharmaceutiquement acceptable.

7. Procédé de préparation selon la revendication 1 de composés selon la revendication 1 pour lesquels X représente une liaison simple, le cas échéant leur isomères et leurs sels d'addition à un acide ou une base pharmaceutiquement acceptable.

8. Procédé de préparation selon la revendication 1 du composé selon la revendication 1 qui est le N [2-(6-méthoxy 3-benzoxazolinonyl) éthyl]acélamide.

9. Procédé de préparation selon la revendication 1 du composé selon la revendication 1 qui est le N [2-(5-méthoxy 3-benzoxazolinonyl) éthyl]acélamide.

10. Procédé de préparation selon la revendication 1 de composés selon la revendication 1 pour lesquels R représente un groupement CH₂-CH₂NHC0-CH₃, B représente un groupement OCH₃ et p vaut 1.

11. Procédé de préparation selon la revendication 1 du composé selon la revendication 1 qui est le N [2-(6-méthoxy 3-benzothiazolinonyl) éthyljacétamide.

12. Procédé de préparation selon la revendication 1 du composé selon la revendication 1 qui est le N [2-(5-méthoxy 3-benzothiazolinonyl) éthyl]acétamide.

13. Procédé de préparation selon la revendication 1 de composés selon la revendication 1 pour lesquels R représente un atome d'hydrogène ou un groupement méthyle et B représente un groupement (CH₂)₂NHCOR₂ avec R₂ représentant un groupement alkyle inférieur ou cycloalkyle ainsi que le cas échéant leurs sels d'addition à une base pharmaceutiquement acceptable.

14. Procédé de préparation selon la revendication 1 du composé selon la revendication 1 qui est le N [2-(3-méthyl 6-benzoxazolinoyl) éthyl] acétamide.

15. Procédé de préparation selon la revendication 1 du composé selon la revendication 1 qui est le N[2-(5-méthoxy 3-benzoxazolinonyl) éthyl]cyclopropane carboxamide.

16. Procédé de préparation selon la revendication 1 du composé selon la revendication 1 qui est le N[2-(5-méthoxy 3-benzothiazolinonyl) éthyl]cyclopropane carboxamide.

17. Procédé de préparation de composition pharmaceutique contenant comme principe actif au moins un composé selon l'une des revendication 1 à 16 en combinaison avec un ou plusieurs excipients ou véhicules inertes non toxiques, pharmaceutiquement acceptables.

18. Procédé de préparation de composition pharmaceutique selon la revendication 17 utile dans le traitement du stress, de l'anxiété, les dépressions saisonnières, les insomnies et fatigues dues aux décalages horaires, schizophrénies, attaque de panique, mélancolie, la régulation de l'appétit, l'insomnie, les troubles psychotiques, l'épilepsie, la maladie de Parkinson, la démence sénile, les divers désordres liés au vieillissement normal ou pathologique, pertes de mémoire, maladie d'Alzheimer, ainsi que les troubles de la circulation cérébrale, du cancer, du psoriasis, de l'acné, de la séborrhée ou en tant qu'inhibiteur de l'ovulation ou en médecine vétérinaire dans les troubles du pelage.

## Revendications (Revendications pour l'(les) Etat(s) contractant(s) suivant(s) : GR)

1. Procédé de préparation de composés de formule générale (1) : 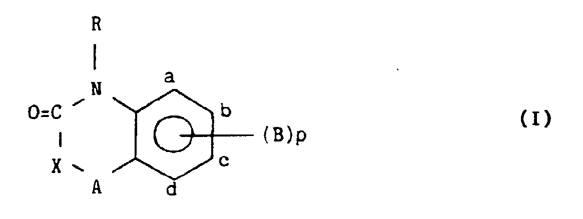 dans laquelle :
A représente un atome d'oxygène ou de soufre,
X représente un groupement CH₂ ou une liaison simple,
R représente
^{*} ou bien un atome d'hydrogène ou un groupement alkyle inférieur et dans ce cas p = et B représente un chaînon -CH₂-CH₂-NRᵢ-CO-R₂ ou R₁ représente un atome d'hydrogène ou un groupement alkyle inférieur linéaire ou ramifié,
et R₂ représente :
un atome d'hydrogène,
un groupement cycloalkyle ou alkyle inférieur linéaire ou ramifié, éventuellement substitué par un atome d'halogène,
un groupement cycloalkyle substitué par un groupement alkyle inférieur linéaire ou ramifié,
un groupement aryle ou hétéroaryle ou arylalkyle inférieur ou aryle substitué ou hétéroaryle substitué ou arylakyle substitué, étant entendu que par groupement hétéroaryle on entend un groupement insaturé mono ou bicyclique comprenant de 1 à 3 hétéroatomes choisis parmi azote, oxygène, ou soufre chaque cycle comprenant 4 ou 5 sommets,
un groupement de formule : 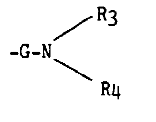
G représentant un groupement alkyle inférieur linéaire ou ramifié,
R₃ et R₄ identiques ou différents représentent chacun un groupement alkyle inférieur ou un atome d'hydrogène ou un groupement phényle ou phénylalkyle inférieur, ou R₃ et R₄ forment avec l'atome d'azote auquel ils sont attachés un système hétérocyclique, aromatique ou non, mono ou bicyclique comprenant éventuellement un autre hétéroatome choisis parmi azote, oxygène ou soufre et éventuellement substitué par un ou plusieurs groupements alkyle inférieur ou oxo, aryle ou arylalkyle inférieur, ou aryle substitué ou arylalkyle inférieur substitué,
étant entendu que dans les définitions de R₂, R₃ et R₄, le terme substitué affectant les groupements aryle et arylalkyle et hétéroaryle signifie que ces groupements sont substitués par un ou plusieurs radicaux choisis parmi alkyle inférieur, alkoxy inférieur, trifluorométhyle ou atome d'halogène, ou bien R₁ forme avec R₂ et le groupement N-CO un système hétérocyclique de formule : 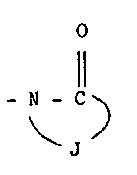 avec J étant un radical alkyle linéaire ou ramifié comprenant de 2 à 8 atomes de carbone,
ou bien un chaînon (CH₂)₂-NR₁-CO-R₂ avec R₁ et R₂ ayant la même définition que précédemment et dans ce cas p vaut 0 ou 1 et B représente un groupement alkoxy inférieur,
le cas échéant leurs isomères, épimères et diastéréoisomères ainsi que le cas échéant, leurs sels d'addition à un acide ou une base pharmaceutiquement acceptable étant entendu que alkyle inférieur et alkoxy inférieur signifient des groupements comprenant de 1 à 6 atomes de carbone et que cycloalkyle signifie des groupements comprenant de 3 à 8 atomes de carbone,
caractérisé lorsque B est différent du chaînon -CH₂CH₂NR₁COR₂ en ce que l'on utilise comme matière première un dérivé de formule (II): 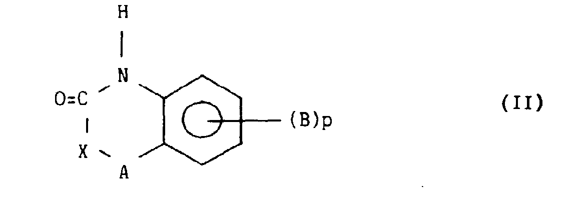
dans laquelle A, B, p et X ont la même définition que dans la formule (1) à l'exception du cas où B représente un chaînon -CH₂-CH₂-NR₁COR₂,
que l'on traite par un agent alcalin pour obtenir un dérivé de formule (III) : 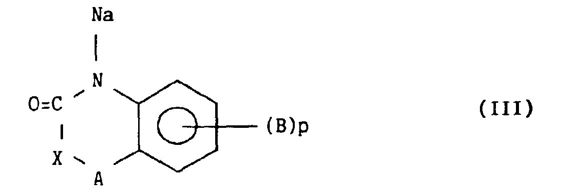 dérivé de formule (III) que l'on traite :
- ou bien par un dérivé de formule (IV/A) : 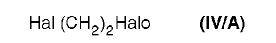 où Hal et Halo identiques ou différents représentent un atome d'halogène, pour obtenir un dérivé de formule (V/A) : 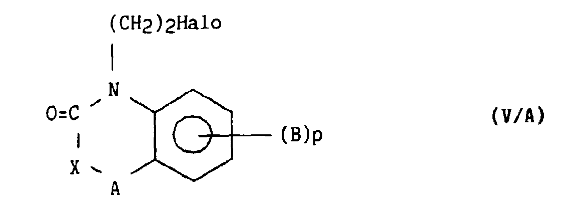 dans laquelle A, X, B, p et Halo ont la même signification que précédemment,
que l'on traite par une amine de formule HNR₁ où R₁ a la même définition que dans la formule (I) pour obtenir un dérivé de formule (VI) : 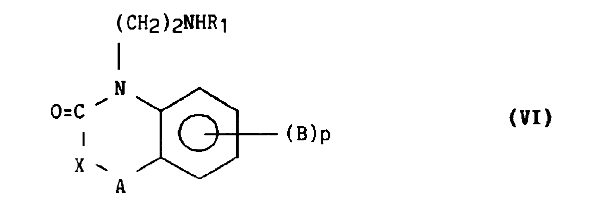 dans laquelle A, B, p, R₁ et X ont la même définition que précédemment,
- ou bien par un dérivé de formule (IV/B) : 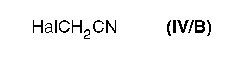 dans laquelle Hal a la même signification que précédemment, pour obtenir un dérivé de formule (V/B) : 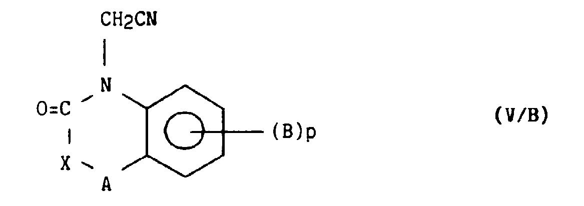 dans laquelle X, A, B et p ont la même signification que précédemment,
dérivé de formule (V/B) que l'on soumet à un agent de réduction puis, lorsque dans le dérivé de formule (I) que l'on souhaite obtenir R₁ représente un groupement alkyle inférieur; à un agent d'alkylation, pour obtenir un dérivé de formule (VI) telle que définie précédemment, dérivé de formule (VI) que l'on traite :
- soit par un dérivé de formule (VII) : 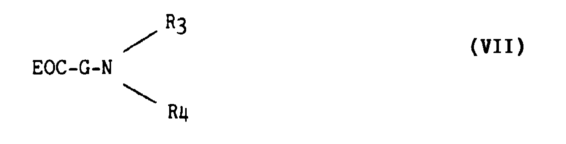 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/A), cas particulier des dérivés de formule (1) : 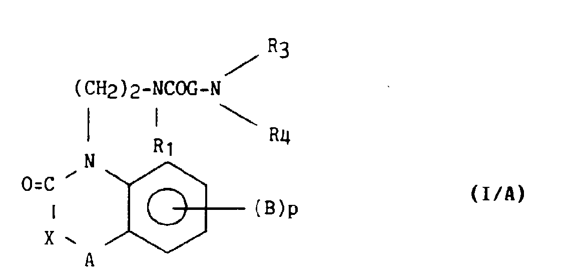 dans laquelle A, B, X, R₁, R₃, R₄, G et p ont la même définition que précédemment, R₂ signifiant ici un groupement : 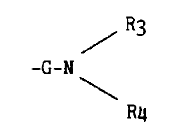 à la condition que B ne représente pas un groupement : 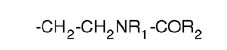 que l'on purifie, si on le désire, par des techniques classiques telles que chromatographie ou cristallisation et que l'on salifie, si on le désire par un acide pharmaceutiquement acceptable,
- soit par un chlorure d'acide de formule (IX) : 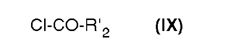 ou par l'anhydride d'acide correspondant,
R'₂ signifiant ici :
- un groupement alkyle inférieur linéaire ou ramifié ou cycloalkyle éventuellement substitué par un atome d'halogène,
- un groupement cycloalkyle substitué par un groupement alkyle inférieur linéaire ou ramifié,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle, le terme hétéroaryle ayant la même signification que précédemment,
pour conduire à un dérivé de formule (I/B) : 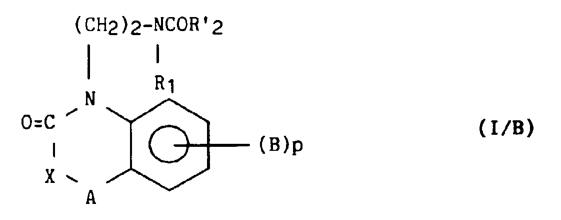 cas particulier des dérivés de formule (I) dans laquelle m, X, A, B, p, R₁ et R'₂ ont la même définition que précédemment, B ne pouvant représenter un chaînon -CH₂-CH₂-NR₁COR₂,
que l'on purifie si nécessaire par des techniques classiques telles que chromatographie et/ou cristallisation, dérivé de formule (I/B) qui dans le cas où R'₂ représente un groupement alkyle inférieur, linéaire ou ramifié, substitué par un atome d'halogène, peut-être soumis, si on le désire à l'action d'une amine de formule (X) : 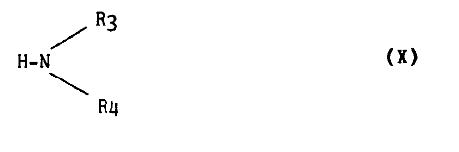 dans laquelle R₃ et R₄ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque R'₂ représente un substituant alkyle linéaire ou ramifié cmnprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/C) : 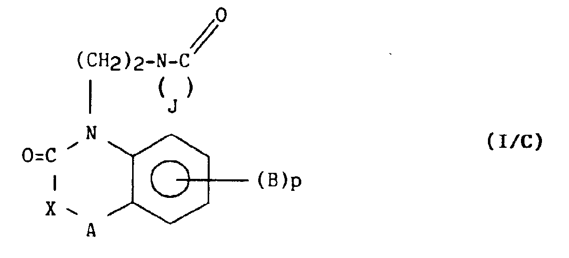 dans laquelle B, p, X et A ont la même signification que précédemment étant entendu que B ne peut représenter un groupement -CH₂-CH₂NRCOR₂ et J représente un groupement alkyle linéaire ou ramifié comprenant de 2 à 8 atomes de carbone, cas particulier des dérivés de formule (I) pour lesquels R₁ et R₂ forment avec NCO un système monocyclique substitué par un groupement oxo et éventuellement substitué par un ou plusieurs groupements alkyles inférieur,
que l'on purifie, si on le désire, par une technique choisie parmi cristallisation ou chromatographie.

2. Procédé de préparation de composés de formule (I) selon la revendication 1 pour lesquels B représente un chaînon -CH₂ CH₂ NR₁ et p représente 1 caractérisé en ce que l'on utilise comme matière première un dérivé de formule (VIII) : 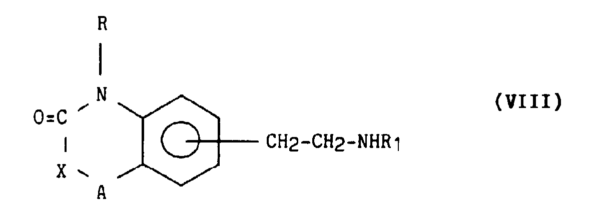 dans laquelle R₁, R, X et A ont la même définition que dans la formule (1), que l'on traite :
^{*} ou bien par un dérivé de formule (VII) : 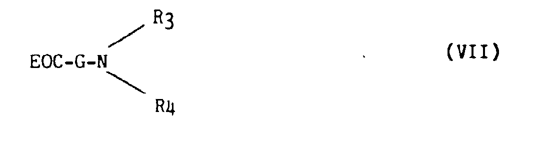 dans laquelle E signifie un groupe partant choisi parmi hydroxyle, alkoxy inférieur ou halogène, G, R₃ et R₄ ayant la même signification que dans la formule (I),
éventuellement en présence d'un agent alcalin,
pour conduire à un dérivé de formule (I/D), cas particulier des dérivés de formule (1) : 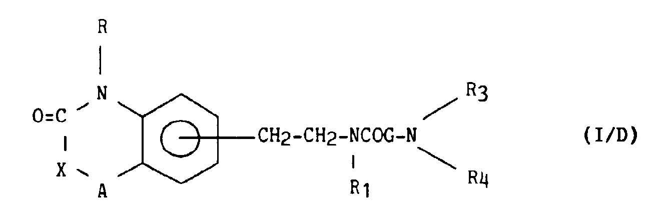 dans laquelle X et A, R₁, R₃, R₄ et G ont la même définition que précédemment, et R représente un atome d'hydrogène ou un groupement alkyle inférieur,
R₂ signifiant ici un groupement : 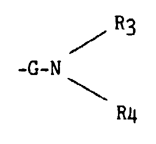 que l'on purifie, si on le désire, par des techniques classiques telles que chromatographie, cristallisation et que l'on salifie, si on le désire, par un acide pharmaceutiquement acceptable, ou lorsque R représente un atome d'hydrogène par une base pharmaceutiquement acceptable,
^{*} ou bien par un chlorure d'acide de formule (IX) : 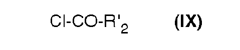 ou par l'anhydride d'acide correspondant,
R'₂ signifiant ici :
- un groupement alkyle inférieur linéaire ou ramifié ou cycloalkyle éventuellement substitué par un atome d'halogène, ou cycloalkyle substitué par un groupement alkyle inférieur linéaire ou ramifié,
- un groupement aryle ou hétéroaryle ou arylalkyle inférieur éventuellememt substitué par un ou plusieurs atomes d'halogène ou groupements choisis parmi alkyle inférieur, alkoxy inférieur, ou trifluorométhyle, le terme hétéroaryle ayant la même signification que précédemment,
- un groupement imidazolyle éventuellement réduit et/ou substitué par un groupement oxo,
pour conduire à un dérivé de formule (I/E) : 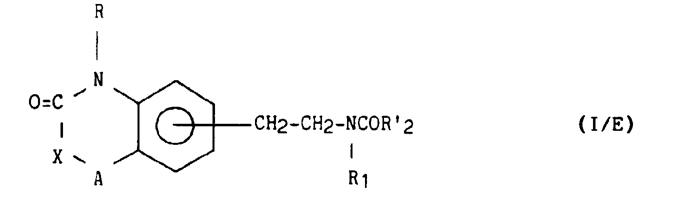 cas particulier des dérivés de formule (I) dans laquelle R₁, X, A et R'₂ ont la même définition que précédemment, R représente un groupement alkyle inférieur ou un atome d'hydrogène,
que l'on purifie si nécessaire par des techniques classiques telles que chromatographie et/ou cristallisation, que l'on salifie si on le désire par une base pharmaceutiquement acceptable lorsque R représente un atome d'hydrogène,
dérivé de formule (I/E) qui dans le cas où R'₂ représente un groupement alkyle inférieur, linéaire ou ramifié, substitué par un atome d'halogène, peut-être soumis, si on le désire à l'action d'une amine de formule (X) : 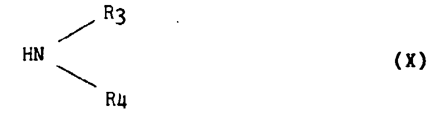 dans laquelle R₃ et R₄ ont la même définition que précédemment,
en excès ou en présence d'une amine tertiaire ou d'un sel de métal alcalin pour conduire à un dérivé de formule (I/D) tel que précédemment défini, qui si on le désire est purifié par une technique classique telle que chromatographie et/ou cristallisation, et/ou salifié par un acide pharmaceutiquement acceptable,
dérivé de formule (I/E) qui lorsque R'₂ représente un substituant alkyle linéaire ou ramifié comprenant au moins deux atomes de carbone et substitué par un atome d'halogène, et lorsque, simultanément R₁ représente un atome d'hydrogène, peut être soumis si on le désire, à l'action d'une base forte et préférentiellement un alcoolate de métal alcalin pour conduire à un dérivé de formule (I/F) : 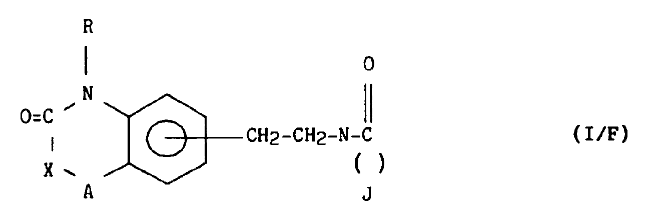 dans laquelle R représente un atome d'hydrogène ou un groupement alkyle inférieur, X et A ont la même signification que précédemment et J représente un groupement alkyle linéaire ou ramifié comprenant de 2 à 8 atomes de carbone, cas particulier des dérivés de formule (1) pour lesquels R₁ et R₂ forment avec NCO un système monocyclique substitué par un groupement oxo et éventuellement substitué par un ou plusieurs groupements alkyles inférieurs, que l'on purifie, si on le désire, par une technique choisie parmi cristallisation ou chromatographie et que l'on salifie, si on le désire, lorsque R représente un atome d'hydrogène par une base pharmaceutiquement acceptable.

3. Procédé de préparation des dérivés de formule (I/K) : 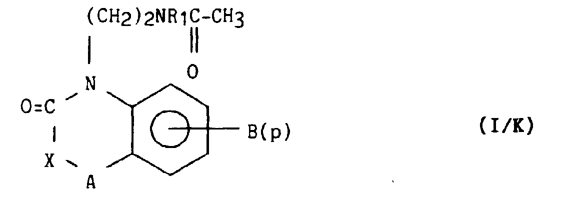 dans laquelle R₁, A, X, B et pont la même signification que dans la formule (I) selon la revendication 1, par réduction catalytique des dérivés de formule (V/K) : 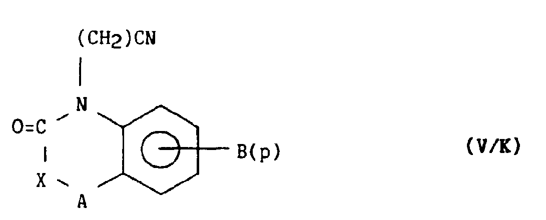 dans laquelle X, A, B et p ont la même signification que dans la formule (I) suivie si nécessaire de purification et lorsque R₁ représente un groupement alkyle inférieur du traitement par un agent alkylant.

4. Procédé de préparation selon la revendication 1 de composés pour lesquels R représente un chaînon (CH₂)₂-NR₁-CO-R₂, B représente un groupement méthoxy et p représente 1, leurs isomères ainsi que le cas échéant leurs sels d'addition à un acide pharmaceutiquement acceptable.

5. Procédé de préparation selon la revendication 1 de composés selon la revendication 1 pour lesquels R représente un chaînon (CH₂)₂NHCOR₂ avec R₂ représentant un groupement alkyle inférieur ou cycloalkyle et B représente un groupement méthoxy et p vaut 1.

6. Procédé de préparation selon la revendication 1 de composés selon la revendication 1 pour lesquels B représente un chaînon (CH₂)₂NHCOR₂ avec R₂ représentant un groupement alkyle inférieur ou cycloalkyle, ainsi que le cas échéant leurs sels d'addition à une base pharmaceutiquement acceptable.

7. Procédé de préparation selon la revendication 1 de composés selon la revendication 1 pour lesquels X représente une liaison simple, le cas échéant leur isomères et leurs sels d'addition à un acide ou une base pharmaceutiquement acceptable.

8. Procédé de préparation selon la revendication 1 du composé selon la revendication 1 qui est le N [2-(6-méthoxy 3-benzoxazolinonyl) éthyljacétamide.

9. Procédé de préparation selon la revendication 1 du composé selon la revendication 1 qui est le N [2-(5-méthoxy 3-benzoxazolinonyl) éthyljacétamide.

10. Procédé de préparation selon la revendication 1 de composés selon la revendication 1 pour lesquels R représente un groupement CH₂-CH₂NHCO-CH₃, B représente un groupement OCH₃ et p vaut 1

11. Procédé de préparation selon la revendication 1 du composé selon la revendication 1 qui est le N [2-(6-méthoxy 3-benzothiazolinonyl) éthyl]acélamide.

12. Procédé de préparation selon la revendication 1 du composé selon la revendication 1 qui est le N [2-(5-méthoxy 3-benzothiazolinonyl) éthyljacétamide.

13. Procédé de préparation selon la revendication 1 de composés selon la revendication 1 pour lesquels R représente un atome d'hydrogène ou un groupement méthyle et B représente un groupement (CH₂)₂NHCOR₂ avec R₂ représentant un groupement alkyle inférieur ou cycloalkyle ainsi que le cas échéant leurs sels d'addition à une base pharmaceutiquement acceptable.

14. Procédé de préparation selon la revendication 1 du composé selon la revendication 1 qui est le N [2-(3-méthyl 6-benzoxazolinoyl) éthyl] acétamide.

15. Procédé de préparation selon la revendication 1 du composé selon la revendication 1 qui est le N[2-(5-méthoxy 3-benzoxazolinonyl) éthyl]cyclopropane carboxamide.

16. Procédé de préparation selon la revendication 1 du composé selon la revendication 1 qui est le N[2-(5-méthoxy 3-benzothiazolinonyl) éthyl]cyclopropane carboxamide.

17. Procédé de préparation de composition pharmaceutique contenant comme principe actif au moins un composé selon l'une des revendication 1 à 16 en combinaison avec un ou plusieurs excipients ou véhicules inertes non toxiques, pharmaceutiquement acceptables.

18. Procédé de préparation de composition pharmaceutique selon la revendication 17 utile dans le traitement du stress, de l'anxiété, les dépressions saisonnières, les insomnies et fatigues dues aux décalages horaires, schizophrénies, attaque de panique, mélancolie, la régulation de l'appétit, l'insomnie, les troubles psychotiques, l'épilepsie, la maladie de Parkinson, la démence sénile, les divers désordres liés au vieillissement normal ou pathologique, pertes de mémoire, maladie d'Alzheimer, ainsi que les troubles de la circulation cérébrale, du cancer, du psoriasis, de l'acné, de la séborrhée ou en tant qu'inhibiteur de l'ovulation ou en médecine vétérinaire dans les troubles du pelage.

## Patentansprüche (Patentansprüche für folgende(n) Vertragsstaat(en) : AT, BE, CH, DE, DK, FR, GB, IT, LI, LU, MC, NL, PT, SE)

1. Verbindungen der allgemeinen Formel (I): 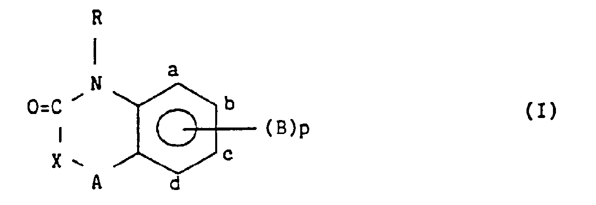 in der
A ein Sauerstoffatom oder ein Schwefelatom.
X eine CH₂-Bindung oder eine Einfachbindung.
R ^{*} entweder ein Wasserstoffatom oder eine Niedrigalkylgruppe, wobei in diesem Fall p 1 darstellt und B eine Kette _CH₂-CH₂-NR,-CO-R₂, in der R₁ ein Wasserstoffatom oder eine geradkettige oder verzweigte Niedrigalkylgruppe darstellt.
und R₂^{:}
ein Wasserstoffatom.
eine Cycloalkylgruppe oder eine niedrigmolekulare geradkettige oder verzweigte Alkylgruppe, die gegebenenfalls durch ein Halogenatom substituiert ist,
eine Cycloalkylgruppe, die durch eine geradkettige oder verzweigte Niedrigalkylgruppe substituiert ist.
eine Arylgruppe oder eine Heteroarylgruppe oder eine Arylniedrigalkylgruppe oder eine substituierte Arylgruppe oder eine substituierte Heteroarylgruppe oder eine substituierte Arylalkylgruppe, wobei unter einer Heteroarylgruppe eine ungesättigte mono- oder bicyclische Gruppe zu verstehen ist, die 1 bis 3 Heteroatome ausgewählt aus Stickstoff. Sauerstoff oder Schwefel enthält und jeder Ring 4 oder 5 Kettenglieder aufweist. eine Gruppe der Formel 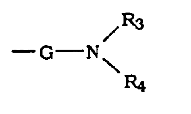 in der G eine geradkettige oder verzweigte Niedrigalkylgruppe und
R₃ und R₄, die gleichartig oder verschieden sein können, jeweils eine Niedrigalkylgruppe oder ein Wasserstoffatom oder eine Phenylgruppe oder eine Phenylniedrigalkylgruppe darstellen oder R₃ und R₄ mit dem Stickstoffatom, an das sie gebunden sind, ein gegebenenfalls aromatisches, mono- oder bicyclisches heterocyclisches System bilden, das gegebenenfalls ein weiteres Heteroatom ausgewählt aus Stickstoff. Sauerstoff und Schwefel enthält und gegebenenfalls durch eine oder mehrere Niedrigalkylgruppen oder Oxogruppen, Arylgruppen oder Arylniedrigalkylgruppen oder substituierte Arylgruppen oder substituierte Arylniedrigalkylgruppen substituiert ist, mit der Maßgabe, daß bei den Definitionen von R₂, R₃ und R₄ der im Zusammenhang mit den Arylgruppen. Arylalkylgruppen und Heteroarylgruppen verwendete Begriff "substituiert" bedeutet, daß diese Gruppen durch einen oder mehrere Reste ausgewählt aus Niedrigalkylgruppen, Niedrigalkoxygruppen, Trifluormethylgruppen oder Halogenatomen substituiert sind, darstellen.
oder R₁ zusammen mit R₂ und der Gruppe N-CO ein heterocyclisches System der Formel 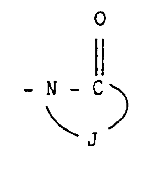 bildet, worin J eine geradkettige oder verzweigte Alkylgruppe mit 2 bis 8 Kohlenstoffatomen darstellt,
^{*} oder eine Kette (CH₂)₂-NR₁-CO-R₂, in der R₁ und R₂ die oben angegebenen Bedeutungen besitzen und in diesem Fall p 0 oder 1 und B eine Niedrigalkoxygruppe darstellen, bedeuten,
und gegebenenfalls deren Isomere, Epimere und Diastereoisomere sowie gegebenenfalls deren Additionssalze mit einer pharmazeutisch annehmbaren Säure oder Base, wobei unter Niedrigalkylgruppen und Niedrigalkoxygruppen Gruppen zu verstehen sind, die 1 bis 6 Kohlenstoffatome aufweisen, und die Cycloalkylgruppe für Gruppen mit 3 bis 8 Kohlenstoffatomen steht.

2. Verbindungen nach Anspruch 1, worin R eine Kette (CH₂)₂-NR₁-CO-R₂, B eine Methoxygruppe und p 1 bedeuten, deren Isomere sowie gegebenenfalls deren Additionssalze mit einer pharmazeutisch annehmbaren Säure.

3. Verbindungen nach Anspruch 1, worin R eine Kette (CH₂)₂NHCOR₂ darstellt, in der R₂ eine Niedrigalkylgruppe oder Cycloalkylgruppe, B eine Methoxygruppe und p 1 bedeuten.

4. Verbindungen nach Anspruch 1, worin B eine Kette (CH₂)₂NHCOR₂ darstellt, worin R₂ eine Niedrigalkylgruppe oder Cycloalkylgruppe bedeutet, sowie gegebenenfalls deren Additionssalze mit einer pharmazeutisch annehmbaren Base.

5. Verbindungen nach Anspruch 1, worin X eine Einfachbindung bedeutet. gegebenenfalls deren Isomere und deren Additionssalze mit einer pharmazeutisch annehmbaren Säure oder Base.

6. Verbindung nach Anspruch 1, nämlich N-[2-(6-Methoxy-3-benzoxazolinonyl)-ethyl]-acetamid.

7. Verbindung nach Anspruch 1, nämlich N-[2-(5-Methoxy-3-benzoxazolinonyl)-ethyl]-acetamid.

8. Verbindung nach Anspruch 1, worin R eine Gruppe CH₂-CH₂NHCO-CH₃, B eine Gruppe OCH₃ und p 1 bedeuten.

9. Verbindung nach Anspruch 1, nämlich N-[2-(6-Methoxy-3-benzothiazolinonyl)-ethyl]-acetamid.

10. Verbindung nach Anspruch 1, nämlich N-[2-(5-Methoxy-3-benzothiazolinonyl)-ethyl]-acetamid.

11. Verbindungen nach Anspruch 1, worin R ein Wasserstoffatom oder eine Methylgruppe und B eine Gruppe (CH₂)₂NHCOR₂, in der R₂ eine Niedrigalkylgruppe oder eine Cycloalkylgruppe darstellt, bedeuten sowie gegebenenfalls deren Additionssalze mit einer pharmazeutisch annehmbaren Base.

12. Verbindung nach Anspruch 1, nämlich N-[2-(3-Methyl-6-benzoxazolinonyl)-ethyl]-acetamid.

13. Verbindung nach Anspruch 1, nämlich N-[2-(5-Methoxy-3-benzoxazolinonyl)-ethyl]-cyclopropan-carboxamid.

14. Verbindung nach Anspruch 1, nämlich N-[2-(5-Melhoxy-3-benzothiazolinonyl)-ethyl]-cyclopropan-carboxamid.

15. Verfahren zur Herstellung der Verbindungen der Formel (I) nach Anspruch 1, in der B von der Kette -CH₂-CH₂NR₁COR₂ verschieden ist,
dadurch gekennzeichnet, daß man als Ausgangsmaterial ein Derivat der Formel (11) verwendet: 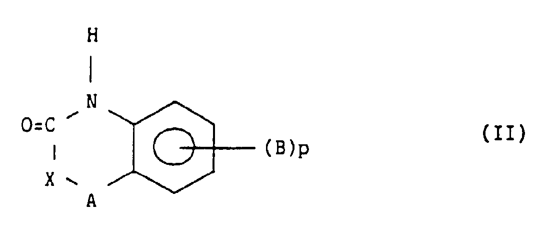 in der A, B, p und X die bezüglich der Formel (I) angegebenen Bedeutungen besitzen, mit Ausnahme des Falls, wo B eine Kette -CH₂-CH₂-NR₁COR₂ darstellt, welches man mit einem alkalischen Mittel behandelt zur Bildung eines Derivats der Formel (III): 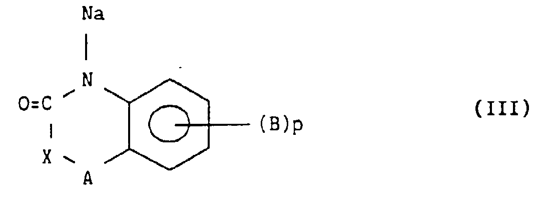 welches Derivat der Formel (III) man:
- entweder mit einem Derivat der Formel (IV/A): 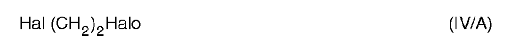 in der Hal und Halo, die gleichartig oder verschieden sein können, Halogenatome darstellen, behandelt, zur Bildung eines Derivats der Formel (V/A): 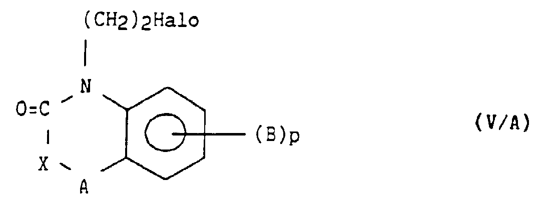 in der A, X, B, p und Halo die oben angegebenen Bedeutungen besitzen, welches man mit einemAmin der Formel HNR₁, in der R₁ die bezüglich der Formel (I) angegebenen Bedeutungen besitzt, behandelt zur Bildung eines Derivats der Formel (VI): 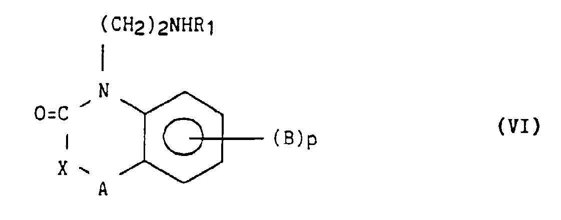 in der A, B, p, R₁ und X die oben angegebenen Bedeutungen besitzen,
- oder mit einem Derivat der Formel (IV/B): 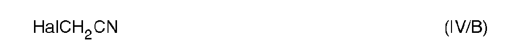 in der Hal die oben angegebenen Bedeutungen besitzt, behandelt, zur Bildung eines Derivats der Formel (V/B): 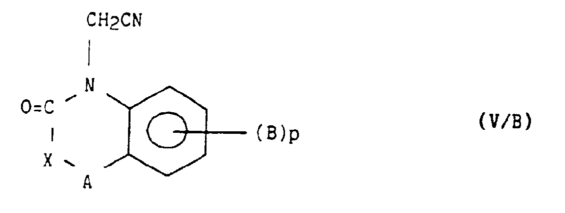 in der X, A, B und p die oben angegebenen Bedeutungen besitzen,
welches Derivat der Formel (V/B) man der Einwirkung eines Reduktionsmittels und dann, wenn in dem herzustellenden Derivat der Formel (I) R₁ eine Niedrigalkylgruppe darstellt, mit einem Alkylierungsmittel behandelt zur Bildung eines Derivats der Formel (VI), wie sie oben definiert worden ist,
welches Derivat der Formel (VI) man:
- entweder mit einem Derivat der Formel (VII): 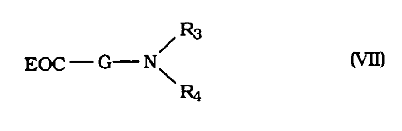 in der E eine aus Hydroxylgruppen, Niedrigalkoxygruppen oder Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt, zur Bildung eines Derivats der Formel (I/ A), einem Sonderfall der Derivate der Formel (I): 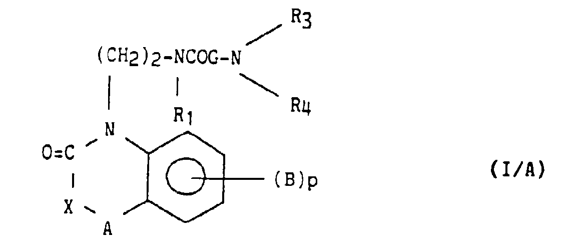 in der A, B, X, R₁, R₃, R₄, G und p die oben angegebenen Bedeutungen besitzen, wobei in diesem Fall R₂ die Gruppe 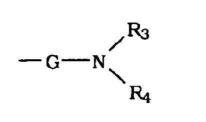 bedeutet, mit der Maßgabe, daß B keine Gruppe: 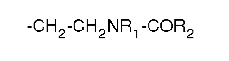 darstellt, welches man gewünschtenfalls mit Hilfe klassischer Verfahrensweisen, wie der Chromatographie oder der Kristallisation, reinigt und gewünschtenfalls mit einer pharmazeutisch annehmbaren Säure in ein Salz überführt. - oder mit einem Säurechlond der Formel (IX): 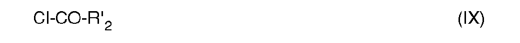 oder mit einem entsprechenden Säureanhydrid behandelt,
wobei in diesem Fall R'₂:
- eine geradkettige oder verzweigte Niedrigalkylgruppe oder Cycloalkylgruppe, die gegebenenfalls durch ein Halogenatom substituiert ist,
- eine Cycloalkylgruppe, die durch eine geradkettige oder verzweigte Niedrigalkylgruppe substituiert ist, oder
- eine Aryl- oder Heteroaryl- oder Arylniedrigalkylgruppe, die gegebenenfalls durch eines oder mehrere Halogenatome oder Gruppen ausgewählt aus Niedrigalkylgruppen. Niedrigalkoxygruppen oder Trifluormethylgruppen substituiert ist, wobei der Begriff Heteroaryl die oben angegebenen Bedeutungen besitzt,
zur Bildung eines Derivats der Formel (I/B): 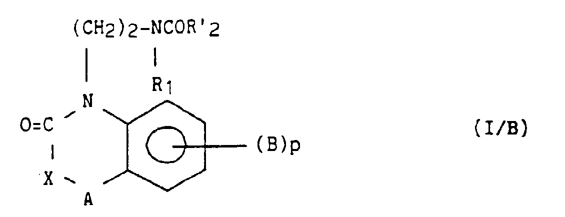 einem Sonderfall der Derivate der Formel (I), in der m, X, A, B, p, R₁ und R'₂ die oben angegebenen Bedeutungen besitzen und B keine Kette -CH₂-CH₂NR₁COR₂ darstellt,
welches man erforderlichenfalls mit Hilfe klassischer Verfahrensweisen, wie der Chromatographie und/oder der Kristallisation, reinigt,
welches Derivat der Formel (I/B) in dem Fall, wo R'₂ eine geradkettige oder verzweigte, durch ein Halogenatom substituierte Niedrigalkylgruppe darstellt, gewünschtenfalls der Einwirkung eines Amins der Formel (X): 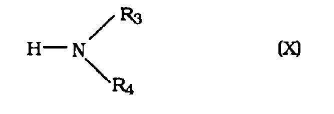 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/A), wie es oben definiert worden ist, welches gewünschtenfalls mit Hilfe einer klassischen Verfahrensweise, wie der Chromatographie und/oder der Kristallisation, gereinigt und/oder mit einer pharmazeutisch annehmbaren Säure in ein Salz überführt werden kann,
welches Derivat der Formel (I/B), wenn R'₂ einen geradkettigen oder verzweigten Alkylsubstituenten, der mindestens zwei Kohlenstoffatome aufweist und durch ein Halogenatom substituiert ist, darstellt und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C): 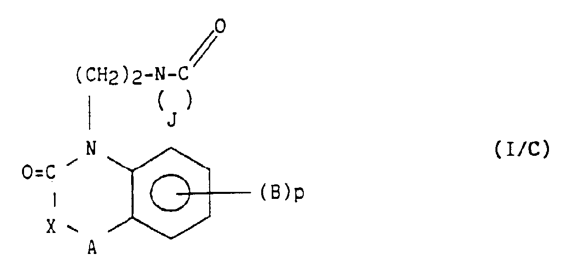 in der B, p, X und A die oben angegebenen Bedeutungen besitzen, wobei es sich versteht, daß B keine Gruppe -CH₂-CH₂NRCOR₂ darstellt und J eine geradkettige oder verzweigte Alkylgruppe mit 2 bis 8 Kohlenstoffatomen darstellt, einem Sonderfall der Derivate der Formel (I), in der R₁ und R₂ zusammen mit NCO ein monocyclisches System bilden, das durch eine Oxogruppe substituiert ist und gegebenenfalls durch eine oder mehrere Niedrigalkylgruppen substituiert ist, welches man gewünschtenfalls mit Hilfe einer aus Kristallisation und Chromatographie ausgewählten Verfahrensweise reinigt.

16. Verfahren zur Herstellung der Verbindungen der Formel (I) nach Anspruch 1, worin B eine Kette -CH₂-CH₂NR₁COR₂ und p 1 bedeuten,
dadurch gekennzeichnet, daß man als Ausgangsmaterial ein Derivat der Formel (VIII): 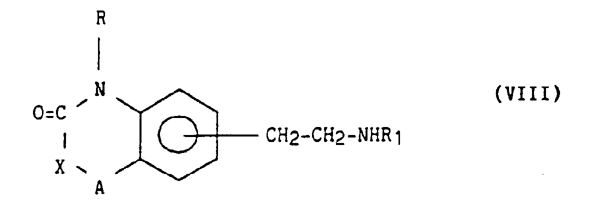 in der R₁; R, X und A die bezüglich der Formel (I) angegebenen Bedeutungen besitzen, verwendet, welches man:
^{*} entweder mit einem Derivat der Formel (VII): 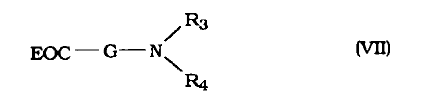 in der E eine aus Hydroxylgruppen, Niedrigalkoxygruppen und Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt,
zur Bildung eines Derivats der Formel (I/D), einem Sonderfall der Derivate der Formel (I): 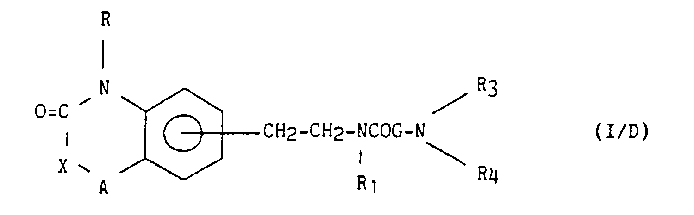 in der X und A, R₁, R₃, R₄ und G die oben angegebenen Bedeutungen besitzen und R ein Wasserstoffatom oder eine Niedrigalkylgruppe bedeutet,
wobei R₂ hier eine Gruppe 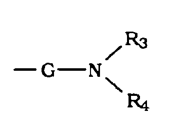 darstellt,
welches man gewünschtenfalls mit Hilfe klassischer Verfahrensweisen, wie der Chromatographie oder der Kristallisation reinigt und gewünschtenfalls mit Hilfe einer pharmazeutisch annehmbaren Säure, oder wenn R ein Wasserstoffatom darstellt, mit Hilfe einer pharmazeutisch annehmbaren Base in ein Salz überführt
^{*} oder mit einem Säurechlorid der Formel (IX): 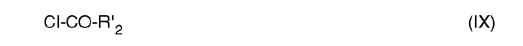 oder einem entsprechenden Säureanhydrid behandelt,
wobei hier R'₂:
- eine geradkettige oder verzweigte Niedrigalkylgruppe oder Cycloalkylgruppe, die gegebenenfalls durch ein Halogenatom substituiert ist oder eine Cycloalkylgruppe, die durch eine geradkettige oder verzweigte Niedrigalkylgruppe substituiert ist.
- eine Arylgruppe oder eine Heteroarylgruppe oder eine Arylniedngalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert sind, wobei der Begriff Heteroarylgruppe die oben angegebenen Bedeutungen besitzt, oder
- eine Imidazolylgruppe, die gegebenenfalls reduziert und/oder durch eine Oxogruppe substituiert ist, darstellt,
zur Bildung eines Derivats der Formel (I/E): 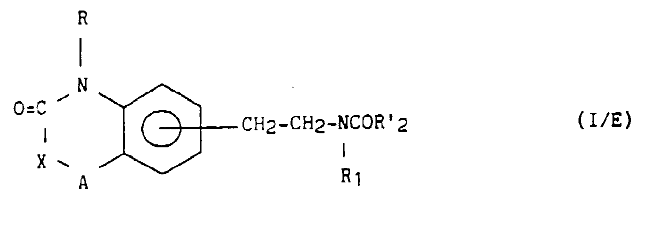 einem Sonderfall der Derivate der Formel (I)_{;} in der R₁, X, A und R'₂ die oben angegebenen Bedeutungen besitzen und R eine Niedrigalkylgruppe oder ein Wasserstoffatom darstellt,
welches man erforderlichenfalls mit Hilfe klassischer Verfahrensweisen, wie der Chromatographie und/oder der Kristallisation reinigt, und gewünschtenfalls mit einer pharmazeutisch annehmbaren Base, wenn R ein Wasserstoffatom darstellt. in ein Salz überführt.
welches Derivat der Formel (I/E) dann, wenn R'₂ eine geradkettige oder verzweigte Niedrigalkylgruppe, die durch ein Halogenatom substituiert ist, bedeutet, gewünschtenfalls der Einwirkung eines Amins der Formel (X): 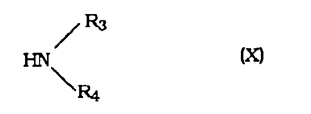 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/D), wie es oben definiert worden ist, welches man gewünschtenfalls mit Hilfe einer klassischen Verfahrensweise, wie der Chromatographie und/oder der Kristallisation reinigt und/oder mit Hilfe einer pharmazeutisch annehmbaren Säure in ein Salz überführt.
welches Derivat der Formel (I/E), wenn R'₂ einen geradkettigen oder verzweigten Alkylsubstituenten darstellt, der mindestens zwei Kohlenstoffatome aufweist und durch ein Halogenatom substituiert ist und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/F): 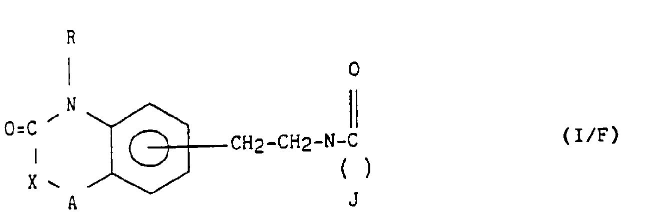 in der Rein Wasserstoffatom oder eine Niedrigalkylgruppe darstellt, X und A die oben angegebenen Bedeutungen besitzen und J eine geradkettige oder verzweigte Alkylgruppe mit 2 bis 8 Kohlenstoffatomen bedeutet, einem Sonderfall der Derivate der Formel (I), in der R₁ und R₂ zusammen mit NCO ein monocyclisches System bilden, das durch eine Oxogruppe substituiert und gegebenenfalls durch eine oder mehrere Niedrigalkylgruppen substituiert ist,
welches man gewünschtenfalls mit Hilfe einer aus Kristallisation oder Chromatographie ausgewählten Verfahrensweise reinigt und gewünschtenfalls, wenn R ein Wasserstoffatom darstellt, mit einer pharmazeutisch annehmbaren Base in ein Salz überführt.

17. Verfahren zur Herstellung der Derivate der Formel (I/K): 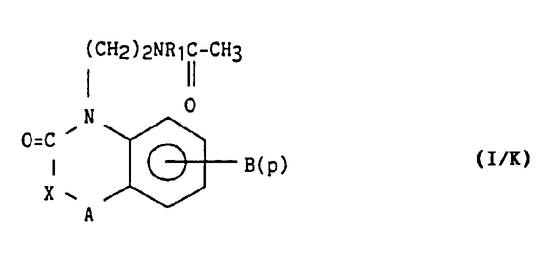 in der R₁, A, X, B und p die bezüglich der Formel (I) angegebenen Bedeutungen besitzen, nach Anspruch 1, durch katalytische Reduktion der Derivate der Formel (V/K): 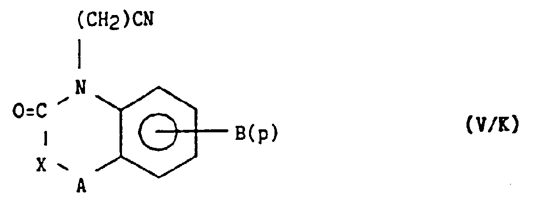 in der X, A, B und p die bezüglich der Formel (I) angegebenen Bedeutungen besitzen, erforderlichenfalls gefolgt von einer Reinigung und wenn R₁ eine Niedrigalkylgruppe darstellt, durch Behandlung mit einem Alkylierungsmittel.

18. Pharmazeutische Zubereitung enthaltend als Wirkstoff mindestens eine Verbindung nach einem der Ansprüche 1 bis 14 in Kombination mit einem oder mehreren inerten, nichttoxischen, pharmazeutisch annehmbaren Trägermaterialien oder Bindemitteln.

19. Pharmazeutische Zubereitung nach Anspruch 18 geeignet zur Behandlung von Streß, der Angst, von saisonbedingten Depressionen, von Schlaflosigkeit und Müdigkeit als Folge der Zeitverschiebung, der Schizophrenie, von Panikanfällen, der Melancholie, zur Appetitsteuerung, der Schlaflosigkeit, von psychotischen Störungen, der Epilepsie, der Parkinsonschen Krankheit, der senilen Demenz, von verschiedenen Störungen, die mit dem normalen oder pathologischen Altern verknüpft sind, von Gedächtnisverlusten, der Alzheimerschen Krankheit sowie von Störungen der Gehirndurchblutung, von Krebs, der Psoriasis, der Akne, der Seborrhoe oder als Ovulationsinhibitor oder in der Veterinärmedizin bei Störungen des Haarkleids.

## Patentansprüche (Patentansprüche für folgende(n) Vertragsstaat(en) : ES)

1. Verfahren zur Herstellung der Verbindungen der allgemeinen Formel (I): 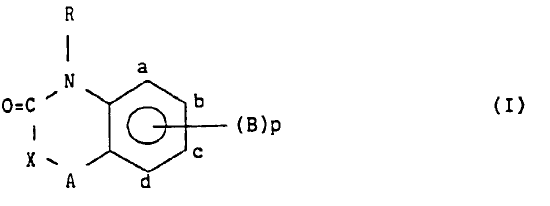 in der
A ein Sauerstoffatom oder ein Schwefelatom.
X eine CH₂-Bindung oder eine Einfachbindung.
R: ^{*} entweder ein Wasserstoffatom oder eine Niedrigalkylgruppe, wobei in diesem Fall p 1 darstellt und B eine Kette -CH₂-CH₂-NR₁-CO-R₂, in der R₁ ein Wasserstoffatom oder eine geradkettige oder verzweigte Niedrigalkylgruppe darstellt,
und R₂^{:}
ein Wasserstoffatom.
eine Cycloalkylgruppe oder eine niedrigmolekulare geradkettige oder verzweigte Alkylgruppe, die gegebenenfalls durch ein Halogenatom substituiert ist,
eine Cycloalkylgruppe, die durch eine geradkettige oder verzweigte Niedrigalkylgruppe substituiert ist.
eine Arylgruppe oder eine Heteroarylgruppe oder eine Arylniedrigalkylgruppe oder eine substituierte Arylgruppe oder eine substituierte Heteroarylgruppe oder eine substituierte Arylalkylgruppe, wobei unter einer Heteroarylgruppe eine ungesättigte mono- oder bicyclische Gruppe zu verstehen ist, die 1 bis 3 Heteroatome ausgewählt aus Stickstoff, Sauerstoff oder Schwefel enthält und jeder Ring 4 oder 5 Kettenglieder aufweist. eine Gruppe der Formel 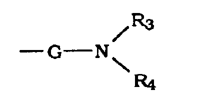 in der
G eine geradkettige oder verzweigte Niedrigalkylgruppe und
R₃ und R₄, die gleichartig oder verschieden sein können, jeweils eine Niedrigalkylgruppe oder ein Wasserstoffatom oder eine Phenylgruppe oder eine Phenylniedrigalkylgruppe darstellen oder R₃ und R₄ mit dem Stickstoffatom, an das sie gebunden sind, ein gegebenenfalls aromatisches, mono- oder bicyclisches heterocyclisches System bilden, das gegebenenfalls ein weiteres Heteroatom ausgewählt aus Stickstoff. Sauerstoff und Schwefel enthält und gegebenenfalls durch eine oder mehrere Niedrigalkylgruppen oder Oxogruppen, Arylgruppen oder Arylniedrigalkylgruppen oder substituierte Arylgruppen oder substituierte Arylniedngalkylgruppen substituiert ist, mit der Maßgabe, daß bei den Definitionen von R₂, R₃ und R₄ der im Zusammenhang mit den Arylgruppen. Arylalkylgruppen und Heteroarylgruppen verwendete Begriff "substituiert" bedeutet, daß diese Gruppen durch einen oder mehrere Reste ausgewählt aus Niedrigalkylgruppen, Niedrigalkoxygruppen, Trifluormethylgruppen oder Halogenatomen substituiert sind, darstellen
oder R₁ zusammen mit R₂ und der Gruppe N-CO ein heterocyclisches System der Formel 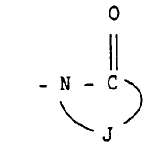
bildet, worin J eine geradkettige oder verzweigte Alkylgruppe mit 2 bis 8 Kohlenstoffatomen darstellt,
^{*} oder eine Kette (CH₂)₂-NR,-CC-R₂, in der R₁ und R₂ die oben angegebenen Bedeutungen besitzen und in diesem Fall p 0 oder 1 und B eine Niedrigalkoxygruppe darstellen, bedeuten,
und gegebenenfalls von deren Isomeren, Epimeren und Diastereoisomeren sowie gegebenenfalls von deren Additionssalzen mit einer pharmazeutisch annehmbaren Säure oder Base, wobei unter Niedrigalkylgruppen und Niedrigalkoxygruppen Gruppen zu verstehen sind, die 1 bis 6 Kohlenstoffatome aufweisen, und die Cycloalkylgruppe für Gruppen mit 3 bis 8 Kohlenstoffatomen steht, dadurch gekennzeichnet, daß, wenn B von der Kette -CH₂CH₂NR₁COR₂ verschieden ist, man als Ausgangsmaterial ein Derivat der Formel (11) verwendet: 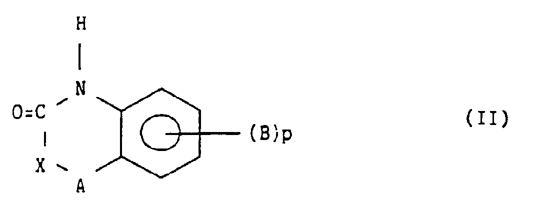 in der A, B, p und X die bezüglich der Formel (I) angegebenen Bedeutungen besitzen, mit Ausnahme des Falls, wo B eine Kette -CH₂-CH₂-NR₁ COR₂ darstellt, welches man mit einem alkalischen Mittel behandelt zur Bildung eines Derivats der Formel (III): 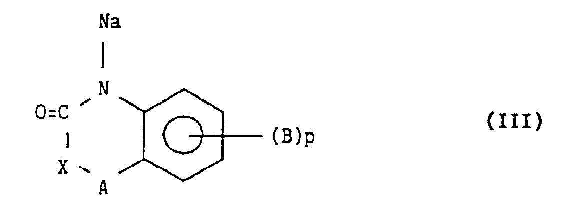 welches Derivat der Formel (III) man:
- entweder mit einem Derivat der Formel (IV/A): 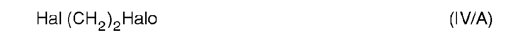 in der Hal und Halo, die gleichartig oder verschieden sein können, Halogenatome darstellen, behandelt, zur Bildung eines Derivats der Formel (V/A): 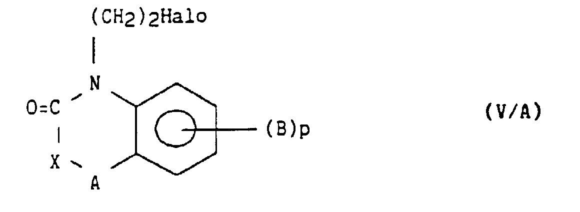 in der A, X, B, p und Halo die oben angegebenen Bedeutungen besitzen, welches man mit einem Amin der Formel HNR₁, in der R₁ die bezüglich der Formel (I) angegebenen Bedeutungen besitzt, behandelt zur Bildung eines Derivats der Formel (VI): 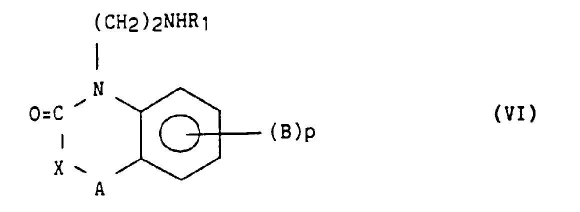 in der A, B, p, R₁ und X die oben angegebenen Bedeutungen besitzen,
- oder mit einem Derivat der Formel (IV/B): 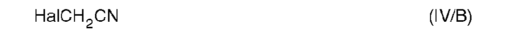 in der Hal die oben angegebenen Bedeutungen besitzt, behandelt, zur Bildung eines Derivats der Formel (V/B): 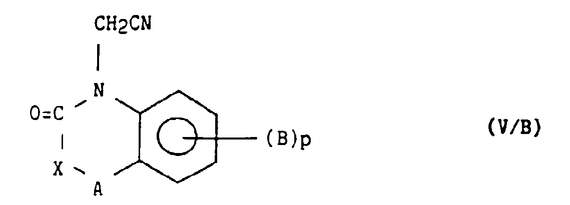 in der X, A, B und p die oben angegebenen Bedeutungen besitzen, welches Derivat der Formel (V/B) man der Einwirkung eines Reduktionsmittels und dann, wenn in dem herzustellenden Derivat der Formel (I) R₁ eine Niedrigalkylgruppe darstellt, mit einem Alkylierungsmittel behandelt zur Bildung eines Derivats der Formel (VI), wie sie oben definiert worden ist,
welches Derivat der Formel (VI) man:
- entweder mit einem Derivat der Formel (VII): 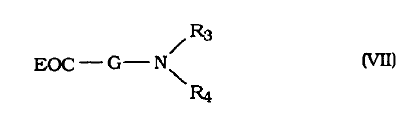 in der E eine aus Hydroxylgruppen, Niedrigalkoxygruppen oder Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt, zur Bildung eines Derivats der Formel (I/A); einem Sonderfall der Derivate der Formel (I): 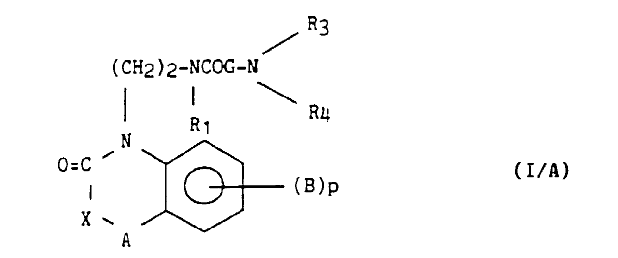 in der A, B, X, R₁, R₃, R₄, G und p die oben angegebenen Bedeutungen besitzen, wobei in diesem Fall R₂ die Gruppe 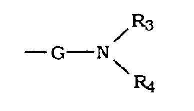 bedeutet, mit der Maßgabe, daß B keine Gruppe: 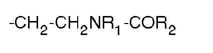 darstellt, welches man gewünschtenfalls mit Hilfe klassischer Verfahrensweisen, wie der Chromatographie oder der Kristallisation, reinigt und gewünschtenfalls mit einer pharmazeutisch annehmbaren Säure in ein Salz überführt,
- oder mit einem Säurechlorid der Formel (IX): 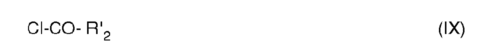 oder mit einem entsprechenden Säureanhydrid behandelt,
wobei in diesem Fall R'₂:
- eine geradkettige oder verzweigte Niedrigalkylgruppe oder Cycloalkylgruppe, die gegebenenfalls durch ein Halogenatom substituiert ist,
- eine Cycloalkylgruppe, die durch eine geradkettige oder verzweigte Niedrigalkylgruppe substituiert ist, oder
- eine Aryl- oder Heteroaryl- oder Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert ist, wobei der Begriff Heteroaryl die oben angegebenen Bedeutungen besitzt,
zur Bildung eines Derivats der Formel (I/B): 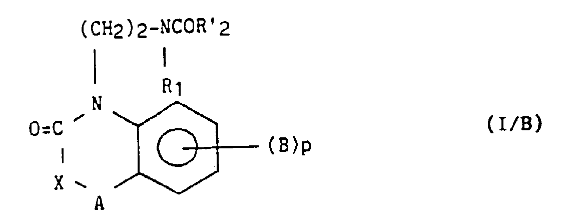 einem Sonderfall der Derivate der Formel (I), in der m, X, A, B, p, R₁ und R'₂ die oben angegebenen Bedeutungen besitzen und B keine Kette -CH₂-CH₂NR₁COR₂ darstellt,
welches man erforderlichenfalls mit Hilfe klassischer Verfahrensweisen, wie der Chromatographie und/oder der Kristallisation, reinigt,
welches Derivat der Formel (I/B) in dem Fall, wo R'₂ eine geradkettige oder verzweigte, durch ein Halogenatom substituierte Niedrigalkylgruppe darstellt, gewünschtenfalls der Einwirkung eines Amins der Formel (X): 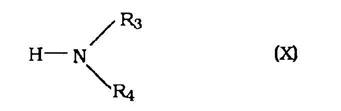 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/A), wie
es oben definiert worden ist, welches gewünschtenfalls mit Hilfe einer klassischen Verfahrensweise, wie der Chromatographie und/oder der Kristallisation, gereinigt und/oder mit einer pharmazeutisch annehmbaren Säure in ein Salz überführt werden kann,
welches Derivat der Formel (I/B), wenn R'₂ einen geradkettigen oder verzweigten Alkylsubstituenten, der mindestens zwei Kohlenstoffatome aufweist und durch ein Halogenatom substituiert ist, darstellt und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C): 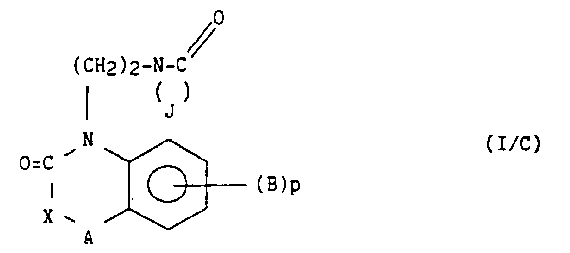 in der B, p, X und A die oben angegebenen Bedeutungen besitzen, wobei es sich versteht, daß B keine Gruppe -CH₂-CH₂NRCOR₂ darstellt und J eine geradkettige oder verzweigte Alkylgruppe mit 2 bis 8 Kohlenstoffatomen darstellt, einem Sonderfall der Derivate der Formel (I), in der R₁ und R₂ zusammen mit NCO ein monocyclisches System bilden, das durch eine Oxogruppe substituiert ist und gegebenenfalls durch eine oder mehrere Niedrigalkylgruppen substituiert ist, welches man gewünschtenfalls mit Hilfe einer aus Kristallisation und Chromatographie ausgewählten Verfahrensweise reinigt.

2. Verfahren zur Herstellung der Verbindungen der Formel (I) nach Anspruch 1, worin B eine Kette -CH₂-CH₂NR₁COR₂ und p 1 bedeuten,
dadurch gekennzeichnet, daß man als Ausgangsmaterial ein Derivat der Formel (VIII): 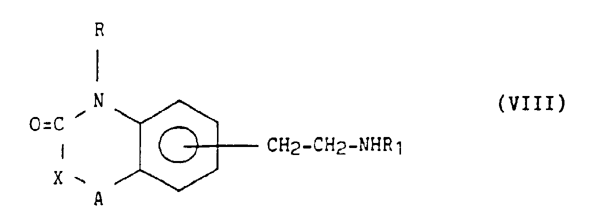 in der R₁; R, X und A die bezüglich der Formel (I) angegebenen Bedeutungen besitzen, verwendet, welches man:
^{*} entweder mit einem Derivat der Formel (VII): 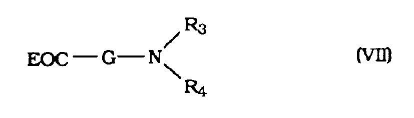 in der E eine aus Hydroxylgruppen, Niedrigalkoxygruppen und Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt, zur Bildung eines Derivats der Formel (I/ D), einem Sonderfall der Derivate der Formel (I): 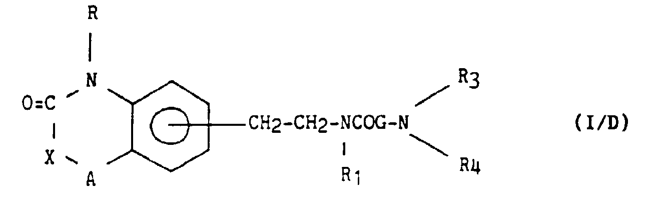 in der X und A, R₁, R₃, R₄ und G die oben angegebenen Bedeutungen besitzen und R ein Wasserstoffatom oder eine Niedrigalkylgruppe bedeutet,
wobei R₂ hier eine Gruppe 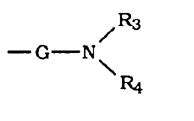 darstellt,
welches man gewünschtenfalls mit Hilfe klassischer Verfahrensweisen, wie der Chromatographie oder der Kristallisation reinigt und gewünschtenfalls mit Hilfe einer pharmazeutisch annehmbaren Säure, oder wenn R ein Wasserstoffatom darstellt, mit Hilfe einer pharmazeutisch annehmbaren Base in ein Salz überführt,
^{*} oder mit einem Säurechlorid der Formel (IX): 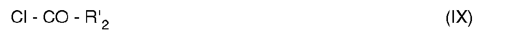 oder einem entsprechenden Säureanhydrid behandelt,
wobei hier R'₂:
- eine geradkettige oder verzweigte Niedrigalkylgruppe oder Cycloalkylgruppe, die gegebenenfalls durch ein Halogenatom substituiert ist oder eine Cycloalkylgruppe, die durch eine geradkettige oder verzweigte Niedrigalkylgruppe substituiert ist,
- eine Arylgruppe oder eine Heteroarylgruppe oder eine Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert sind, wobei der Begriff Heteroarylgruppe die oben angegebenen Bedeutungen besitzt, oder
- eine Imidazolylgruppe, die gegebenenfalls reduziert und/oder durch eine Oxogruppe substituiert ist, darstellt,
zur Bildung eines Derivats der Formel (I/E): 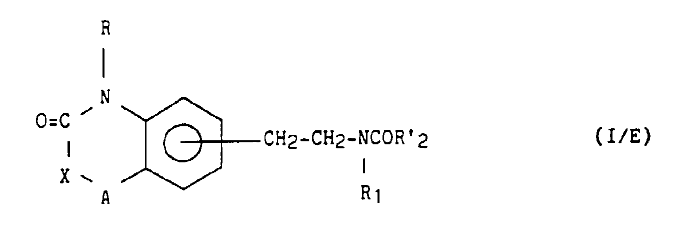 einem Sonderfall der Derivate der Formel (I), in der R₁, X, A und R'₂ die oben angegebenen Bedeutungen besitzen und R eine Niedrigalkylgruppe oder ein Wasserstoffatom darstellt,
welches man erforderlichenfalls mit Hilfe klassischer Verfahrensweisen, wie der Chromatographie und/oder der Kristallisation reinigt, und gewünschtenfalls mit einer pharmazeutisch annehmbaren Base, wenn R ein Wasserstoffatom darstellt, in ein Salz überführt,
welches Derivat der Formel (I/E) dann, wenn R'₂ eine geradkettige oder verzweigte Niedrigalkylgruppe, die durch ein Halogenatom substituiert ist, bedeutet, gewünschtenfalls der Einwirkung eines Amins der Formel (X): 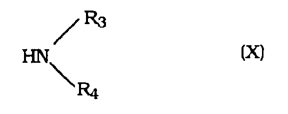 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/D), wie es oben definiert worden ist, welches man gewünschtenfalls mit Hilfe einer klassischen Verfahrensweise, wie der Chromatographie und/oder der Kristallisation reinigt und/oder mit Hilfe einer pharmazeutisch annehmbaren Säure in ein Salz überführt,
welches Derivat der Formel (I/E), wenn R'₂ einen geradkettigen oder verzweigten Alkylsubstituenten darstellt, der mindestens zwei Kohlenstoffatome aufweist und durch ein Halogenatom substituiert ist und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/F): 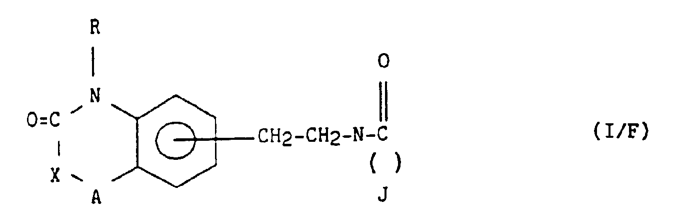 in der R ein Wasserstoffatom oder eine Niedrigalkylgruppe darstellt, X und A die oben angegebenen Bedeutungen besitzen und J eine geradkettige oder verzweigte Alkylgruppe mit 2 bis 8 Kohlenstoffatomen bedeutet, einem Sonderfall der Derivate der Formel (I), in der R₁ und R₂ zusammen mit NCO ein monocyclisches System bilden, das durch eine Oxogruppe substituiert und gegebenenfalls durch eine oder mehrere Niedrigalkylgruppen substituiert ist,
welches man gewünschtenfalls mit Hilfe einer aus Kristallisation oder Chromatographie ausgewählten Verfahrensweise reinigt und gewünschtenfalls, wenn R ein Wasserstoffatom darstellt, mit einer pharmazeutisch annehmbaren Base in ein Salz überführt.

3. Verfahren zur Herstellung der Derivate der Formel (I/K): 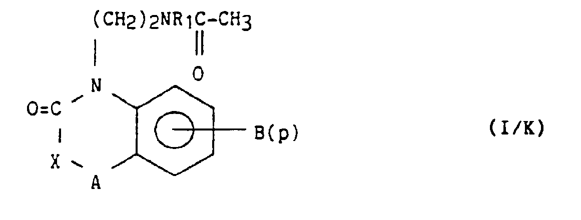 in der R₁, A, X, B und p die bezüglich der Formel (I) angegebenen Bedeutungen besitzen, nach Anspruch 1, durch katalytische Reduktion der Derivate der Formel (V/K): 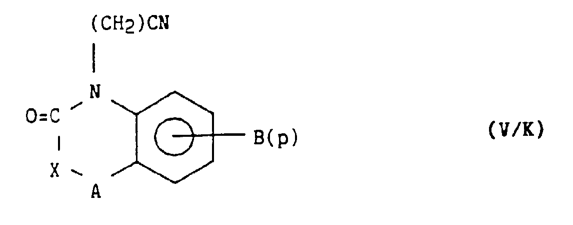 in der X, A, B und p die bezüglich der Formel (I) angegebenen Bedeutungen besitzen, erforderlichenfalls gefolgt von einer Reinigung und wenn R₁ eine Niedrigalkylgruppe darstellt, durch Behandlung mit einem Alkylierungsmittel.

4. Verfahren nach Anspruch 1 zur Herstellung von Verbindungen, worin R ein Kettenglied (CH₂)₂-NR₁-CO-R₂, Beine Methoxygruppe und p 1 bedeuten, von deren Isomeren sowie gegebenenfalls von deren Additionssalzen mit einer pharmazeutisch annehmbaren Säure.

5. Verfahren nach Anspruch 1 zur Herstellung der Verbindungen nach Anspruch 1, worin Reine Kette (CH₂)₂NHCOR₂ darstellt, in der R₂ eine Niedrigalkylgruppe oder eine Cycloalkylgruppe darstellt und B eine Methoxygruppe und p 1 bedeuten.

6. Verfahren nach Anspruch 1 zur Herstellung der Verbindungen nach Anspruch 1, worin B eine Kette (CH₂)₂NHCOR₂ darstellt, in der R₂ eine Niedrigalkylgruppe oder eine Cycloalkylgruppe bedeutet, sowie gegebenenfalls von deren Additionssalzen mit einer pharmazeutisch annehmbaren Base.

7. Verfahren nach Anspruch 1 zur Herstellung der Verbindungen nach Anspruch 1, worin X eine Einfachbindung bedeutet, gegebenenfalls von deren Isomeren und von deren Additionssalzen mit einer pharmazeutisch annehmbaren Säure oder Base.

8. Verfahren nach Anspruch 1 zur Herstellung der Verbindung nach Anspruch 1, nämlich von N-[2-(6-Methoxy-3-benzoxazolinonyl)-ethyl]-acetamid.

9. Verfahren nach Anspruch 1 zur Herstellung der Verbindung nach Anspruch 1, nämlich von N-[2-(5-Methoxy-3-benzoxazolinonyl)-ethyl]-acetamid.

10. Verfahren nach Anspruch 1 zur Herstellung der Verbindung nach Anspruch 1, worin R eine Gruppe CH₂-CH₂NHC0-CH₃, B eine Gruppe OCH₃ und p 1 bedeuten.

11. Verfahren nach Anspruch 1 zur Herstellung der Verbindung nach Anspruch 1, nämlich von N-[2-(6-Methoxy-3-benzothiazolinonyl)-ethyl]-acotamid.

12. Verfahren nach Anspruch 1 zur Herstellung der Verbindung nach Anspruch 1, nämlich von N-[2-(5-Methoxy-3-benzothiazolinonyl)-ethyl]-acetamid.

13. Verfahren nach Anspruch 1 zur Herstellung der Verbindungen nach Anspruch 1, worin R ein Wasserstoffatom oder eine Methylgruppe und B eine Gruppe (CH₂)₂NHCOR₂ darstellen, in der R₂ eine Niedrigalkylgruppe oder eine Cycloalkylgruppe bedeutet sowie gegebenenfalls von deren Additionssalzen mit einer pharmazeutisch annehmbaren Base.

14. Verfahren nach Anspruch 1 zur Herstellung der Verbindung nach Anspruch 1, nämlich von N-[2-(3-Melhyl-6-benzoxazolinonyl)-ethyl]-acetamid.

15. Verfahren nach Anspruch 1 zur Herstellung der Verbindung nach Anspruch 1, nämlich von N-[2-(5-Methoxy-3-benzoxazolinonyl)-ethyl]-cyclopropan-carboxamid.

16. Verfahren nach Anspruch 1 zur Herstellung der Verbindung nach Anspruch 1, nämlich von N-[2-(5-Methoxy-3-benzothiazolinonyl)-ethyl]-cyclopropan-carboxamid.

17. Verfahren zur Herstellung einer pharmazeutischen Zubereitung, die als Wirkstoff mindestens eine Verbindung nach einem der Ansprüche 1 bis 16 in Kombination mit einem oder mehreren inerten, nichttoxischen, pharmazeutisch annehmbaren Trägermaterialien oder Bindemitteln enthält.

18. Verfahren zur Herstellung der pharmazeutischen Zubereitung nach Anspruch 17, die zur Behandlung von Stref3, der Angst, von saisonbedingten Depressionen, von Schlaflosigkeit und Müdigkeit als Folge der Zeitverschiebung, der Schizophrenie, von Panikanfällen, der Melancholie, der Appetitsteuerung, der Schlaflosigkeit, von psychotischen Störungen, der Epilepsie, der Parkinsonschen Krankheit, der senilen Demenz, von verschiedenen Störungen, die mit dem normalen oder pathologischen Altern verknüpft sind, von Gedächtnisverlusten, der Alzheimerschen Krankheit sowie von Störungen der Gehirndurchblutung, von Krebs, der Psoriasis, der Akne, der Seborrhoe oder als Ovulationsinhibitor oder in der Veterinärmedizin bei Störungen des Haarkleids geeignet ist.

## Patentansprüche (Patentansprüche für folgende(n) Vertragsstaat(en) : GR)

1. Verfahren zur Herstellung der Verbindungen der allgemeinen Formel (I): 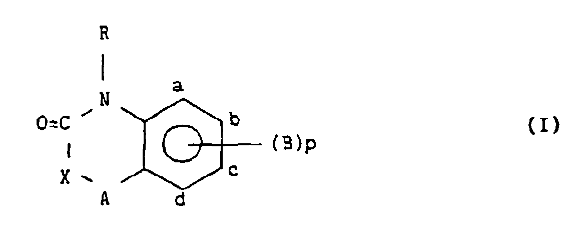 in der
A ein Sauerstoffatom oder ein Schwefelatom.
X eine CH₂-Bindung oder eine Einfachbindung,
R: ^{*} entweder ein Wasserstoffatom oder eine Niedrigalkylgruppe, wobei in diesem Fall p 1 darstellt und Beine Kette -CH₂-CH₂-NR₁-CO-R₂, in der R₁ ein Wasserstoffatom oder eine geradkettige oder verzweigte Niedrigalkylgruppe darstellt,
und R₂^{:}
ein Wasserstoffatom,
eine Cycloalkylgruppe oder eine niedrigmolekulare geradkettige oder verzweigte Alkylgruppe, die gegebenenfalls durch ein Halogenatom substituiert ist,
eine Cycloalkylgruppe, die durch eine geradkettige oder verzweigte Niedrigalkylgruppe substituiert ist,
eine Arylgruppe oder eine Heteroarylgruppe oder eine Arylniedrigalkylgruppe oder eine substituierte Arylgruppe oder eine substituierte Heteroarylgruppe oder eine substituierte Arylalkylgruppe, wobei unter einer Heteroarylgruppe eine ungesättigte mono- oder bicyclische Gruppe zu verstehen ist, die 1 bis 3 Heteroatome ausgewählt aus Stickstoff, Sauerstoff oder Schwefel enthält und jeder Ring 4 oder 5 Kettenglieder aufweist, eine Gruppe der Formel 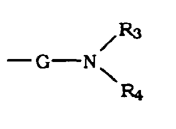 in der
G eine geradkettige oder verzweigte Niedrigalkylgruppe und
R₃ und R₄, die gleichartig oder verschieden sein können, jeweils eine Niedrigalkylgruppe oder ein Wasserstoffatom oder eine Phenylgruppe oder eine Phenylniedrigalkylgruppe darstellen oder R₃ und R₄ mit dem Stickstoffatom, an das sie gebunden sind, ein gegebenenfalls aromatisches, mono- oder bicyclisches heterocyclisches System bilden, das gegebenenfalls ein weiteres Heteroatom ausgewählt aus Stickstoff, Sauerstoff und Schwefel enthält und gegebenenfalls durch eine oder mehrere Niedrigalkylgruppen oder Oxogruppen, Arylgruppen oder Arylniedrigalkylgruppen oder substituierte Arylgruppen oder substituierte Arylniedrigalkylgruppen substituiert ist, mit der Maßgabe, daß bei den Definitionen von R₂, R₃ und R₄ der im Zusammenhang mit den Arylgruppen, Arylalkylgruppen und Heleroarylgruppen verwendete Begriff "substituiert" bedeutet, daß diese Gruppen durch einen oder mehrere Reste ausgewählt aus Niedrigalkylgruppen, Niedrigalkoxygruppen, Trifluormethylgruppen oder Halogenatomen substituiert sind, darstellen
oder R₁ zusammen mit R₂ und der Gruppe N-CO ein heterocyclisches System der Formel 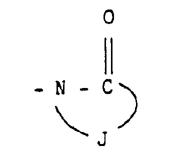
bildet, worin J eine geradkettige oder verzweigte Alkylgruppe mit 2 bis 8 Kohlenstoffatomen darstellt,
^{*} oder eine Kette (CH₂)₂-NR,-CO-R₂, in der R₁ und R₂ die oben angegebenen Bedeutungen besitzen und in diesem Fall p 0 oder 1 und B eine Niedrigalkoxygruppe darstellen, bedeuten,
und gegebenenfalls von deren Isomeren, Epimeren und Diastereoisomeren sowie gegebenenfalls von deren Additionssalzen mit einer pharmazeutisch annehmbaren Säure oder Base, wobei unter Niedrigalkylgruppen und Niedrigalkoxygruppen Gruppen zu verstehen sind, die 1 bis 6 Kohlenstoffatome aufweisen, und die Cycloalkylgruppe für Gruppen mit 3 bis 8 Kohlenstoffatomen steht, dadurch gekennzeichnet, daß, wenn B von der Kette -C H₂CH₂NR₁ COR₂ verschieden ist, man als Ausgangsmaterial ein Derivat der Formel (II) verwendet: 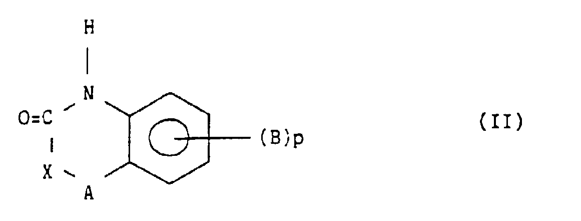 in der A, B, p und X die bezüglich der Formel (I) angegebenen Bedeutungen besitzen, mit Ausnahme des Falls, wo B eine Kette -CH₂-CH₂-NR₁ COR₂ darstellt, welches man mit einem alkalischen Mittel behandelt zur Bildung eines Derivats der Formel (III): 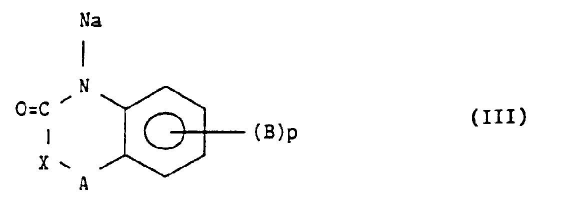 welches Derivat der Formel (III) man:
- entweder mit einem Derivat der Formel (IV/A): 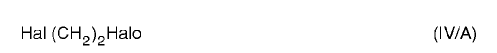 in der Hal und Halo, die gleichartig oder verschieden sein können, Halogenatome darstellen, behandelt, zur Bildung eines Derivats der Formel (V/A): 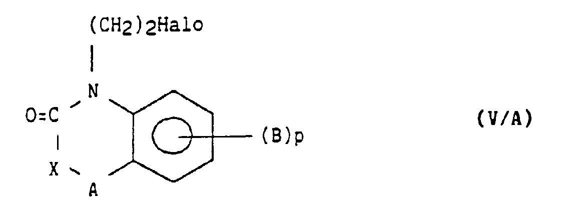 in der A, X, B, p und Halo die oben angegebenen Bedeutungen besitzen, welches man mit einem Amin der Formel HNR₁, in der R₁ die bezüglich der Formel (I) angegebenen Bedeutungen besitzt, behandelt zur Bildung eines Derivats der Formel (VI): 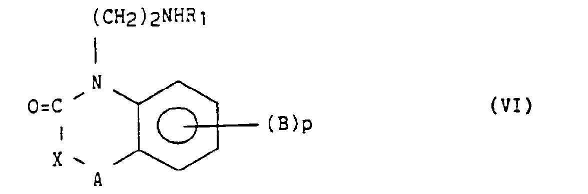 in der A, B, p, R₁ und X die oben angegebenen Bedeutungen besitzen,
- oder mit einem Derivat der Formel (IV/B): 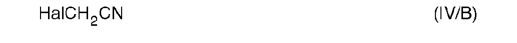 in der Hal die oben angegebenen Bedeutungen besitzt, behandelt, zur Bildung eines Derivats der Formel (V/B): 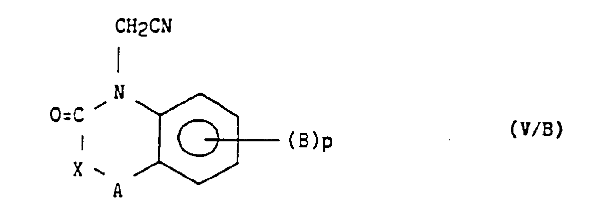 in der X, A, B und p die oben angegebenen Bedeutungen besitzen, welches Derivat der Formel (V/B) man der Einwirkung eines Reduktionsmittels und dann, wenn in dem herzustellenden Derivat der Formel (I) R₁ eine Niedrigalkylgruppe darstellt, mit einem Alkylierungsmittel behandelt zur Bildung eines Derivats der Formel (VI), wie sie oben definiert worden ist,
welches Derivat der Formel (VI) man:
- entweder mit einem Derivat der Formel (VII): 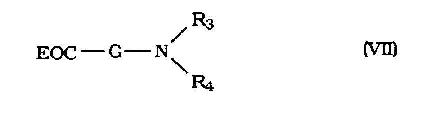 in der E eine aus Hydroxylgruppen, Niedrigalkoxygruppen oder Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt, zur Bildung eines Derivats der Formel (I/A), einem Sonderfall der Derivate der Formel (I): 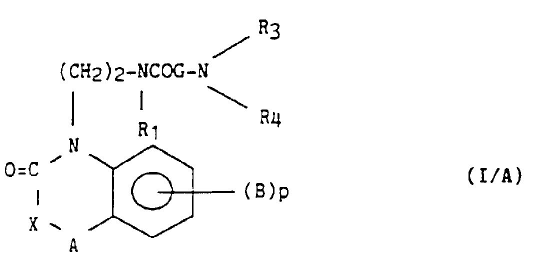 in der A, B, X, R₁, R₃, R₄, G und p die oben angegebenen Bedeutungen besitzen, wobei in diesem Fall R₂ die Gruppe 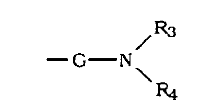 bedeutet, mit der Maßgabe, daß B keine Gruppe: 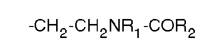 darstellt, welches man gewünschtenfalls mit Hilfe klassischer Verfahrensweisen, wie der Chromatographie oder der Kristallisation, reinigt und gewünschtenfalls mit einer pharmazeutisch annehmbaren Säure in ein Salz überführt,
- oder mit einem Säurechlorid der Formel (IX): 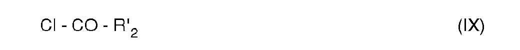 oder mit einem entsprechenden Säureanhydrid behandeld,
wobei in diesem Fall R'₂:
- eine geradkettige oder verzweigte Niedrigalkylgruppe oder Cycloalkylgruppe, die gegebenenfalls durch ein Halogenatom substituiert ist,
- eine Cycloalkylgruppe, die durch eine geradkettige oder verzweigte Niedrigalkylgruppe substituiert ist, oder
- eine Aryl- oder Heteroaryl- oder Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert ist, wobei der Begriff Heteroaryl die oben angegebenen Bedeutungen besitzt,
zur Bildung eines Derivats der Formel (I/B): 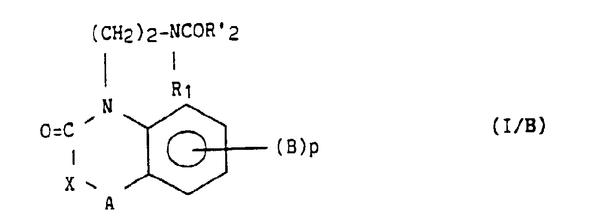 einem Sonderfall der Derivate der Formel (I), in der m, X, A, B, p, R₁ und R'₂ die oben angegebenen Bedeutungen besitzen und B keine Kette -CH₂-CH₂NR₁COR₂ darstellt,
welches man erforderlichenfalls mit Hilfe klassischer Verfahrensweisen, wie der Chromatographie und/oder der Kristallisation, reinigt,
welches Derivat der Formel (I/B) in dem Fall, wo R'₂ eine geradkettige oder verzweigte, durch ein Halogenatom substituierte Niedrigalkylgruppe darstellt, gewünschtenfalls der Einwirkung eines Amins der Formel (X): 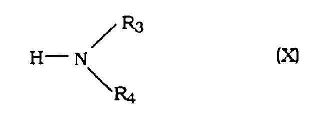 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/A), wie es oben definiert worden ist, welches gewünschtenfalls mit Hilfe einer klassischen Verfahrensweise, wie der Chromatographie und/oder der Kristallisation, gereinigt und/oder mit einer pharmazeutisch annehmbaren Säure in ein Salz überführt werden kann,
welches Derivat der Formel (I/B), wenn R'₂ einen geradkettigen oder verzweigten Alkylsubstituenten, der mindestens zwei Kohlenstoffatome aufweist und durch ein Halogenatom substituiert ist, darstellt und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C): 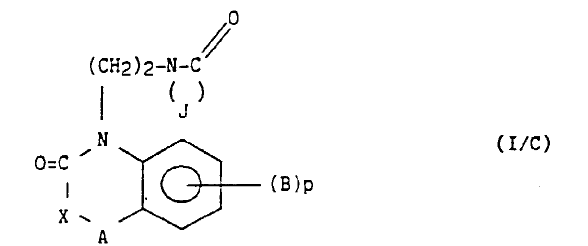 in der B, p, X und A die oben angegebenen Bedeutungen besitzen, wobei es sich versteht, daß B keine Gruppe -CH₂-CH₂NRCOR₂ darstellt und J eine geradkettige oder verzweigte Alkylgruppe mit 2 bis 8 Kohlenstoffatomen darstellt, einem Sonderfall der Derivate der Formel (I), in der R₁ und R₂ zusammen mit NCO ein monocyclisches System bilden, das durch eine Oxogruppe substituiert ist und gegebenenfalls durch eine oder mehrere Niedrigalkylgruppen substituiert ist, welches man gewünschtenfalls mit Hilfe einer aus Kristallisation und Chromatographie ausgewählten Verfahrensweise reinigt.

2. Verfahren zur Herstellung der Verbindungen der Formel (I) nach Anspruch 1, worin B eine Kette -CH₂-CH₂NR₁COR₂ und p 1 bedeuten,
dadurch gekennzeichnet, daß man als Ausgangsmaterial ein Derivat der Formel (VIII): 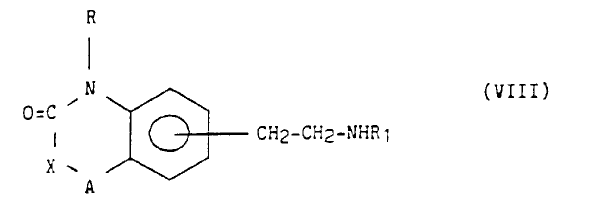 in der R₁; R, X und A die bezüglich der Formel (I) angegebenen Bedeutungen besitzen, verwendet, welches man:
^{*} entweder mit einem Derivat der Formel (VII): 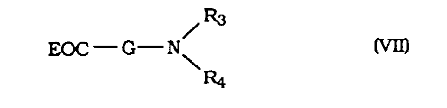 in der E eine aus Hydroxylgruppen, Niedrigalkoxygruppen und Halogenatomen ausgewählte austretende Gruppe darstellt und G, R₃ und R₄ die bezüglich der Formel (I) angegebenen Bedeutungen besitzen, gegebenenfalls in Gegenwart eines alkalischen Mittels behandelt, zur Bildung eines Derivats der Formel (I/ D), einem Sonderfall der Derivate der Formel (I): 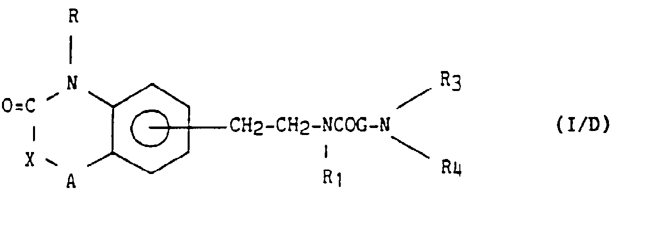 in der X und A, R₁, R₃, R₄ und G die oben angegebenen Bedeutungen besitzen und R ein Wasserstoffatom oder eine Niedrigalkylgruppe bedeutet, wobei R₂ hier eine Gruppe 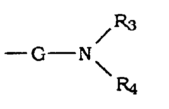 darstellt, welches man gewünschtenfalls mit Hilfe klassischer Verfahrensweisen, wie der Chromatographie oder der Kristallisation reinigt und gewünschtenfalls mit Hilfe einer pharmazeutisch annehmbaren Säure, oder wenn R ein Wasserstoffatom darstellt, mit Hilfe einer pharmazeutisch annehmbaren Base in ein Salz überführt,
^{*} oder mit einem Säurechlorid der Formel (IX): 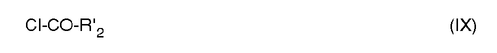 oder einem entsprechenden Säureanhydrid behandelt,
wobei hier R'₂:
- eine geradkettige oder verzweigte Niedrigalkylgruppe oder Cycloalkylgruppe, die gegebenenfalls durch ein Halogenatom substituiert ist oder eine Cycloalkylgruppe, die durch eine geradkettige oder verzweigte Niedrigalkylgruppe substituiert ist,
- eine Arylgruppe oder eine Heteroarylgruppe oder eine Arylniedrigalkylgruppe, die gegebenenfalls durch eines oder mehrere Halogenatome oder Gruppen ausgewählt aus Niedrigalkylgruppen. Niedrigalkoxygruppen oder Trifluormethylgruppen substituiert sind, wobei der Begriff Heteroarylgruppe die oben angegebenen Bedeutungen besitzt, oder
- eine Imidazolylgruppe, die gegebenenfalls reduziert und/oder durch eine Oxogruppe substituiert ist, darstellt,
zur Bildung eines Derivats der Formel (I/E): 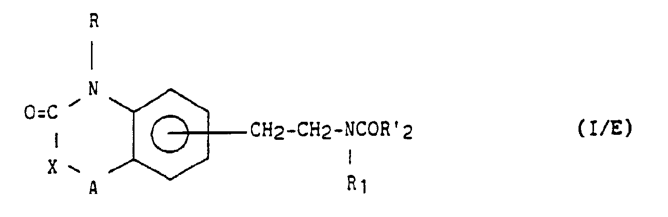 einem Sonderfall der Derivate der Formel (I), in der R₁, X, A und R'₂ die oben angegebenen Bedeutungen besitzen und R eine Niedrigalkylgruppe oder ein Wasserstoffatom darstellt,
welches man erforderlichenfalls mit Hilfe klassischer Verfahrensweisen, wie der Chromatographie und/oder der Kristallisation reinigt, und gewünschtenfalls mit einer pharmazeutisch annehmbaren Base, wenn R ein Wasserstoffatom darstellt, in ein Salz überführt,
welches Derivat der Formel (I/E) dann, wenn R'₂ eine geradkettige oder verzweigte Niedrigalkylgruppe, die durch ein Halogenatom substituiert ist, bedeutet, gewünschtenfalls der Einwirkung eines Amins der Formel (X): 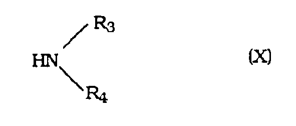 in der R₃ und R₄ die oben angegebenen Bedeutungen besitzen,
im Überschuß oder in Gegenwart eines tertiären Amins oder eines Alkalimetallsalzes unterworfen werden kann zur Bildung eines Derivats der Formel (I/D), wie es oben definiert worden ist, welches man gewünschtenfalls mit Hilfe einer klassischen Verfahrensweise, wie der Chromatographie und/oder der Kristallisation reinigt und/oder mit Hilfe einer pharmazeutisch annehmbaren Säure in ein Salz überführt,
welches Derivat der Formel (I/E), wenn R'₂ einen geradkettigen oder verzweigten Alkylsubstituenten darstellt, der mindestens zwei Kohlenstoffatome aufweist und durch ein Halogenatom substituiert ist und wenn gleichzeitig R₁ ein Wasserstoffatom darstellt, gewünschtenfalls der Einwirkung einer starken Base und vorzugsweise eines Alkalimetallalkoholats unterworfen werden kann zur Bildung eines Derivats der Formel (I/F): 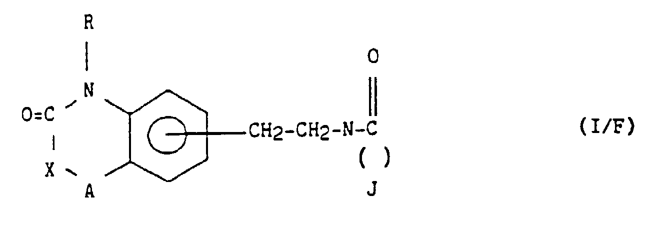 in der R ein Wasserstoffatom oder eine Niedrigalkylgruppe darstellt, X und A die oben angegebenen Bedeutungen besitzen und J eine geradkettige oder verzweigte Alkylgruppe mit 2 bis 8 Kohlenstoffatomen bedeutet, einem Sonderfall der Derivate der Formel (I), in der R₁ und R₂ zusammen mit NCO ein monocyclisches System bilden, das durch eine Oxogruppe substituiert und gegebenenfalls durch eine oder mehrere Niedrigalkylgruppen substituiert ist,
welches man gewünschtenfalls mit Hilfe einer aus Kristallisation oder Chromatographie ausgewählten Verfahrensweise reinigt und gewünschtenfalls, wenn R ein Wasserstoffatom darstellt, mit einer pharmazeutisch annehmbaren Base in ein Salz überführt.

3. Verfahren zur Herstellung der Derivate der Formel (I/K): 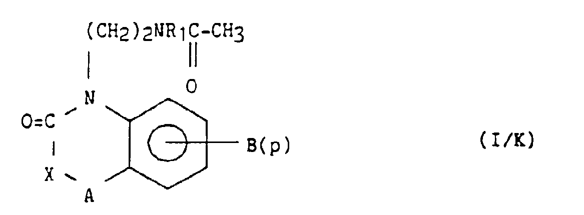 in der R₁, A, X, B und p die bezüglich der Formel (I) angegebenen Bedeutungen besitzen, nach Anspruch 1, durch katalytische Reduktion der Derivate der Formel (V/K): 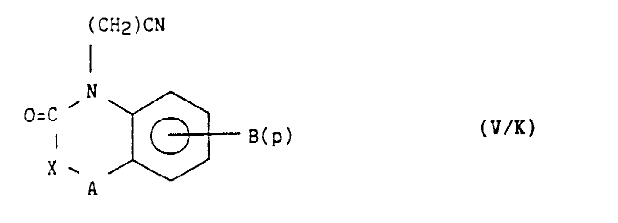 In der X, A, B und p die bezüglich der Formel (I) angegebenen Bedeutungen besitzen, erforderlichenfalls gefolgt von einer Reinigung und wenn R₁ eine Niedrigalkylgruppe darstellt, durch Behandlung mit einem Alkylierungsmittel.

4. Verfahren nach Anspruch 1 zur Herstellung von Verbindungen, worin Rein Kettenglied (CH₂)₂-NR,-CO-R₂, B eine Methoxygruppe und p 1 bedeuten, von deren Isomeren sowie gegebenenfalls von deren Additionssalzen mit einer pharmazeutisch annehmbaren Säure.

5. Verfahren nach Anspruch 1 zur Herstellung der Verbindungen nach Anspruch 1, worin R eine Kette (CH₂)₂NHCOR₂ darstellt, in der R₂ eine Niedrigalkylgruppe oder eine Cycloalkylgruppe darstellt und B eine Methoxygruppe und p 1 bedeuten.

6. Verfahren nach Anspruch 1 zur Herstellung der Verbindungen nach Anspruch 1, worin B eine Kette (CH₂)₂NHCOR₂ darstellt, in der R₂ eine Niedrigalkylgruppe oder eine Cycloalkylgruppe bedeutet, sowie gegebenenfalls von deren Additionssalzen mit einer pharmazeutisch annehmbaren Base.

7. Verfahren nach Anspruch 1 zur Herstellung der Verbindungen nach Anspruch 1, worin X eine Einfachbindung bedeutet, gegebenenfalls von deren Isomeren und von deren Additionssalzen mit einer pharmazeutisch annehmbaren Säure oder Base.

8. Verfahren nach Anspruch 1 zur Herstellung der Verbindung nach Anspruch 1, nämlich von N-[2-(6-Methoxy-3-benzoxazolinonyl)-ethyl]-acetamid.

9. Verfahren nach Anspruch 1 zur Herstellung der Verbindung nach Anspruch 1, nämlich von N-[2-(5-Methoxy-3-benzoxazolinonyl)-ethyl]-acetamid.

10. Verfahren nach Anspruch 1 zur Herstellung der Verbindung nach Anspruch 1, worin R eine Gruppe CH₂-CH₂NHCO-CH₃, B eine Gruppe OCH₃ und p 1 bedeuten.

11. Verfahren nach Anspruch 1 zur Herstellung der Verbindung nach Anspruch 1, nämlich von N-[2-(6-Methoxy-3-benzothiazolinonyl)-ethyl]-acciamid.

12. Verfahren nach Anspruch 1 zur Herstellung der Verbindung nach Anspruch 1, nämlich von N-[2-(5-Methoxy-3-benzothiazolinonyl)-ethyl]-acelamid.

13. Verfahren nach Anspruch 1 zur Herstellung der Verbindungen nach Anspruch 1, worin R ein Wasserstoffatom oder eine Methylgruppe und B eine Gruppe (CH₂)₂NHCOR₂ darstellen, in der R₂ eine Niedrigalkylgruppe oder eine Cycloalkylgruppe bedeutet sowie gegebenenfalls von deren Additionssalzen mit einer pharmazeutisch annehmbaren Base.

14. Verfahren nach Anspruch 1 zur Herstellung der Verbindung nach Anspruch 1, nämlich von N-[2-(3-Melhyl-6-benzoxazolinonyl)-ethyl]-acetamid.

15. Verfahren nach Anspruch 1 zur Herstellung der Verbindung nach Anspruch 1, nämlich von N-[2-(5-Methoxy-3-benzoxazolinonyl)-ethyl]-cyclopropan-carboxamid.

16. Verfahren nach Anspruch 1 zur Herstellung der Verbindung nach Anspruch 1, nämlich von N-[2-(5-Methoxy-3-benzothiazolinonyl)-ethyl]-cyclopropan-carboxamid.

17. Verfahren zur Herstellung einer pharmazeutischen Zubereitung, die als Wirkstoff mindestens eine Verbindung nach einem der Ansprüche 1 bis 16 in Kombination mit einem oder mehreren inerten, nichttoxischen, pharmazeutisch annehmbaren Trägermaterialien oder Bindemitteln enthält.

18. Verfahren zur Herstellung der pharmazeutischen Zubereitung nach Anspruch 17, die zur Behandlung von Streß, der Angst, von saisonbedingten Depressionen, von Schlaflosigkeit und Müdigkeit als Folge der Zeitverschiebung, der Schizophrenie, von Panikanfällen, der Melancholie, der Appetitsteuerung, der Schlaflosigkeit, von psychotischen Störungen, der Epilepsie, der Parkinsonschen Krankheit, der senilen Demenz, von verschiedenen Störungen, die mit dem normalen oder pathologischen Altern verknüpft sind, von Gedächtnisverlusten, der Alzheimerschen Krankheit sowie von Störungen der Gehirndurchblutung, von Krebs, der Psoriasis, der Akne, der Seborrhoe oder als Ovulationsinhibitor oder in der Veterinärmedizin bei Störungen des Haarkleids geeignet ist.

## Claims (Claims for the following Contracting State(s) : AT, BE, CH, DE, DK, FR, GB, IT, LI, LU, MC, NL, PT, SE)

1. Compounds of the general formula (I) : 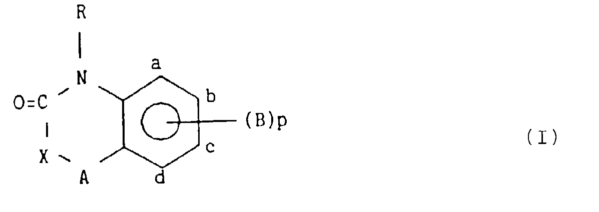 wherein :
A represents an oxygen or sulphur atom,
X represents a CH₂ group or a single bond,
R represents:
^{*} either a hydrogen atom or a lower alkyl group, in which case p = and B represents a -CH₂-CH₂-NR₁-CO-R₂ chain in which R₁ represents a hydrogen atom or a straight-chain or branched lower alkyl group,
and R₂ represents :
a hydrogen atom,
a cycloalkyl group or a straight-chain or branched lower alkyl group each optionally substituted by a halogen atom,
a cycloalkyl group substituted by a straight-chain or branched lower alkyl group,
an aryl or heteroaryl or aryl-lower alkyl or substituted aryl or substituted heteroaryl or substituted arylalkyl group, there being understood by heteroaryl group an unsaturated mono- or bi-cyclic group containing from 1 to 3 hetero atoms selected from nitrogen, oxygen and sulphur, each ring comprising 4 or 5 ring members,
a group of formula: 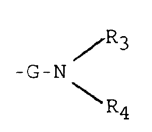
G representing a straight-chain or branched lower alkyl group, and
R₃ and R₄, which are identical or different, each representing a lower alkyl group or a hydrogen atom or a phenyl or phenyl-lower alkyl group, or
R₃ and R₄ forming together with the nitrogen atom to which they are attached an aromatic or nonaromatic, mono- or bi-cyclic heterocyclic system that optionally contains an additional hetero atom selected from nitrogen, oxygen and sulphur and is optionally substituted by one or more lower alkyl, oxo, aryl, aryl-lower alkyl,
substituted aryl or substituted aryl-lower alkyl groups,
it being understood that in the definitions of R₂, R₃ and R₄ the term substituted defining the aryl and arylalkyl and heteroaryl groups denotes that those groups are substituted by one or more radicals selected from lower alkyl, lower alkoxy, trifluoromethyl and halogen,
or Rᵢ forms together with R₂ and the group N-CO a heterocyclic system of formula : 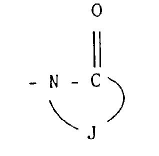
in which J is a straight-chain or branched alkyl radical having from 2 to 8 carbon atoms,
^{*} or a (CH₂)₂-NR₁-CO-R₂ chain in which R₁ and R₂ have the same meaning as above, in which case p is 0 or 1 and B represents a lower alkoxy group,
where appropriate their isomers, epimers and diastereoisomers and also, where appropriate, the addition salts thereof with a pharmaceutically acceptable acid or base, it being understood that lower alkyl and lower alkoxy denote groups containing from 1 to 6 carbon atoms and that cycloalkyl denotes groups containing from 3 to 8 carbon atoms.

2. Compounds according to claim 1 wherein R represents a-(CH2)2-NRi-CO-R2 chain, B represents a methoxy group and p represents 1, their isomers and also, where appropriate, the addition salts thereof with a pharmaceutically acceptable acid.

3. Compounds according to claim 1 wherein R represents a (CH₂)₂NHCOR₂ chain in which R₂ represents a lower alkyl or cycloalkyl group, and B represents a methoxy group and p is 1.

4. Compounds according to claim 1 wherein B represents a (CH₂)₂NHCOR₂ chain in which R₂ represents a lower alkyl or cycloalkyl group, and also, where appropriate, the addition salts thereof with a pharmaceutically acceptable base.

5. Compounds according to claim 1 wherein X represents a single bond, where appropriate their isomers, and the addition salts thereof with a pharmaceutically acceptable acid or base.

6. Compound according to claim 1 that is N-[2-(6-methoxy-3-benzoxazolinonyl)ethyl]acetamide.

7. Compound according to claim 1 that is N-[2-(5-methoxy-3-benzoxazolinonyl)ethyl]acetamide.

8. Compound according to claim 1 in which R represents a CH₂-CH₂NHCO-CH₃ group, B represents an OCH₃ group and p is 1.

9. Compound according to claim 1 that is N-[2-(6-methoxy-3-benzothiazolinonyl)ethyl]acetamide.

10. Compound according to claim 1 that is N-[2-(5-methoxy-3-benzothiazolinonyl)ethyl]acetamide.

11. Compounds according to claim 1 wherein R represents a hydrogen atom or a methyl group and B represents a (CH₂)₂NHCOR₂ group in which R₂ represents a lower alkyl or cycloalkyl group, and also, where appropriate, the addition salts thereof with a pharmaceutically acceptable base.

12. Compound according to claim 1 that is N-[2-(3-methyl-6-benzoxazolinonyl)ethyl]acetamide.

13. Compound according to claim 1 that is N-[2-(5-methoxy-3-benzoxazolinonyl)ethyl]cyclopropane carboxamide.

14. Compound according to claim 1 that is N-[2-(5-methoxy-3-benzothiazolinonyl)ethyl]cyclopropane carboxamide.

15. Process for the preparation of compounds of formula (I) according to claim 1 wherein B is other than the chain -CH₂-CH₂NR₁COR₂,
characterised in that the starting material used is a compound of formula (II): 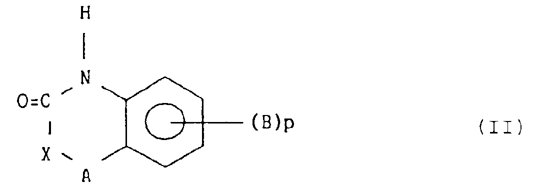 wherein A, B, p and X are as defined for formula (I), with the exception of the case where B represents a -CH₂-CH₂-NR₁COR₂ chain,
which is treated with an alkaline agent to obtain a compound of formula (III): 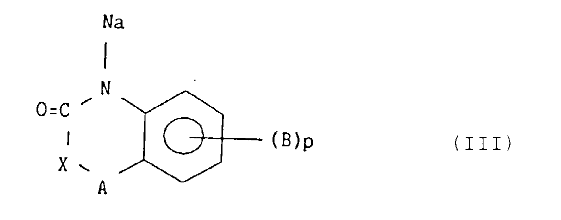 which compound of formula (III) is treated :
- either with a compound of formula (IV/A) : 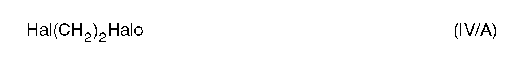 wherein Hal and Halo, which are identical or different, each represents a halogen atom to obtain a compound of formula (VIA) : 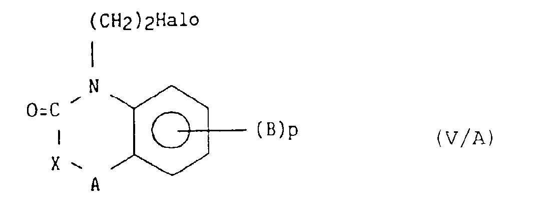 wherein A, X, B, p and Halo are as defined above,
which is treated with an amine of formula HNR₁ in which R₁ is as defined for formula (I), to obtain a compound of formula (VI) : 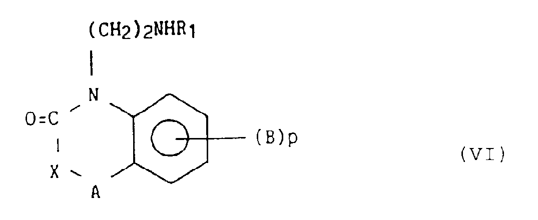 wherein A, B, p, R₁ and X are as defined above,
- or with a compound of formula (IV/B) : 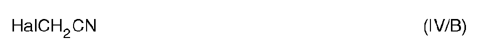 wherein Hal is as defined above,
to obtain a compound of formula (V/B) : 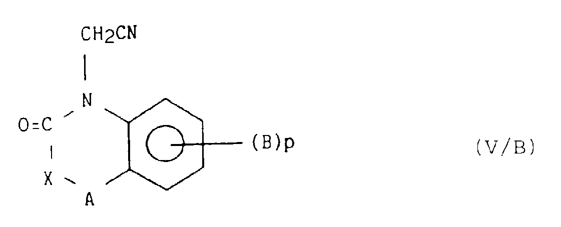 wherein X, A, B and p are as defined above,
which compound of formula (V/B) is subjected to the action of a reducing agent and then, if in the compound of formula (I) it is desired to obtain R₁ represents a lower alkyl group, to the action of an alkylating agent to obtain a compound of formula (VI) as defined above,
which compound of formula (VI) is treated :
- either with a compound of formula (VII): 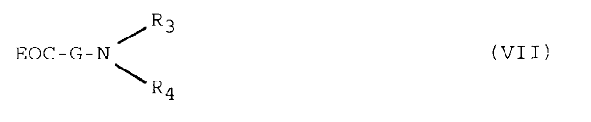 wherein E denotes a leaving group selected from hydroxyl, lower alkoxy and halogen, G, R₃ and R₄ having the same meaning as in formula (I), optionally in the presence of an alkaline agent,
to yield a compound of formula (I/A) : 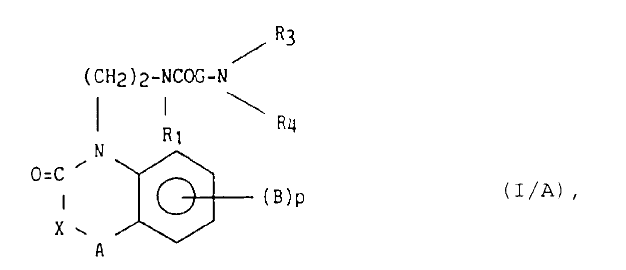 a particular case of compounds of formula (I) wherein A, B, X, R₁, R₃, R₄, G and p are as defined above, R₂ here denoting a group : 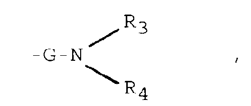 with the proviso that B does not represent a group : 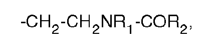 which, if desired, is purified by conventional techniques such as chromatography or crystallisation and which, if desired, is converted into a salt with a pharmaceutically acceptable acid,
or with an acid chloride of formula (IX) : 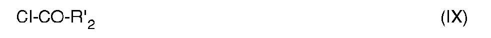 or with the corresponding acid anhydride,
R'₂ here denoting :
- a straight-chain or branched lower alkyl group or a cycloalkyl group each optionally substituted by a halogen atom,
- a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
to yield a compound of formula (I/B) : 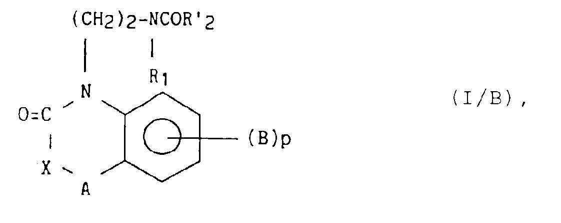 a particular case of compounds of formula (I) wherein m, X, A, B, p, R₁ and R'₂ are as defined above, it not being possible for B to represent a -CH₂-CH₂-NR₁COR₂ chain,
which, if necessary, is purified by conventional techniques such as chromatography and/or crystallisation, which compound of formula (I/B), when R'₂ represents a straight-chain or branched lower alkyl group substituted by a halogen atom, may, if desired, be subjected to the action of an amine of formula (X) : 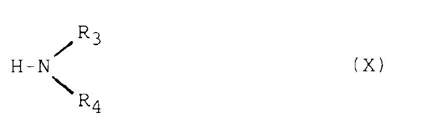 wherein R₃ and R₄ are as defined above,
in excess or in the presence of a tertiary amine or an alkali metal salt to yield a compound of formula (I/A) as defined above which, if desired, is purified by a conventional technique such as chromatography and/or crystallisation, and/or converted into a salt with a pharmaceutically acceptable acid,
which compound of formula (I/B), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/C) : 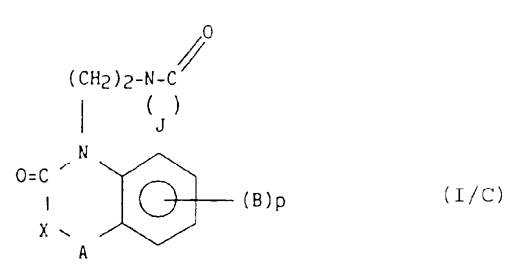 wherein B, p, X and A are as defined above, it being understood that B cannot represent a -CH₂-CH₂NRCOR₂ group, and J represents a straight-chain or branched alkyl group containing from 2 to 8 carbon atoms, a particular case of compounds of formula (I) in which R₁ and R₂ form together with NCO a monocyclic system substituted by an oxo group and optionally substituted by one or more lower alkyl groups,
which, if desired, is purified by a technique selected from crystallisation and chromatography

16. Process for the preparation of compounds of formula (I) according to claim 1 wherein B represents a -CH₂-CH₂NR₁COR₂ chain and p represents 1,
characterised in that the starting material used is a compound of formula (VIII) : 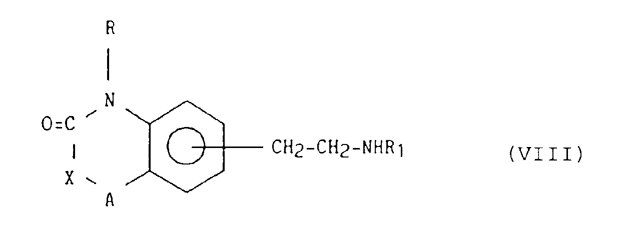 wherein R₁, R, X and A are as defined for formula (I), which is treated :
^{*} either with a compound of formula (VII) : 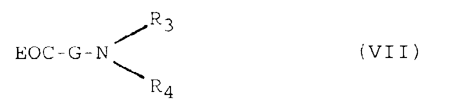
wherein E denotes a leaving group selected from hydroxyl, lower alkoxy and halogen, G, R₃ and R₄ having the same meaning as in formula (I),
optionally in the presence of an alkaline agent,
to yield a compound of formula (I/D) : 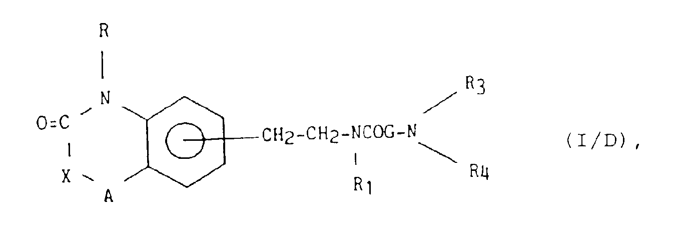
a particular case of compounds of formula (I) wherein X and A, R₁, R₃, R₄ and G are as defined above and R represents a hydrogen atom or a lower alkyl group,
R₂ here denoting a group : 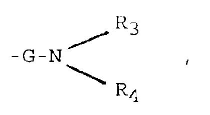
which, if desired, is purified by conventional techniques, such as chromatography, crystallisation and, if desired, is converted into a salt with a pharmaceutically acceptable acid or, when R represents a hydrogen atom, with a pharmaceutically acceptable base,
^{*} or with an acid chloride of formula (IX) : 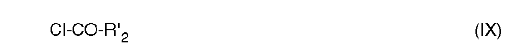 or with the corresponding acid anhydride,
R'₂ here denoting :
- a straight-chain or branched lower alkyl group or a cycloalkyl group each optionally substituted by a halogen atom, or a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
- an imidazolyl group optionally reduced and/or substituted by an oxo group, to yield a compound of formula (I/E) : 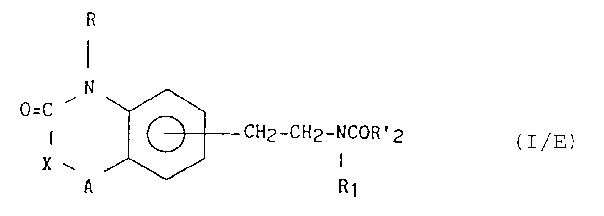 a particular case of compounds of formula (I) wherein R₁, X, A and R'₂ are as defined above, and R represents a lower alkyl group or a hydrogen atom,
which, if necessary, is purified by conventional techniques such as chromatography and/or crystallisation and, if desired, is converted into a salt with a pharmaceutically acceptable base when R represents a hydrogen atom, which compound of formula (I/E), when R'₂ represents a straight-chain or branched lower alkyl group substituted by a halogen atom, may, if desired, be subjected to the action of an amine of formula (X) : 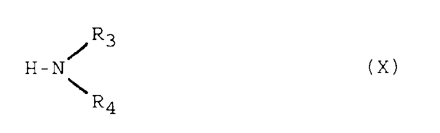 wherein R₃ and R₄ are as defined above,
in excess or in the presence of a tertiary amine or an alkali metal salt to yield a compound of formula (I/D) as defined above which, if desired, is purified by a conventional technique such as chromatography and/or crystallisation, and/or is converted into a salt with a pharmaceutically acceptable acid,
which compound of formula (I/E), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/F) : 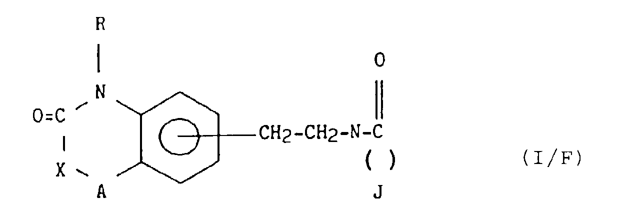 wherein R represents a hydrogen atom or a lower alkyl group, X and A are as defined above and J represents a straight-chain or branched alkyl group containing from 2 to 8 carbon atoms, a particular case of compounds of formula (I) wherein R₁ and R₂ form together with NCO a monocyclic system substituted by an oxo group and optionally substituted by one or more lower alkyl groups,
which, if desired, is purified by a technique selected from crystallisation and chromatography and, if desired, is converted into a salt with a pharmaceutically accept able base when R represents a hydrogen atom.

17. Process for the preparation of compounds of formula (I/K) : 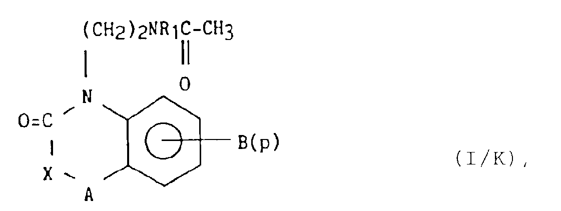 wherein R₁, A, X, Band pare as defined for formula (I) according to claim 1, by catalytic reduction of the compounds of formula (V/K) 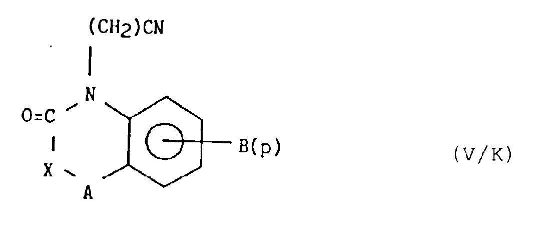 wherein X, A, B and p are as defined for formula (I), followed, if necessary, by purification and, when R₁ represents a lower alkyl group, by treatment with an alkylating agent.

18. Pharmaceutical composition comprising as active ingredient at least one compound according to any one of claims 1 to 14 in combination with one or more pharmaceutically acceptable, inert, non-toxic excipients or carriers.

19. Pharmaceutical composition according to claim 18 for use in the treatment of stress, anxiety, seasonal depression, insomnia and fatigue resulting from shiftwork, schizophrenia, panic attack, melancholia, regulation of the appetite, insomnia, psychotic disorders, epilepsy, Parkinson's disease, senile dementia, various disorders associated with normal or pathological ageing, memory loss, Alzheimer's disease, and also cerebral circulation disorders, cancer, psoriasis, acne, seborrhoea, or as an ovulation inhibitor or in veterinary medicine in coat disorders.

## Claims (Claims for the following Contracting State(s) : ES)

1. Process for the preparation of compounds of the general formula (I) : 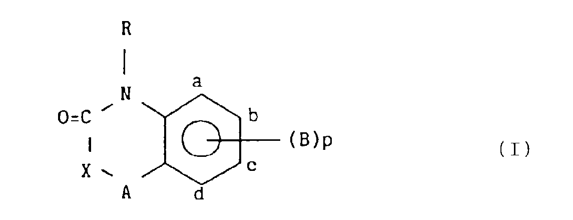 wherein :
A represents an oxygen or sulphur atom,
X represents a CH₂ group or a single bond,
R represents:
^{*} either a hydrogen atom or a lower alkyl group, in which case p = 1 and B represents a -CH₂-CH₂-NR₁-CO-R₂ chain in which R₁ represents a hydrogen atom or a straight-chain or branched lower alkyl group,
and R₂ represents :
a hydrogen atom,
a cycloalkyl group or a straight-chain or branched lower alkyl group each optionally substituted by a halogen atom,
a cycloalkyl group substituted by a straight-chain or branched lower alkyl group,
an aryl or heteroaryl or aryl-lower alkyl or substituted aryl or substituted heteroaryl or substituted arylalkyl group, there being understood by heteroaryl group an unsaturated mono- or bi-cyclic group containing from 1 to 3 hetero atoms selected from nitrogen, oxygen and sulphur, each ring comprising 4 or 5 ring members,
a group of formula: 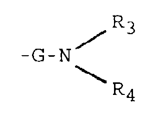
G representing a straight-chain or branched lower alkyl group, and
R₃ and R₄, which are identical or different, each representing a lower alkyl group or a hydrogen atom or a phenyl or phenyl-lower alkyl group, or
R₃ and R₄ forming together with the nitrogen atom to which they are attached an aromatic or nonaromatic, mono- or bi-cyclic heterocyclic system that optionally contains an additional hetero atom selected from nitrogen, oxygen and sulphur and is optionally substituted by one or more lower alkyl, oxo, aryl, aryl-lower alkyl,
substituted aryl or substituted aryl-lower alkyl groups,
it being understood that in the definitions of
R₂, R₃ and R₄ the term substituted defining the aryl and arylalkyl and heteroaryl groups denotes that those groups are substituted by one or more radicals selected from lower alkyl, lower alkoxy, trifluoromethyl and halogen,
or R₁ forms together with R₂ and the group N-CO a heterocyclic system of formula : 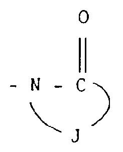
in which J is a straight-chain or branched alkyl radical having from 2 to 8 carbon atoms,
^{*} or a (CH₂)₂-NR₁-CO-R₂ chain in which R₁ and R₂ have the same meaning as above, in which case p is 0 or 1 and B represents a lower alkoxy group,
where appropriate their isomers, epimers and diastereoisomers and also, where appropriate, the addition salts thereof with a pharmaceutically acceptable acid or base, it being understood that lower alkyl and lower alkoxy denote groups containing from 1 to 6 carbon atoms and that cycloalkyl denotes groups containing from 3 to 8 carbon atoms,
characterised, when B is other than the chain -CH₂CH₂NR,COR₂, in that the starting material used is a compound of formula (II) : 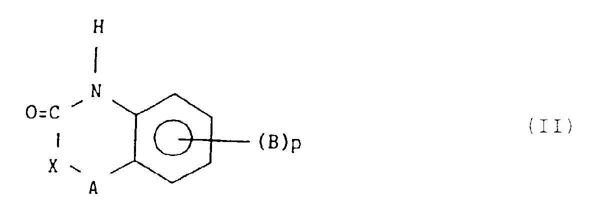
wherein A, B, p and X are as defined for formula (I), with the exception of the case where B represents a -CH₂-CH₂-NR₁COR₂ chain,
which is treated with an alkaline agent to obtain a compound of formula (III) : 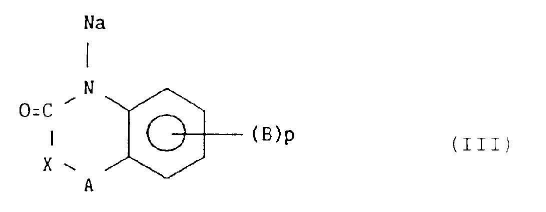
which compound of formula (III) is treated :
- either with a compound of formula (IV/A) : 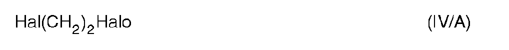 wherein Hal and Halo, which are identical or different, each represents a halogen atom to obtain a compound of formula (V/A) : 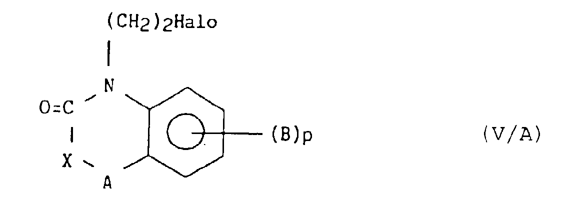 wherein A, X, B, p and Halo are as defined above,
which is treated with an amine of formula HNR₁ in which R₁ is as defined for formula (I), to obtain a compound of formula (VI) : 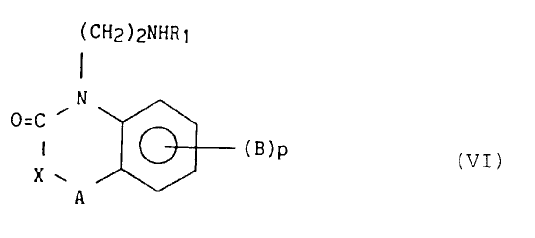 wherein A, B, p, R₁ and X are as defined above,
- or with a compound of formula (IV/B) : 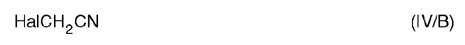 wherein Hal is as defined above,
to obtain a compound of formula (V/B) : 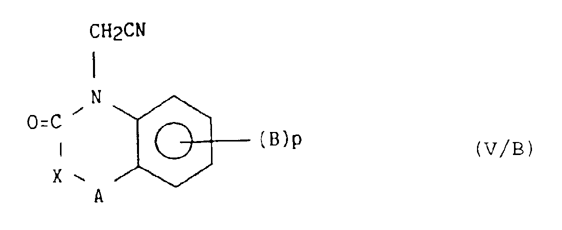 wherein X, A, B and p are as defined above,
which compound of formula (V/B) is subjected to the action of a reducing agent and then, if in the compound of formula (I) it is desired to obtain R₁ represents a lower alkyl group, to the action of an alkylating agent to obtain a compound of formula (VI) as defined above,
which compound of formula (VI) is treated :
- either with a compound of formula (VII) : 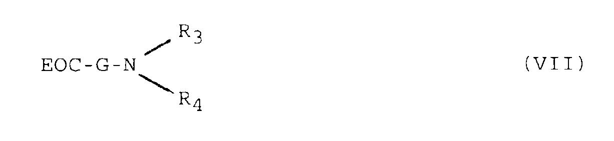 wherein E denotes a leaving group selected from hydroxyl, lower alkoxy and halogen, G, R₃ and R₄ having the same meaning as in formula (I), optionally in the presence of an alkaline agent,
to yield a compound of formula (I/A) : 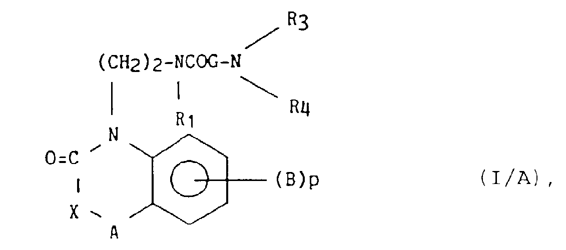 a particular case of compounds of formula (I) wherein A, B, X, R₁, R₃, R₄, G and p are as defined above, R₂ here denoting a group : 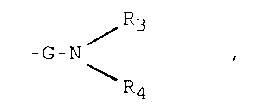 with the proviso that B does not represent a group : 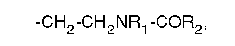 which, if desired, is purified by conventional techniques such as chromatography or crystallisation and which, if desired, is converted into a salt with a pharmaceutically acceptable acid,
- or with an acid chloride of formula (IX) : 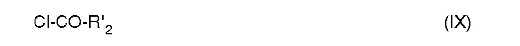 or with the corresponding acid anhydride,
R'₂ here denoting:
- a straight-chain or branched lower alkyl group or a cycloalkyl group each optionally substituted by a halogen atom,
- a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
to yield a compound of formula (I/B) : 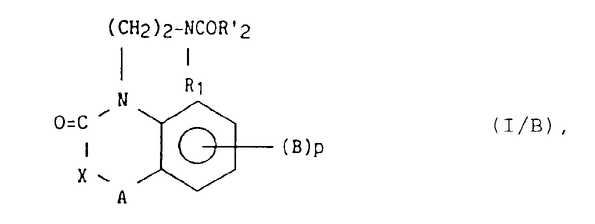 a particular case of compounds of formula (I) wherein m, X, A, B, p, R₁ and R'₂ are as defined above, it not being possible for B to represent a -CH₂-CH₂-NR,COR₂ chain,
which, if necessary, is purified by conventional techniques such as chromatography and/or crystallisation, which compound of formula (I/B), when R'₂ represents a straight-chain or branched lower alkyl group substituted by a halogen atom, may, if desired, be subjected to the action of an amine of formula (X) : 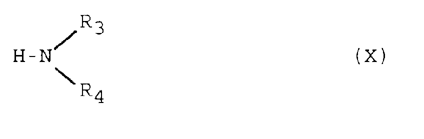 wherein R₃ and R₄ are as defined above,
in excess or in the presence of a tertiary amine or an alkali metal salt to yield a compound of formula (I/A) as defined above which, if desired, is purified by a conventional technique such as chromatography and/or crystallisation, and/or converted into a salt with a pharmaceutically acceptable acid,
which compound of formula (I/B), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/C) : 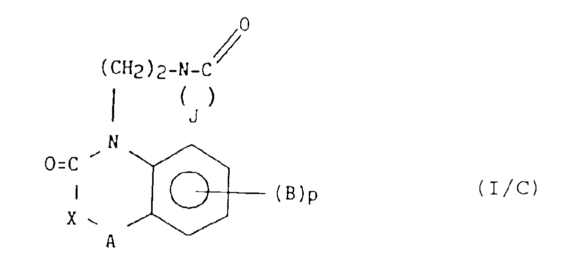 wherein B, p, X and A are as defined above, it being understood that B cannot represent a -CH₂-CH₂NRCOR₂ group, and J represents a straight-chain or branched alkyl group containing from 2 to 8 carbon atoms, a particular case of compounds of formula (I) in which R₁ and R₂ form together with NCO a monocyclic system substituted by an oxo group and optionally substituted by one or more lower alkyl groups,
which, if desired, is purified by a technique selected from crystallisation and chromatography.

2. Process for the preparation of compounds of formula (I) according to claim 1 wherein B represents a -CH₂-CH₂-NR₁COR₂ chain and p represents 1, characterised in that the starting material used is a compound of formula (VIII) : 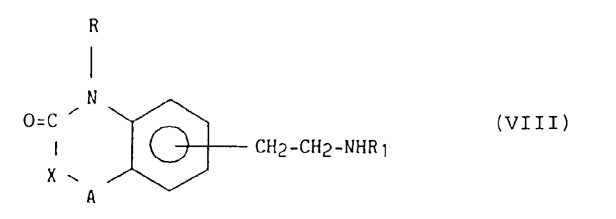 wherein R₁, R, X and A are as defined for formula (I), which is treated :
^{*} either with a compound of formula (VII) : 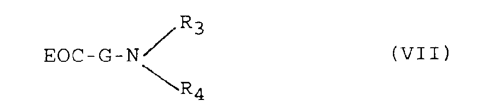
wherein E denotes a leaving group selected from hydroxyl, lower alkoxy and halogen, G, R₃ and R₄ having the same meaning as in formula (I),
optionally in the presence of an alkaline agent,
to yield a compound of formula (I/D) : 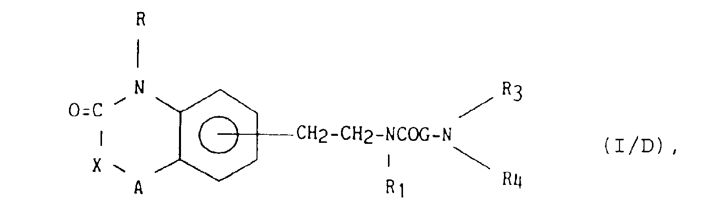
a particular case of compounds of formula (I) wherein X and A, R₁, R₃, R₄ and G are as defined above and R represents a hydrogen atom or a lower alkyl group,
R₂ here denoting a group : 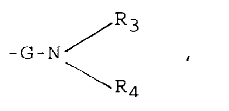
which, if desired, is purified by conventional techniques, such as chromatography, crystallisation and, if desired, is converted into a salt with a pharmaceutically acceptable acid or, when R represents a hydrogen atom, with a pharmaceutically acceptable base,
^{*} or with an acid chloride of formula (IX) : 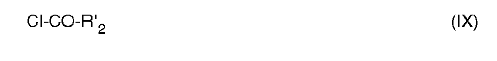
or with the corresponding acid anhydride,
R'₂ here denoting :
- a straight-chain or branched lower alkyl group or a cycloalkyl group each optionally substituted by a halogen atom, or a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
- an imidazolyl group optionally reduced and/or substituted by an oxo group,
to yield a compound of formula (I/E) : 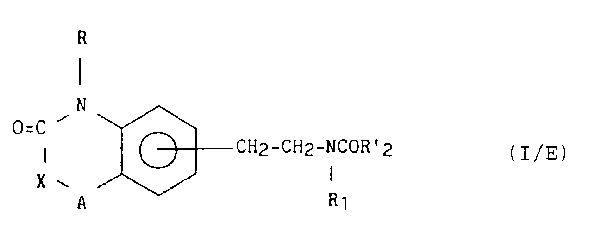 a particular case of compounds of formula (I) wherein Rᵢ, X, A and R'₂ are as defined above, and R represents a lower alkyl group or a hydrogen atom,
which, if necessary, is purified by conventional techniques such as chromatography and/or crystallisation and, if desired, is converted into a salt with a pharmaceutically acceptable base when R represents a hydrogen atom, which compound of formula (I/E), when R'₂ represents a straight-chain or branched lower alkyl group substituted by a halogen atom, may, if desired, be subjected to the action of an amine of formula (X) : 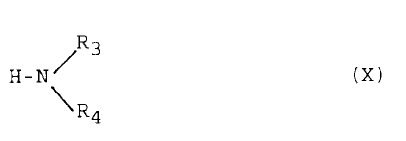 wherein R₃ and R₄ are as defined above,
in excess or in the presence of a tertiary amine or an alkali metal salt to yield a compound of formula (I/D) as defined above which, if desired, is purified by a conventional technique such as chromatography and/or crystallisation, and/or is converted into a salt with a pharmaceutically acceptable acid,
which compound of formula (I/E), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/F) : 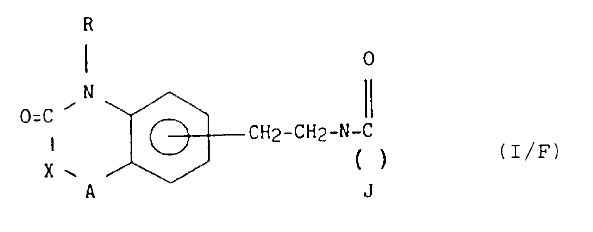 wherein R represents a hydrogen atom or a lower alkyl group, X and A are as defined above and J represents a straight-chain or branched alkyl group containing from 2 to 8 carbon atoms, a particular case of compounds of formula (I) wherein R₁ and R₂ form together with NCO a monocyclic system substituted by an oxo group and optionally substituted by one or more lower alkyl groups,
which, if desired, is purified by a technique selected from crystallisation and chromatography and, if desired, is converted into a salt with a pharmaceutically acceptable base when R represents a hydrogen atom.

3. Process for the preparation of compounds of formula (I/K) 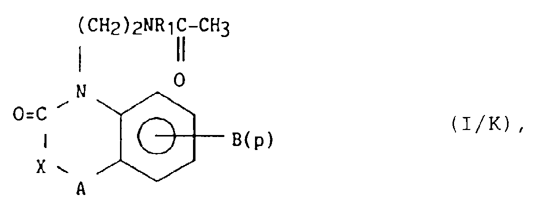 wherein R₁, A, X, Band pare as defined for formula (I) according to claim 1, by catalytic reduction of the compounds of formula (V/K) 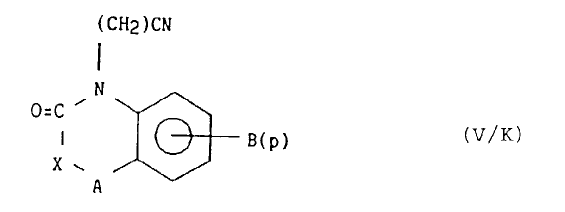 wherein X, A, B and p are as defined for formula (I), followed, if necessary, by purification and, when R₁ represents a lower alkyl group, by treatment with an alkylating agent.

4. Process according to claim 1 for the preparation of compounds wherein R represents a -(CH₂)₂-NR₁-CO-R₂ chain, B represents a methoxy group and p represents 1, their isomers and also, where appropriate, the addition salts thereof with a pharmaceutically acceptable acid.

5. Process according to claim 1 for the preparation of compounds according to claim 1 wherein R represents a (CH₂)₂NHCOR₂ chain in which R₂ represents a lower alkyl or cycloalkyl group, and B represents a methoxy group and p is 1.

6. Process according to claim 1 for the preparation of compounds according to claim 1 wherein B represents a (CH₂)₂NHCOR₂ chain in which R₂ represents a lower alkyl or cycloalkyl group, and also, where appropriate, the addition salts thereof with a pharmaceutically acceptable base.

7. Process according to claim 1 for the preparation of compounds according to claim 1 wherein X represents a single bond, where appropriate their isomers, and the addition salts thereof with a pharmaceutically acceptable acid or base.

8. Process according to claim 1 for the preparation of the compound according to claim 1 that is N-[2-(6-methoxy-3-benzoxazolinonyl)ethyllacetamide.

9. Process according to claim 1 for the preparation of the compound according to claim 1 that is N-[2-(5-methoxy-3-benzoxazolinonyl)ethyllacetamide.

10. Process according to claim 1 for the preparation of the compound according to claim 1 in which R represents a CH₂-CH₂NHCO-CH₃ group, B represents an OCH₃ group and p is 1.

11. Process according to claim 1 for the preparation of the compound according to claim 1 that is N-[2-(6-methoxy-3-benzothiazolinonyl)ethyl]acetamide.

12. Process according to claim 1 for the preparation of the compound according to claim 1 that is N-[2-(5-methoxy-3-benzothiazolinonyl)ethyl]acetamide.

13. Process according to claim 1 for the preparation of compounds according to claim 1 wherein R represents a hydrogen atom or a methyl group and B represents a (CH₂)₂NHCOR₂ group in which R₂ represents a lower alkyl or cycloalkyl group, and also, where appropriate, the addition salts thereof with a pharmaceutically acceptable base.

14. Process according to claim 1 for the preparation of the compound according to claim 1 that is N-[2-(3-methyl-6-benzoxazolinonyl)ethyllacetamide.

15. Process according to claim 1 for the preparation of the compound according to claim 1 that is N-[2-(5-methoxy-3-benzoxazolinonyl)ethyl]cyclopropane carboxamide.

16. Process according to claim 1 for the preparation of the compound according to claim 1 that is N-[2-(5-methoxy-3-benzothiazolinonyl)ethyl]cyclopropane carboxamide.

17. Process for the preparation of a pharmaceutical composition comprising as active ingredient at least one compound according to any one of claims 1 to 16 in combination with one or more pharmaceutically acceptable, inert, non-toxic excipients or carriers.

18. Process for the preparation of a pharmaceutical composition according to claim 17 for use in the treatment of stress, anxiety, seasonal depression, insomnia and fatigue resulting from shiftwork, schizophrenia, panic attack, melancholia, regulation of the appetite, insomnia, psychotic disorders, epilepsy, Parkinson's disease, senile dementia, various disorders associated with normal or pathological ageing, memory loss, Alzheimer's disease, and also cerebral circulation disorders, cancer, psoriasis, acne, seborrhoea, or as an ovulation inhibitor or in veterinary medicine in coat disorders.

## Claims (Claims for the following Contracting State(s) : GR)

1. Process for the preparation of compounds of the general formula (I) : 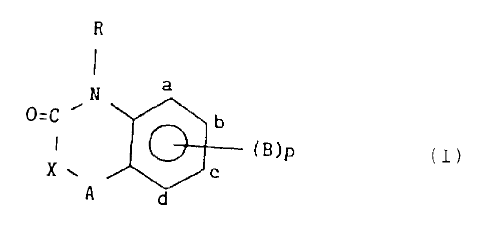 wherein :
A represents an oxygen or sulphur atom,
X represents a CH₂ group or a single bond,
R represents :
^{*} either a hydrogen atom or a lower alkyl group, in which case p = 1 and B represents a -CH₂-CH₂-NR₁-CO-R₂ chain in which R₁ represents a hydrogen atom or a straight-chain or branched lower alkyl group, and R₂ represents:
a hydrogen atom,
a cycloalkyl group or a straight-chain or branched lower alkyl group each optionally substituted by a halogen atom,
a cycloalkyl group substituted by a straight-chain or branched lower alkyl group,
an aryl or heteroaryl or aryl-lower alkyl or substituted aryl or substituted heteroaryl or substituted arylalkyl group, there being understood by heteroaryl group an unsaturated mono- or bi-cyclic group containing from 1 to 3 hetero atoms selected from nitrogen, oxygen and sulphur, each ring comprising 4 or 5 ring members,
a group of formula: 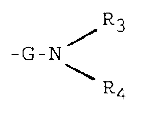
G representing a straight-chain or branched lower alkyl group, and
R₃ and R₄, which are identical or different, each representing a lower alkyl group or a hydrogen atom or a phenyl or phenyl-lower alkyl group, or
R₃ and R₄ forming together with the nitrogen atom to which they are attached an aromatic or nonaromatic, mono- or bi-cyclic heterocyclic system that optionally contains an additional hetero atom selected from nitrogen, oxygen and sulphur and is optionally substituted by one or more lower alkyl, oxo, aryl, aryl-lower alkyl,
substituted aryl or substituted aryl-lower alkyl groups,
it being understood that in the definitions of
R₂, R₃ and R₄ the term substituted defining the aryl and arylalkyl and heteroaryl groups denotes that those groups are substituted by one or more radicals selected from lower alkyl, lower alkoxy, trifluoromethyl and halogen,
or R₁ forms together with R₂ and the group N-CO a heterocyclic system of formula : 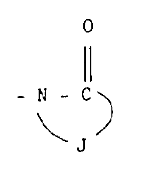 in which J is a straight-chain or branched alkyl radical having from 2 to 8 carbon atoms,
^{*} or a (CH₂)₂-NR₁-CO-R₂ chain in which R₁ and R₂ have the same meaning as above, in which case p is 0 or 1 and B represents a lower alkoxy group,
where appropriate their isomers, epimers and diastereoisomers and also, where appropriate, the addition salts thereof with a pharmaceutically acceptable acid or base, it being understood that lower alkyl and lower alkoxy denote groups containing from 1 to 6 carbon atoms and that cycloalkyl denotes groups containing from 3 to 8 carbon atoms,
characterised, when B is other than the chain -CH₂CH₂NR,COR₂, in that the starting material used is a compound of formula (II) : 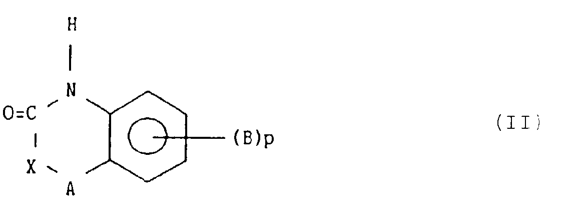
wherein A, B, p and X are as defined for formula (I), with the exception of the case where B represents a -CH₂-CH₂-NR₁COR₂ chain,
which is treated with an alkaline agent to obtain a compound of formula (III) : 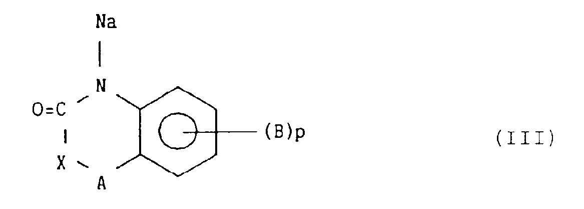
which compound of formula (III) is treated :
- either with a compound of formula (IV/A) : 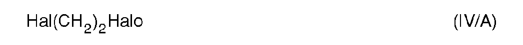
wherein Hal and Halo, which are identical or different,
each represents a halogen atom
to obtain a compound of formula (VIA) : 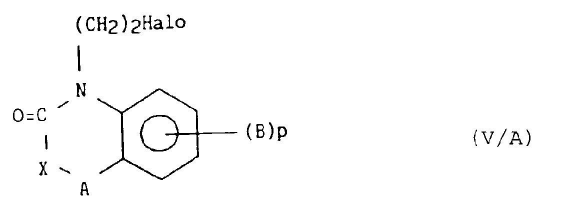
wherein A, X, B, p and Halo are as defined above,
which is treated with an amine of formula HNR₁ in which R₁ is as defined for formula (I), to obtain a compound of formula (VI) : 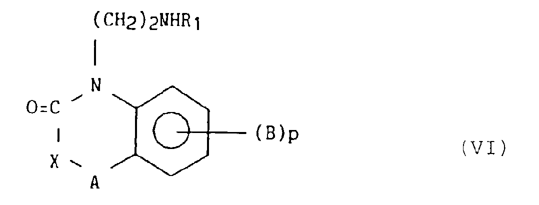
wherein A, B, p, R₁ and X are as defined above,
- or with a compound of formula (IV/B) : 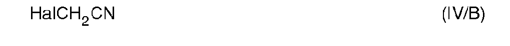
wherein Hal is as defined above,
to obtain a compound of formula (V/B) : 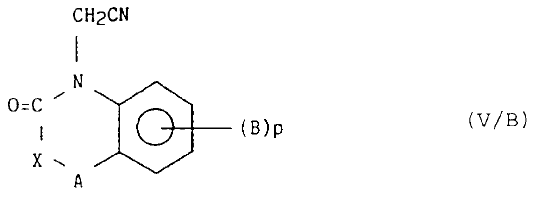
wherein X, A, B and p are as defined above,
which compound of formula (V/B) is subjected to the action of a reducing agent and then, if in the compound of formula (I) it is desired to obtain R₁ represents a lower alkyl group, to the action of an alkylating agent to obtain a compound of formula (VI) as defined above,
which compound of formula (VI) is treated :
- either with a compound of formula (VII) : 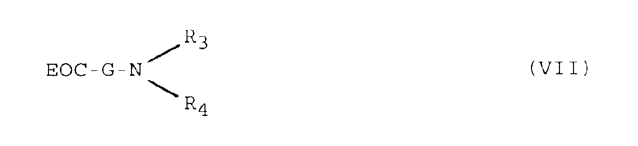 wherein E denotes a leaving group selected from hydroxyl, lower alkoxy and halogen, G, R₃ and R₄ having the same meaning as in formula (I), optionally in the presence of an alkaline agent, to yield a compound of formula (I/A) : 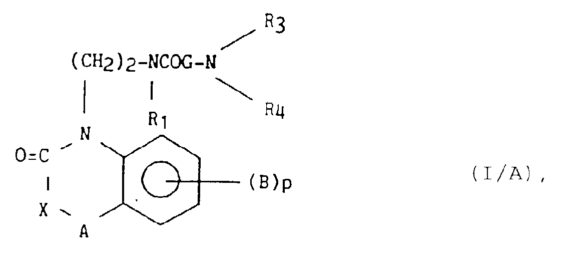 a particular case of compounds of formula (I) wherein A, B, X, R₁, R₃, R₄, G and p are as defined above, R₂ here denoting a group : 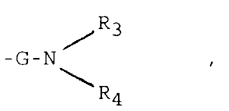 with the proviso that B does not represent a group : 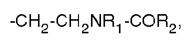 which, if desired, is purified by conventional techniques such as chromatography or crystallisation and which, if desired, is converted into a salt with a pharmaceutically acceptable acid,
- or with an acid chloride of formula (IX) : 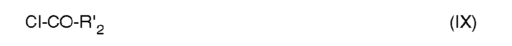 or with the corresponding acid anhydride,
R'₂ here denoting :
- a straight-chain or branched lower alkyl group or a cycloalkyl group each optionally substituted by a halogen atom,
- a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
to yield a compound of formula (I/B) : 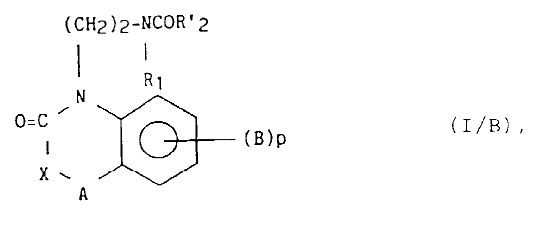 a particular case of compounds of formula (I) wherein m, X, A, B, p, R₁ and R'₂ are as defined above, it not being possible for B to represent a -CH₂-CH₂-NR,COR₂ chain,
which, if necessary, is purified by conventional techniques such as chromatography and/or crystallisation,
which compound of formula (I/B), when R'₂ represents a straight-chain or branched lower alkyl group substituted by a halogen atom, may, if desired, be subjected to the action of an amine of formula (X) : 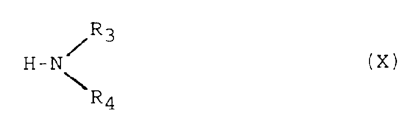 wherein R₃ and R₄ are as defined above,
in excess or in the presence of a tertiary amine or an alkali metal salt to yield a compound of formula (I/A) as defined above which, if desired, is purified by a conventional technique such as chromatography and/or crystallisation, and/or converted into a salt with a pharmaceutically acceptable acid,
which compound of formula (I/B), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/C) : 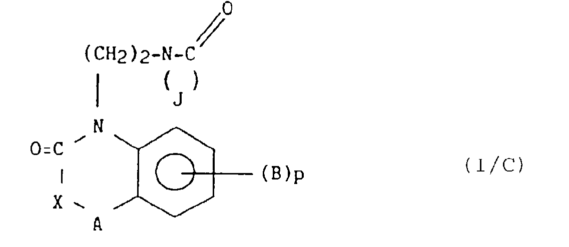 wherein B, p, X and A are as defined above, it being understood that B cannot represent a -CH₂-CH₂NRCOR₂ group, and J represents a straight-chain or branched alkyl group containing from 2 to 8 carbon atoms, a particular case of compounds of formula (I) in which R₁ and R₂ form together with NCO a monocyclic system substituted by an oxo group and optionally substituted by one or more lower alkyl groups,
which, if desired, is purified by a technique selected from crystallisation and chromatography.

2. Process for the preparation of compounds of formula (I) according to claim 1 wherein B represents a -CH₂-CH₂-NR₁ COR₂ chain and p represents 1, characterised in that the starting material used is a compound of formula (VIII) : 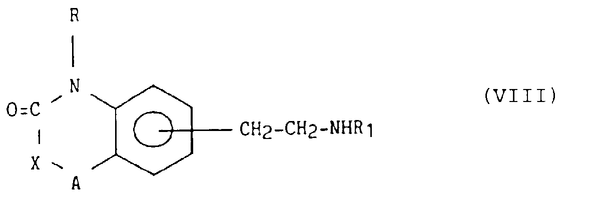 wherein R₁, R, X and A are as defined for formula (I), which is treated :
^{*} either with a compound of formula (VII) : 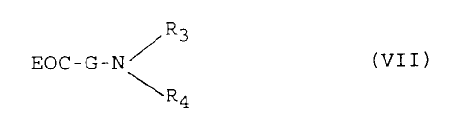
wherein E denotes a leaving group selected from hydroxyl, lower alkoxy and halogen, G, R₃ and R₄ having the same meaning as in formula (I),
optionally in the presence of an alkaline agent,
to yield a compound of formula (I/D) : 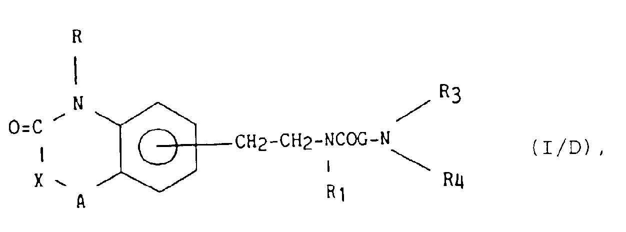 a particular case of compounds of formula (I) wherein X and A, R₁, R₃, R₄ and G are as defined above and R represents a hydrogen atom or a lower alkyl group,
R₂ here denoting a group : 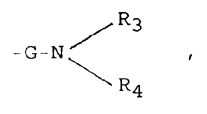
which, if desired, is purified by conventional techniques, such as chromatography, crystallisation and, if desired, is converted into a salt with a pharmaceutically acceptable acid or, when R represents a hydrogen atom, with a pharmaceutically acceptable base,
^{*} or with an acid chloride of formula (IX) : 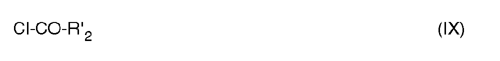 or with the corresponding acid anhydride,
R'₂ here denoting :
- a straight-chain or branched lower alkyl group or a cycloalkyl group each optionally substituted by a halogen atom, or a cycloalkyl group substituted by a straight-chain or branched lower alkyl group,
- an aryl or heteroaryl or aryl-lower alkyl group optionally substituted by one or more halogen atoms or groups selected from lower alkyl, lower alkoxy and trifluoromethyl, the term heteroaryl having the same meaning as given above,
- an imidazolyl group optionally reduced and/or substituted by an oxo group,
to yield a compound of formula (I/E) : 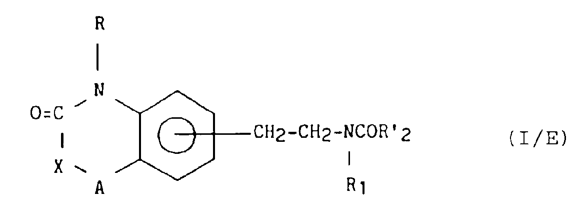 a particular case of compounds of formula (I) wherein Rᵢ, X, A and R'₂ are as defined above, and R represents a lower alkyl group or a hydrogen atom,
which, if necessary, is purified by conventional techniques such as chromatography and/or crystallisation and, if desired, is converted into a salt with a pharmaceutically acceptable base when R represents a hydrogen atom, which compound of formula (I/E), when R'₂ represents a straight-chain or branched lower alkyl group substituted by a halogen atom, may, if desired, be subjected to the action of an amine of formula (X) : 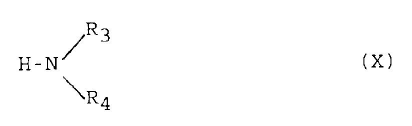 wherein R₃ and R₄ are as defined above, in excess or in the presence of a tertiary amine or an alkali metal salt to yield a compound of formula (I/D) as defined above which, if desired, is purified by a conventional technique such as chromatography and/or crystallisation, and/or is converted into a salt with a pharmaceutically acceptable acid, which compound of formula (I/E), when R'₂ represents a straight-chain or branched alkyl substituent containing at least two carbon atoms and substituted by a halogen atom and when, simultaneously, R₁ represents a hydrogen atom, may, if desired, be subjected to the action of a strong base and preferably an alkali metal alcoholate to yield a compound of formula (I/F) : 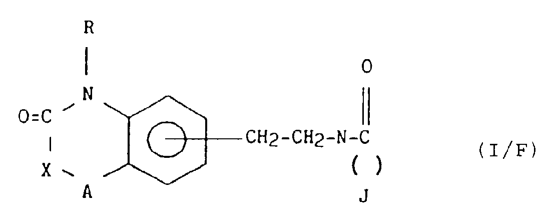 wherein R represents a hydrogen atom or a lower alkyl group, X and A are as defined above and J represents a straight-chain or branched alkyl group containing from 2 to 8 carbon atoms, a particular case of compounds of formula (I) wherein R₁ and R₂ form together with NCO a monocyclic system substituted by an oxo group and optionally substituted by one or more lower alkyl groups,
which, if desired, is purified by a technique selected from crystallisation and chromatography and, if desired, is converted into a salt with a pharmaceutically acceptable base when R represents a hydrogen atom.

3. Process for the preparation of compounds of formula (I/K) 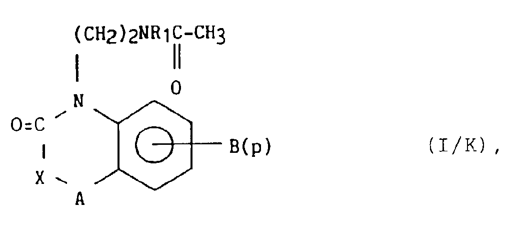 wherein R,, A, X, Band pare as defined for formula (I) according to claim 1, by catalytic reduction of the compounds of formula (V/K) 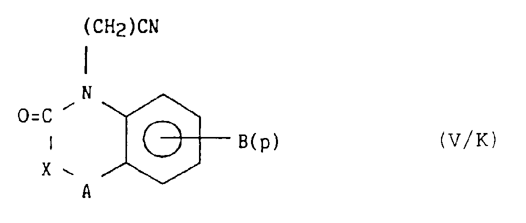 wherein X, A, B and p are as defined for formula (I), followed, if necessary, by purification and, when R₁ represents a lower alkyl group, by treatment with an alkylating agent.

4. Process according to claim 1 for the preparation of compounds wherein R represents a -(CH₂)₂-NR₁-CO-R₂ chain, B represents a methoxy group and p represents 1, their isomers and also, where appropriate, the addition salts thereof with a pharmaceutically acceptable acid.

5. Process according to claim 1 for the preparation of compounds according to claim 1 wherein R represents a (CH₂)₂NHCOR₂ chain in which R₂ represents a lower alkyl or cycloalkyl group, and B represents a methoxy group and p is 1.

6. Process according to claim 1 for the preparation of compounds according to claim 1 wherein B represents a (CH₂)₂NHCOR₂ chain in which R₂ represents a lower alkyl or cycloalkyl group, and also, where appropriate, the addition salts thereof with a pharmaceutically acceptable base.

7. Process according to claim 1 for the preparation of compounds according to claim 1 wherein X represents a single bond, where appropriate their isomers, and the addition salts thereof with a pharmaceutically acceptable acid or base.

8. Process according to claim 1 for the preparation of the compound according to claim 1 that is N-[2-(6-methoxy-3-benzoxazolinonyl)ethyl]acetamide.

9. Process according to claim 1 for the preparation of the compound according to claim 1 that is N-[2-(5-methoxy-3-benzoxazolinonyl)ethyl]acelamide.

10. Process according to claim 1 for the preparation of the compound according to claim 1 in which R represents a CH₂-CH₂NHCO-CH₃ group, B represents an OCH₃ group and p is 1.

11. Process according to claim 1 for the preparation of the compound according to claim 1 that is N-[2-(6-methoxy-3-benzothiazolinonyl)ethyl]acetamide.

12. Process according to claim 1 for the preparation of the compound according to claim 1 that is N-[2-(5-methoxy-3-benzothiazolinonyl)ethyl]acetamide.

13. Process according to claim 1 for the preparation of compounds according to claim 1 wherein R represents a hydrogen atom or a methyl group and B represents a (CH₂)₂NHCOR₂ group in which R₂ represents a lower alkyl or cycloalkyl group, and also, where appropriate, the addition salts thereof with a pharmaceutically acceptable base.

14. Process according to claim 1 for the preparation of the compound according to claim 1 that is N-[2-(3-methyl-6-benzoxazolinonyl)ethyl]acetamide.

15. Process according to claim 1 for the preparation of the compound according to claim 1 that is N-[2-(5-methoxy-3-benzoxazolinonyl)ethyl]cyclopropane carboxamide.

16. Process according to claim 1 for the preparation of the compound according to claim 1 that is N-[2-(5-methoxy-3-benzothiazolinonyl)ethyl]cyclopropane carboxamide.

17. Process for the preparation of a pharmaceutical composition comprising as active ingredient at least one compound according to any one of claims 1 to 16 in combination with one or more pharmaceutically acceptable, inert, non-toxic excipients or carriers.

18. Process for the preparation of a pharmaceutical composition according to claim 17 for use in the treatment of stress, anxiety, seasonal depression, insomnia and fatigue resulting from shiftwork, schizophrenia, panic attack, melancholia, regulation of the appetite, insomnia, psychotic disorders, epilepsy, Parkinson's disease, senile dementia, various disorders associated with normal or pathological ageing, memory loss, Alzheimer's disease, and also cerebral circulation disorders, cancer, psoriasis, acne, seborrhoea, or as an ovulation inhibitor or in veterinary medicine in coat disorders.
